(19) Europäisches Patentamt
European Patent Office
Office européen des brevets

(11) Veröffentlichungsnummer : **0 071 819 B2**

(12) # NEUE EUROPÄISCHE PATENTSCHRIFT

(45) Veröffentlichungstag der neuen Patentschrift : **10.06.92 Patentblatt 92/24**

(21) Anmeldenummer : **82106537.2**

(22) Anmeldetag : **20.07.82**

(51) Int. Cl.$^5$ : **C07D 211/90,** C07D 211/84, C07D 401/04, C07D 471/04, C07D 491/04, C07D 491/10, C07D 491/048, C07D 491/052, A61K 31/435, // (C07D498/04, 265:00, 221:00), (C07D471/04, 221:00, 209:00), (C07D491/04, 307:00, 221:00), (C07D491/10, 311:00, 221:00), (C07D491/04, 311:00, 221:00)

(54) **Dihydropyridine mit positiv inotroper Wirkung, neue Verbindungen, ihre Verwendung in Arzneimitteln und Verfahren zu ihrerHerstellung.**

Die Akte enthält technische Angaben, die nach dem Eingang der Anmeldung eingereicht wurden und die nicht in dieserPatentschrift enthalten sind.

(30) Priorität : **30.07.81 DE 3130041**
**25.02.82 DE 3206671**

(43) Veröffentlichungstag der Anmeldung : **16.02.83 Patentblatt 83/07**

(45) Bekanntmachung des Hinweises auf die Patenterteilung : **22.04.87 Patentblatt 87/17**

(45) Bekanntmachung des Hinweises auf die Entscheidung über den Einspruch : **10.06.92 Patentblatt 92/24**

(84) Benannte Vertragsstaaten : **AT BE CH DE FR GB IT LI LU NL SE**

(56) Entgegenhaltungen :
**EP-A- 0 002 208**
**EP-A- 0 011 706**
**EP-A- 0 026 317**
**EP-A- 0 031 801**
**EP-A- 0 039 863**
**EP-A- 0 042 092**
**EP-A- 0 042 093**
**EP-A- 0 042 566**
**EP-A- 0 042 569**
**DE-A- 2 629 892**
**DE-A- 2 658 804**
**GB-A- 1 305 793**

(73) Patentinhaber : **BAYER AG**
**W-5090 Leverkusen 1 Bayerwerk (DE)**

(72) Erfinder : **Franckowiak, Gerhard, Dr.**
**Pahlkestrasse 17**
**W-5600 Wuppertal 1 (DE)**
Erfinder : **Böshagen, Horst, Dr.**
**Wiesenstrasse 4**
**W-5657 Haan (DE)**
Erfinder : **Bossert, Friedrich, Dr.**
**Claudiusweg 7**
**W-5600 Wuppertal 1 (DE)**
Erfinder : **Goldmann, Siegfried, Dr.**
**Kuckuckstrasse 41**
**W-5600 Wuppertal 2 (DE)**
Erfinder : **Meyer, Horst, Dr.**
**Henselweg 11**
**W-5600 Wuppertal 1 (DE)**
Erfinder : **Wehinger, Egbert, Dr.**
**Gellertweg 33**
**W-5600 Wuppertal 1 (DE)**
Erfinder : **Stoltefuss, Jürgen, Dr.**
**Parkstrasse 20**
**W-5657 Haan 2 (DE)**
Erfinder : **Schramm, Matthias, Dr.**
**Paffrather Strasse 38**
**W-5000 Köln 80 (DE)**
Erfinder : **Thomas, Günter, Dr.**
**Claudiusweg 9**
**W-5600 Wuppertal 1 (DE)**
Erfinder : **Towart, Robertson, Dr.**
**6 Pennylets Green**
**Stoke Poges Slough SL2 4BT (GB)**

EP 0 071 819 B2

Jouve, 18, rue Saint-Denis, 75001 PARIS

(56) Entgegenhaltungen :
**GB-A- 1 358 951**
**Goldman. Angew. Chemie 93, 1981 pp. 798-799**
**S. Kokubun et al. PNAS USA 81, 1984pp.4824-4827**
**S. Kokubun et al.Molecular Pharmacology, 30 pp. 571-584**
**M. Schramm et al.Journal of cardiovascular Pharmacology 7.1985. pp.492-496**
**Vorankündigung des. Int. Kongresses für Herzforschung mai 1989**
**J. Puls et alXII Congress of the int. Diabetes Fed. Sept. 1985**
**P.Hess et al. Nature 311, 1984pp.538-544**
**M. Beecham et al. TIPS vol. 9 1988 pp. 257-261**
**Deutsche Physiologische Ges. Abstracts of 64th meeting March 1987, Homburg/Saar. No. 146 Kohlhardt et al.**
**B: Nilius"Calcium block of normal and DP1 201-106 modified single sodium channels from guinea pig heart"**
**Puls et al. Rats. Diabetes Research & Clinical Practice supplement vol. 1 p. 1189, 1985**
**Puls et al. Aktion Endokrin Stoffw. 6 p. 103 1985**
**Morgan et al. Biochem. J. 231 pp. 629-634, 1985**
**Panten et al. Naunyn-Schmiedebergs Arch. Pharmacol. pp 328,351- 353, 1985**
**Malaisse-Lagae et al. Adv. Exp. Med. Biol. 211 pp.201-205 1986**
**Malaisse-Lagae et al. Biochem. Biophys. Re. Committee,123 pp. 1062-1068.1984**

## Beschreibung

Die vorliegende Erfindung betrifft die Verwendung von neuen und bekannten Dihydropyridinen in Arzneimitteln mit positiv inotroper Wirkung, neue Verbindungen aus dieser Stoffklasse, diese enthaltende Arzneimittel und ihre Herstellung.

Es ist bereits bekannt geworden, dass 1,4-Dihydropyridine gefässerweiternde Eigenschaften besitzen und als Coronarmittel und Antihypertensiva verwendet werden können (vgl. britisches Patent 1 173 062; britisches Patent 1 358 951; DE-OS 2 629 892 und DE-OS 2 752 820). Weiterhin ist bekannt, dass 1,4-Dihydropyridine als Calciumantagonisten eine Hemmung der Kontraktionskraft von glatten und kardialen Muskeln bewirken und zur Behandlung von Coronar- und Gefässerkrankungen eingesetzt werden können (vgl. A. Fleckenstein, Ann. Rev. Pharmacol. Toxicol. 17, 149-166 (1977)). Bei Kenntnis dieser Eigenschaften der Dihydropyridine war es nicht vorhersehbar, dass die erfindungsgemässen Verbindungen aus dieser Stoffklasse keine kontraktionshemmende, sondern eine kontraktionskraftverstärkende, am Herzmuskel positiv inotrope Wirkung besitzen.

Die Erfindung betrifft die Verwendung von 1,4-Dihydropyridinen der allgemeinen Formel (I)

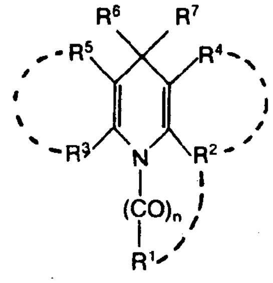

(I)

in welcher

n für 0, 1 oder 2 steht,

$R^1$

a) für Wasserstoff, einen geradkettigen, verzweigten, cyclischen, gesättigten oder ungesättigten aliphatischen Kohlenwasserstoffrest steht, der gegebenenfalls 1 bis 3 gleiche oder verschiedene Hetero-Kettenglieder aus der Gruppe O, CO, $SO_m$ (m = 0, 1 oder 2), = N-, $NR^I$ oder $SiR^{II}R^{III}$ enthält und wobei dieser Kohlenwasserstoffrest gegebenenfalls substituiert ist durch Halogen, $NO_2$, CN, $N_3$, Hydroxy, Aryl oder Heteroaryl oder

b) für einen Aryl- oder Heteroarylrest steht, wobei diese Reste gegebenenfalls 1 bis 5 gleiche oder verschiedene Substituenten aus der Gruppe Aryl, Alkyl, Alkenyl, Alkinyl, Alkenoxy, Alkinoxy, Aralkyl, Acyl, Alkylen, Dioxyalkylen, Halogen, $CF_3$, $OCF_3$, $SCF_3$, $NO_2$, NO, CN, $N_3$, $COR^{IV}$, $COOR^V$, $OR^{VI}$, $NR^I$ oder $NR^{VIII}R^{VIII}$ tragen, wobei die Alkyl-, Alkoxy- und Arylreste der obengenannten Substituenten ihrerseits wieder durch Halogen, $COR^V$ oder $NR^{VII}R^{VIII}$ substituiert sein können öder

c) für den Rest $NR^{VII}R^I$ steht, und wobei die unter a), b) und c) genannten Reste $R^I$, $R^{II}$, $R^{III}$, $R^{IV}$, $R^V$, $R^{VI}$, $R^{VII}$ und $R^{VIII}$ die unten angegebene Bedeutung haben,

$R^2$

a) für einen der unter $R^1$ angegebenen Substituenten steht, aber nicht mit diesem identisch sein muss oder

b) für die Reste $NHR^I$ oder

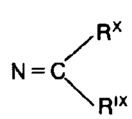

steht,

wobei $R^I$, $R^{IX}$ und $R^X$ die unten angegebene Bedeutung besitzen,

oder die Substituenten $R^1$ und $R^2$ gemeinsam einen 5- bis 8-gliedrigen, gesättigten oder ungesättigten Ring bilden, der gegebenenfalls 1, 2 oder 3 gleiche oder verschiedene Ringglieder aus der Gruppe O, S, $NR^I$ oder CO enthält und der gegebenenfalls 1 bis 3 gleiche oder verschiedene Substituenten aus der Gruppe Halogen, Hydroxy, Alkyl, Alkoxy, Aryl oder Aralkyl enthält,

$R^3$ für einen der unter $R^2$ angegebenen Substituenten steht, und mit diesem gleich oder von diesem verschieden ist, wobei nur einer der beiden Substituenten $R^2$ oder $R^3$ jeweils für Alkoxy, Alkylthio oder $NHR^I$ steht,

$R^4$

a) für Wasserstoff, $NO_2$, NO, CN, $SO_m$-$R^{XI}$ (m = 0, 1 oder 2), Halogen,

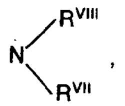, 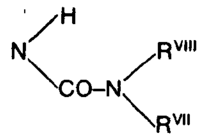, 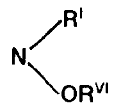

oder 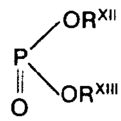

steht, wobei $R^I$, $R^{VII}$, $R^{VIII}$, $R^{XI}$ und $R^{XIII}$ die unten angegebene Bedeutung haben, oder

b) für einen verzweigten oder unverzweigten, cyclischen, gesättigten oder ungesättigten aliphatischen Kohlenwasserstoffrest steht, der gegebenenfalls substituiert ist durch Halogen, OH, CN, Alkoxy, Alkylthio, Aryloxy, $COOR^V$ oder

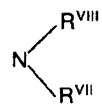

wobei $R^V$, $R^{VII}$ und $R^{VIII}$ die unten angegebene Bedeutung haben, oder

c) für einen aromatischen Kohlenwasserstoffrest oder für einen 5- bis 7-gliedrigen gesättigten oder ungesättigten Heteroring mit 1 bis 4 gleichen oder verschiedenen Heterogliedern aus der Gruppe O, S, -N =, $NR^I$, wobei dieser Heteroring entweder über ein Kohlenstoffatom oder ein Stickstoffatom mit dem Dihydropyridinring verknüpft ist, und wobei der aromatische Kohlenwasserstoffrest und die Heteroringe gegebenenfalls 1 bis 3 gleiche oder verschiedene Substituenten aus der Gruppe Halogen, OH, CN, $CF_3$, $OCF_3$, $SCF_3$, $NO_2$, Alkyl, Alkoxy, Aryl und

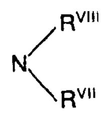 tragen,

d) für den Rest

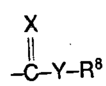

steht, wobei X Sauerstoff, Schwefel oder $NR^I$ bedeutet und Y für eine einfache Bindung, O, S oder $NR^I$ steht, und $R^8$ die für $R^1$ angegebene Bedeutung hat und mit dem Substituenten $R^1$ gleich oder von diesem verschieden ist, oder

e) für den Rest

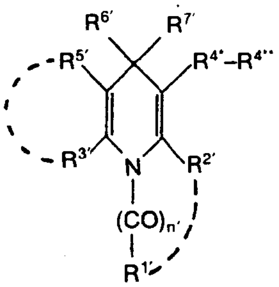

steht,

wobei n′, $R^{1'}$, $R^{2'}$, $R^{3'}$, $R^{5'}$, $R^{6'}$und $R^{7'}$ die für n, $R^1$, $R^2$, $R^3$, $R^5$, $R^6$ und $R^7$ angegebene Bedeutung haben und mit diesen gleich oder von diesen verschieden sind, und $R^{4*}$ und $R^{4**}$ gleich oder verschieden sind und jeweils für einen um ein Wasserstoff verminderten Rest der für $R^4$ unter a) bis d) angegebenen Substituenten stehen, oder

$R^2$ und $R^4$ gemeinsam einen verzweigten, unverzweigten, gesättigten oder ungesättigten 5- bis 8-gliedrigen Ring bilden, der gegebenenfalls 1, 2 oder 3 gleiche oder verschiedene Ringglieder aus der Gruppe O, CO, CS, C = $NR^I$, = N-, $NR^I$, $SO_m$ (m = 0, 1 oder 2) oder $SiR^{II}R^{III}$ enthält, und der gegebenenfalls substituiert ist durch Halogen, Hydroxy, Alkoxy, Aryl, Aralkyl,

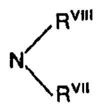

oder durch eine geradkettige oder verzweigte Alkylenkette mit 3 bis 8 Kohlenstoffatomen disubstituiert ist, wobei dieser gemeinsame Ring von $R^2$ und $R^4$ auch direkt mit dem gemeinsamen Ring von $R^1$ und $R^2$ anneliert sein kann,
wobei die Reste $R^I$, $R^{II}$, $R^{III}$, $R^{VII}$, $R^{VIII}$ die unten angegebene Bedeutung haben,

$R^5$ die für $R^4$ angegebene Bedeutung besitzt und mit $R^4$ gleich oder von diesem verschieden ist, oder $R^5$ und $R^3$ gemeinsam einen Ring bilden, der mit dem durch $R^2$ und $R^4$ gebildeten Ring gleich oder von diesem verschieden ist,

$R^6$ für Wasserstoff, Alkyl oder Halogenalkyl steht, und

$R^7$

a) für einen gesättigten, ungesättigten, cyclischen, geradkettigen oder verzweigten aliphatischen Kohlenwasserstoffrest steht, der gegebenenfalls substituiert ist durch Halogen, Aryl oder Heteroaryl, oder

b) für einen Aryl- oder Heteroarylrest steht, der gegebenenfalls 1 bis 5 gleiche oder verschiedene Substituenten aus der Gruppe $No_2$, CN, $N_3$, NO, $CF_3$, Halogen, $COR^{IV}$, $COOR^V$, $OR^{VI}$

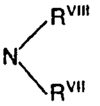 , $SO_mR^{XI}$ (m = 0, 1 oder 2), 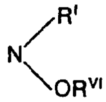 ,

Alkyl, Aryl, Alkenyl, Alkinyl, Alkenoxy, Alkinoxy, Aralkyl, Acyl, Alkylen, oder Dioxyalkylen enthält, wobei die vorgenannten Alkyl- und Aryl-Substituenten ihrerseits wieder durch $COOR^V$ oder

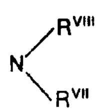

substituiert sein können, und wobei in den vorgenannten Definitionen der Substituenten $R^1$ bis $R^8$ $R^I$ für Wasserstoff, Alkyl, Aryl, Aralkyl, Heteroaryl oder Acyl steht, $R^{II}$ und $R^{III}$ gleich oder verschieden sind und jeweils für Alkyl, Aryl, Aralkyl oder Heteroaryl stehen, $R^{IV}$, $R^V$ und $R^{VI}$ jeweils gleich oder verschieden sind und für Wasserstoff, Alkyl, Aryl, Aralkyl oder Heteroaryl stehen, wobei die Alkyl- und Acylreste gegebenenfalls durch Halogen, Alkylthio, Alkoxy, Trifluormethylnitro oder $R^{VII}$ und $R^{VIII}$ jeweils gleich oder verschieden sind und für Wasserstoff, Aryl oder Aralkyl stehen oder für Alkyl, welches gegebenenfalls durch O, S oder $NR^{I}$ unterbrochen ist, stehen oder
gemeinsam mit dem Stickstoffatom einen 5- bis 7-gliedrigen Ring bilden, der 1 oder 2 gleiche oder verschiedene Heteroringglieder aus der Gruppe O, S oder $NR^{I}$ enthalten kann oder
wobei einer der Reste $R^{VIII}$ oder $R^{VIII}$ für eine aliphatische Acylgruppe mit bis zu 6 Kohlenstoffatomen steht, $R^{IX}$, $R^{X}$, $R^{XI}$, $R^{XII}$ und $R^{XIII}$ jeweils gleich oder verschieden sind und für Alkyl, Aryl oder Aralkyl stehen,
wobei die unter $R^1$ bis $R^8$ und unter $R^I$ bis $R^{XIII}$ genannten Alkyl-, Aryl-, Aralkyl-, Heteroaryl- und Acylreste sowie der mit $R^{VII}$ und $R^{VIII}$ gebildete Heteroring ihrerseits gegebenenfalls substituiert sind durch OH, $CF_3$, $OCF_3$, CN, $NO_2$, Halogen, niederes Thioalkyl, niederes Alkyl, niederes Alkoxy, Aryl oder Aralkyl,
und wobei als Heteroaryl folgende Substituenten genannt seien:
Thienyl, Furyl, Pyryl, Pyridyl, Chinolyl, Isochinolyl, Pyrimidyl, Pyridazinyl, Chinazolyl, Chinoxalyl, Benzothienyl, Pyrazolyl, Imidazolyl, Oxazolyl, Isoxazolyl, Thiazolyl, Triazolyl, Oxydiazolyl, Pyrazinyl, Oxazinyl, Thiazinyl, Indolizinyl, Indolyl, Benzofuranyl, Indazolyl, Benzothienyl, Benzimidazolyl, Benzoxazolyl, Benzthiazolyl, Benztriazolyl, Benzoxadiazolyl, Cinnolinyl, Phthalazinyl, Naphthyridinyl oder Benzotriazinyl, in Form von Isomeren, Isomerengemischen, Racematen und optischen Antipoden sowie ihrer pharmazeutisch unbedenklichen Salze, zur Herstellung von Arzneimitteln mit positiv inotroper Wirkung, die sich insbesondere als Cardiotonika eignen. Dieser kontraktionskraftverstärkende Effekt beruht auch darauf, dass die erfindungsgemässen Verbindungen den $Ca^{++}$-Einstrom in die Zelle erhöhen und somit auch zur Behandlung von hypotonen Kreislaufzuständen, zur Senkung von Blutzucker, zur Abschwellung von Schleimhäuten und zur Beeinflussung des Salz- und Flüssigkeitshaushaltes geeignet sind.

Die Verbindungen der allgemeinen Formel (I) sind solche, die ab einer Konzentration von $10^{-5}$ g/ml am linken Vorhof des ilosierten Meerschweinchenherzens eine Kontraktionsverstärkung um mindestens 25% bewirken.
Die Versuchanordnung ist wie folgt:

Die linken Vorhöfe von Meerschweinchenherzen werden isoliert und in ein thermostatisiertes Organbad gehängt, welches eine isotonische Mineralsalzlösung, die dem Ionenmilieu und dem pH-Wert von Körperflüssigkeiten angepasst ist und geeignete Nährstoffe enthält. Dieses Organbad wird mit einem Gasgemisch bestehend aus Sauerstoff und Kohlendioxyd begast, wobei der Kohlendioxydgehalt so bemessen ist, dass der pH-Wert des Organbades konstant bleibt. Die linken Vorhöfe werden in das Organbad eingespannt, die Spannung wird mittels eines Kraftaufnehmers registriert, wobei ein bestimmter Grundtonus eingestellt wird. Anschliessend werden die linken Vorhöfe kontinuierlich in bestimmten Abständen elektrisch gereizt und die dabei erfolgenden Kontraktionen registriert. Nach Zugabe des Wirkstoffs werden die Kontraktionen weiterhin registriert. Eine Kontraktionsverstärkung um mindestens 25% gilt als signifikante positiv-inotrope Wirkung.

Die erfindungsgemässen Verbindungen der allgemeinen Formel (I) können nach üblichen Methoden auf verschiedene Weise hergestellt werden. Ihre Synthese erfolgt z. B.

A) durch direkten Aufbau der die Hydropyridinstruktur nach bekannten Dihydropyridinsynthesen oder

B) durch Umwandlung von funktionellen Gruppen am Dihydropyridingerüst nach bekannten Reaktionsschemen.

A) Aufbau der 1,4-Dihydropyridin-Grundstruktur Der Aufbau der 1,4-Dihydropyridin-Grundstruktur kann erfolgen, indem man

a) Carbonylverbindungen der allgemeinen Formel (II)

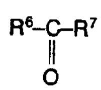 (II)

in welcher $R^6$ und $R^7$ die oben angegebene Bedeutung haben,

mit Ketonen der Formel (III) und (IV)

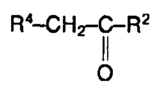 (III) 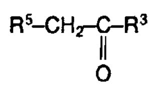 (IV)

in welchen $R^2$, $R^3$, $R^4$ und $R^5$ die oben angegebene Bedeutung haben,

und primären Aminen der Formel (V)

$$H_2N\text{-}(CO)_n\text{-}R^1 \quad (V)$$

in welcher n = 0 und $R^1$ die oben angegebene Bedeutung besitzt,

gegebenenfalls in Gegenwart von inerten Lösungsmitteln umsetzt,

oder

b) Carbonylverbindungen der Formel (II)

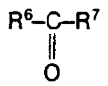

(II)

in welcher $R^6$ und $R^7$ die oben angegebene Bedeutung haben,

mit Enaminen der Formel (VI)

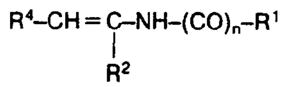

(VI)

in welcher $R^1$, $R^2$, $R^4$ und n die oben angegebene Bedeutung haben und

Ketonen der Formel (IV)

$$R^3\text{-CO-CH}_2\text{-}R^5 \qquad (IV)$$

in welcher $R^3$ und $R^5$ die oben angegebene Bedeutung haben,

gegebenenfalls in Gegenwart von inerten Lösungsmitteln umsetzt oder

c) Enamine der Formel (VI)

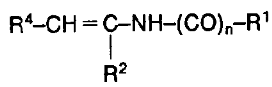

(VI)

in welcher $R^1$, $R^2$, $R^4$ und n die oben angegebene Bedeutung haben

mit Ylidenverbindungen der Formel (VII)

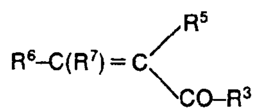

(VII)

in welcher $R^3$, $R^5$, $R^6$ und $R^7$ die oben angegebene Bedeutung haben,

gegebenenfalls in Gegenwart von inerten Lösungsmitteln umsetzt.

B) Umwandlung der funktionellen Gruppen

Durch Umwandlung einer oder mehrerer funktioneller Gruppen bekannter Dihydropyridine oder auch neuer Dihydropyridine die gemäss Verfahren A) a) bis c) erhalten wurden, können ebenfalls erfindungsgemäss Verbindungen der allgemeinen Formel (I) nach üblichen Methoden hergestellt werden (vgl. Houben-Weyl, Methoden der Organischen Chemie, Georg Thieme Verlag, Stuttgart 1966; Organicum, VEB Deutscher Verlag der Wissenschaften, Berlin 1969; W. Foerst, Neuere Methoden der präparativen organischen Chemie, Bd. 1-5, Verlag Chemie, Weinheim 1961; C. Ferri, Reaktionen der organischen Chemie, Georg Thieme Verlag, Stuttgart 1978; Fieser+ Fieser, Reagents for Organic Synthesis, Vol. 1-8, J. Wiley & Sons, Inc., London 1967.

Als bevorzugte Umwandlungsreaktionen seien genannt: sauer oder basisch katalysierte Hydrolyse, Veresterung oder Umesterung, Lactamisierung, Kondensation, Acylierung, Reduktion oder Cyclisierung mit jeweils geeigneten Reaktionspartnern oder intramolekularer Art. Als besonders vorteilhaft sei die Lactonisierung erwähnt. Hierzu werden Verbindungen der allgemeinen Formel (I), in denen die Substituenten $R^2$ und $R^4$ oder/und $R^3$ und $R^5$ freie und/oder durch Schutzgruppen blokkierte Hydroxyl- und Carbonsäurefunktionen enthalten, unter geeigneten basisch oder sauer katalysierten Reaktionsbedingungen, gegebenenfalls unter vorhergehender ganz- oder teilweiser Abspaltung der Schutzgruppen, zu erfindungsgemässen Lactonen der allgemeinen Formel (I) cyclisiert.

In der deutschen Offenlegungsschrift Nr. 2 629 892 werden einige der möglichen Herstellungsmethoden ausführlicher beschrieben.

Bevorzugt seien Verbindungen der allgemeinen Formel (I) genannt, in welcher

n für 0, 1 oder 2 steht,

$R^1$

a) für Wasserstoff, einen geradkettigen, verzweigten, cyclischen gesättigten oder ungesättigten aliphatischen Kohlenwasserstoffrest mit bis zu 10 C-Atomen steht der gegebenenfalls 1 oder 2 gleiche oder verschiedene Heterokettenglieder aus der Gruppe O, CO, S, $SO_2$, =N- oder $NR^I$ enthält und wobei dieser Kohlenwasserstoffrest gegebenenfalls substituiert ist durch Halogen, $NO_2$, CN, $N_3$, Hydroxy, mit 1 bis 4 C-Atomen, Phenyl, Naphthyl oder Heteroaryl oder

b) für einen Phenyl-, Naphthyl- oder Heteroarylrest steht, wobei diese Reste gegebenenfalls 1 bis 3 gleiche oder verschiedene Substituenten aus der Gruppe Phenyl, Alkyl, Alkenyl, Alkinyl, Alkenoxy, Alkinoxy mit jeweils bis zu 4 C-Atomen, Aralkyl mit 7 bis 14 C-Atomen, Acyl mit bis zu 6 C-Atomen, Alkylen, Dioxyalkylen mit bis zu 4 Kohlenstoffatomen in der Alkylenkette, Halogen, $CF_3$, $OCF_3$, $SCF_3$, $NO_2$, CN, $N_3$, $COR^{IV}$, $COOR^V$, $OR^{VI}$, $NR^I$ oder $NR^{VII}R^{VIII}$ tragen, wobei die Alkyl-, Alkoxy-und Arylreste der oben genannten Substituenten ihrerseits wieder durch Halogen, $COOR^V$ oder $NR^{VII}R^{VIII}$ substituiert sein können oder

c) für den Rest $NR^{VII}R^I$ steht,

und wobei die unter a), b) und c) genannten Reste $R^I$, $R^{II}$, $R^{III}$, $R^{IV}$, $R^V$, $R^{VI}$, $R^{VII}$ und $R^{VIII}$ die unten angegebene Bedeutung haben,

$R^2$ ein

a) für einen der unter $R^1$ angegebenen Substituenten steht aber nicht mit diesen identisch sein muss oder

b) für die Reste $NHR^I$ oder

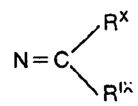

steht,

wobei $R^I$, $R^{IX}$ und $R^X$ die unten angegebene Bedeutung besitzen,

oder die Substituenten

$R^1$ und $R^2$ gemeinsam einen 5- bis 7-gliedrigen gesättigten oder ungesättigten Ring bilden, der gegebenenfalls ein oder zwei gleiche oder verschiedene Ringglieder aus der Gruppe O, S, $NR^I$ oder CO enthält und der gegebenenfalls ein bis drei gleiche oder verschiedene Substituenten aus der Gruppe Halogen, Hydroxy, Alkyl, Alkoxy mit jeweils 1 bis 4 C-Atomen, Phenyl, Naphthyl oder Aralkyl mit 7 bis 14 C-Atomen enthält,

$R^3$ für einen der unter $R^2$ angegebenen Substituenten steht und mit diesem gleich oder von diesem verschieden ist, wobei nur einer der beiden Substituenten $R^2$ oder $R^3$ jeweils für Alkoxy, Alkylthio, oder $NHR^I$ steht,

$R^4$

a) für Wasserstoff, $NO_2$, NO, CN, $SO_m$-$R^{IX}$ (m = 0 oder 2), Halogen,

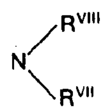 , 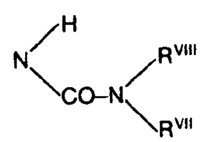 , 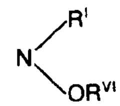

oder 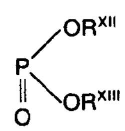 steht,

wobei $R^I$, $R^{VII}$, $R^{VIII}$, $R^{XI}$ und $R^{XIII}$ die unten angegebene Bedeutung haben, oder

b) für einen verzweigten oder unverzweigten, cyclischen, gesättigten oder ungesättigten aliphatischen Kohlenwasserstoffrest mit bis zu 10 C-Atomen steht, der gegebenenfalls substituiert ist durch Halogen, OH, CN, Alkoxy, Alkylthio mit jeweils 1 bis 4 Kohlenstoffatomen, Phenyloxy, Naphthoxy, $COOR^V$ oder

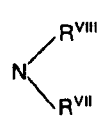

wobei $R^V$, $R^{VII}$ und $R^{VIII}$ die unten angegebene Bedeutung haben, oder

c) für einen aromatischen Kohlenwasserstoffrest mit 6 bis 10 C-Atomen oder für einen 5- bis 7-gliedrigen

gesättigten oder ungesättigten Heteroring mit 1 bis 3 gleichen oder verschiedenen Heterogliedern aus der Gruppe O, S, -N =, $NR^I$, wobei dieser Heteroring entweder über ein Kohlenstoffatom oder ein Stickstoffatom mit dem Dihydropyridinring verknüpft ist, und wobei der aromatische Kohlenwasserstoffrest und die Heteroringe gegebenenfalls 1 bis 3 gleiche oder verschiedene Substituenten aus der Gruppe Halogen, OH, CN, $CF_3$, $OCF_3$, $SCF_3$, $NO_2$, Alkyl, Alkoxy mit jeweils 1 bis 4 C-Atomen, Phenyl, Naphthyl und

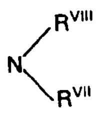
,

tragen,
oder
c) für den Rest

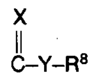

steht, wobei X Sauerstoff, Schwefel oder $NR^1$ bedeutet und Y für eine einfache Bindung, O, S oder $NR^I$ steht, und $R^8$ die für $R^1$ angegebene Bedeutung hat und mit dem Substituenten $R^1$ gleich oder von diesem verschieden ist, oder
e) für den Rest

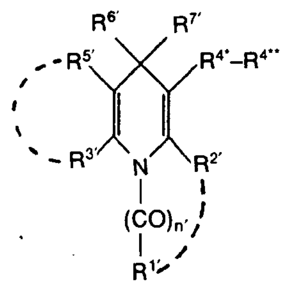

steht,

wobei n′, $R^{1'}$, $R^{2'}$, $R^{3'}$, $R^{5'}$, $R^{6'}$ und $R^{7'}$ die für n, $R^1$, $R^2$, $R^3$, $R^5$, $R^6$ und $R^7$ angegebene Bedeutung haben und mit diesen gleich oder von diesen verschieden sind, und

$R^{4*}$ und $R^{4**}$ gleich oder verschieden sind und jeweils für einen um ein Wasserstoff verminderten Rest der für $R^4$ unter a) bis d) angegebenen Substituenten stehen, oder

$R^2$ und $R^4$ gemeinsam einen verzweigten, unverzweigten, gesättigten oder ungesättigten 5- bis 7-gliedrigen Ring bilden, der gegebenenfalls 1, 2 oder 3 gleiche oder verschiedene Ringglieder aus der Gruppe O, CO, CS, C = $NR^I$, = N-, $NR^I$ oder $SO_m$ (m = 0 oder 2) enthält und der gegebenenfalls substituiert ist durch Halogen, Hydroxy, Alkoxy mit 1 bis 4 C-Atomen, Phenyl, Naphthyl, Aralkyl mit 7 bis 14 C-Atomen,

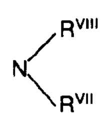

oder durch eine geradkettige oder verzweigte Alkylenkette mit 3 bis 8 Kohlenstoffatomen disubstituiert ist, wobei dieser gemeinsame Ring von $R^2$ und $R^4$ auch direkt mit dem gemeinsamen Ring von $R^1$ und $R^2$ anneliert sein kann, wobei die Reste $R^I$, $R^{II}$, $R^{III}$, $R^{VII}$, $R^{VIII}$ die unten angegebene Bedeutung haben,

$R^5$ die für $R^4$ angegebene Bedeutung besitzt und mit $R^4$ gleich oder von diesem verschieden ist,

$R^6$ für Wasserstoff, Alkyl oder Halogenalkyl mit jeweils 1 bis 4 C-Atomen steht und

$R^7$

a) für einen gesättigten, ungesättigten cyclischen, geradkettigen oder verzweigten aliphatischen Kohlenwasserstoffrest mit bis zu 10 C-Atomen steht, der gegebenenfalls substituiert ist durch Halogen, Phenyl, Naphthyl oder Heteroaryl oder

b) einen Phenyl-, Naphthyl- oder Heteroarylrest steht, der gegebenenfalls 1 bis 3 gleiche oder verschiedene Substituenten aus der Gruppe $NO_2$, Halogen, CN, $N_3$, NO, $CF_3$, $COR^{IV}$, $COOR^{V}$, $OR^{VI}$,

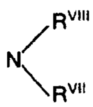 , $SO_mR^{XI}$ (m = 0, 1 oder 2), 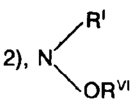 ,

Alkyl mit 1 bis 4 C-Atomen, Phenyl, Naphthyl, Alkenyl, Alkinyl, Alkenoxy, Alkinoxy mit jeweils bis zu 4 C-Atomen, Aralkyl mit 7 bis 14 C-Atomen, Acyl mit 1 bis 4 C-Atomen, Alkylen oder Dioxyalkylen mit jeweils bis zu 4 C-Atomen enthält, wobei die vorgenannten Alkyl- und Arylsubstituenten ihrerseits wieder durch Halogen, $COOR^{V}$ oder

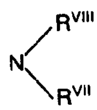

substituiert sein können, und wobei in den vorgenannten Definitionen der Substituenten $R^1$ bis $R^7$ $R^I$ für Wasserstoff, Alkyl mit 1 bis 6 C-Atomen, Phenyl, Naphthyl, Aralkyl mit 7 bis 12 C-Atomen, Heteroaryl oder Acyl mit bis zu 7 C-Atomen steht, $R^{II}$ und $R^{III}$ gleich oder verschieden sind und jeweils für Alkyl mit 1 bis 6 C-Atomen, Phenyl, Naphthyl, Aralkyl mit 7 bis 12 C-Atomen oder Heteroaryl stehen,
$R^{VII}$, $R^{V}$ und $R^{VI}$ jeweils gleich oder verschieden sind und für Wasserstoff, Alkyl mit 1 bis 6 C-Atomen, Phenyl, Naphthyl, Aralkyl mit 7 bis 12 C-Atomen oder Heteroaryl stehen,
$R^{VII}$ und $R^{VIII}$ jeweils gleich oder verschieden sind und für Wasserstoff, Phenyl, Naphthyl oder Aralkyl mit 7 bis 12 C-Atomen stehen oder für Alkyl mit 1 bis 6 C-Atomen, welches gegebenenfalls durch O, S oder $NR^I$ unterbrochen ist, stehen oder gemeinsam mit dem Stickstoffatom einen 5- bis 7-gliedrigen Ring bilden, der 1 oder 2 gleiche oder verschiedene Heteroringglieder aus der Gruppe O, S oder $NR^I$ enthalten kann oder
wobei einer der Reste $R^{VII}$ und $R^{VIII}$ eine aliphatische Acylgruppe mit bis zu 6 Kohlenstoffatomen steht,
$R^{IX}$, $R^{X}$, $R^{XII}$ und $R^{XIII}$ jeweils gleich oder verschieden sind und für Alkyl mit 1 bis 6 C-Atomen, Phenyl, Naphthyl oder Aralkyl mit 7 bis 12 C-Atomen stehen,
wobei die unter $R^1$ bis $R^8$ und unter $R^I$ bis $R^{VIII}$ genannten Alkyl-, Aryl-, Aralkyl-, Heteroaryl- und Acylreste sowie der mit $R^{VII}$ und $R^{VIII}$ gebildete Heteroring ihrerseits gegebenenfalls substituiert sind durch OH, $CF_3$, $OCF_3$, CN, $NO_2$, Halogen, Alkyl mit 1 bis 4 C-Atomen, Alkoxy mit 1 bis 4 C-Atomen, Phenyl- oder Benzyl und wobei als Heteroaryl folgende Substituenten genannt seien:

Thienyl, Furyl, Pyryl, Pyridyl, Chinolyl, Isochinolyl, Pyrimidyl, Pyridazinyl, Chinazolyl, Chinoxalyl, Benzothienyl, Pyrazolyl, Imidazolyl, Oxazolyl, Isoxazolyl, Thiazolyl, Triazolyl, Oxydiazolyl, Pyrazinyl, Oxazinyl, Thiazinyl, Indolizinyl, Indolyl, Benzofuranyl, Indazolyl, Benzothienyl, Benzimidazolyl, Benzoxazolyl, Benzisoxazolyl, Benzthiazolyl, Benztriazolyl, Benzoxadiazolyl, Cinnolinyl, Phthalazinyl, Naphthyridinyl oder Benzotriazinyl, in Form von Isomeren, Isomerengemischen, Racematen und optischen Antipoden sowie ihrer pharmazeutisch unbedenklichen Salze. Von besonderem Interesse sind Verbindungen der allgemeinen Formel (I), in welcher n für 0 oder 1 steht,

$R^1$

a) für Wasserstoff, einen geradkettigen, verzweigten cyclischen gesättigten oder ungesättigten aliphatischen Kohlenwasserstoffrest mit bis zu 10 C-Atomen steht, der gegebenenfalls durch ein oder zwei gleiche oder verschiedene Heterokettenglieder aus der Gruppe O, CO, S, = N- oder $NR^I$ enthält und wobei dieser Kohlenwasserstoffrest gegebenenfalls substituiert ist durch F, Cl, Br, $NO_2$, CN, OH, Phenyl oder Pyridyl oder

b) für einen Phenyl-, Naphthyl- oder Pyridylrest steht, wobei diese Reste gegebenenfalls 1 oder 2 gleiche oder verschiedene Substituenten aus der Gruppe Phenyl, Alkyl, Alkenyl, Alkoxy, Alkenoxy mit jeweils bis zu 4 C-Atomen, Benzyl, Acetyl, Alkylen, Dioxyalkylen mit 2 bis 4 C-Atomen, Fluor, Chlor, Brom, $CF_3$, $OCF_3$, $SCF_3$, $NO_2$, CN, $COOR^{V}$, $OR^{VI}$, $NR^I$ oder $NR^{VII}R^{VIII}$ tragen, wobei die Alkyl-, Alkoxy- und Arylreste der o.g. Substituenten ihrerseits wieder Halogen substituiert sein können oder

c) für den Rest $NR^{VII}R^{VIII}$ steht,

und wobei die unter a), b) und c) genannten Reste $R^I$ bis $R^{VIII}$ die unten angegebene Bedeutung haben,

$R^2$

a) für einen der unter $R^1$ angegebenen Substituenten steht aber nicht mit diesen identisch sein muss oder

b) für die Reste $NHR^I$ oder

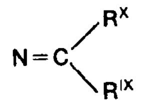

steht,

wobei $R^I$, $R^{IX}$ und $R^X$ die unten angegebene Bedeutung besitzen

oder die Substituenten

$R^1$ und $R^2$ gemeinsam einen 5- bis 7-gliedrigen gesättigten oder ungesättigten Ring bilden, der gegebenenfalls 1 oder 2 gleiche oder verschiedene Ringglieder aus der Gruppe O, S, $NR^I$ oder CO enthält und der gegebenenfalls 1 oder 2 gleiche oder verschiedene Substituenten aus der Gruppe Halogen, Hydroxy, Alkyl, Alkoxy mit je 1 bis 4 C-Atomen, Phenyl oder Benzyl enthält,

$R^3$ für einen der unter $R^2$ angegebenen Substituenten steht und mit diesem gleich oder von diesem verschieden ist, wobei nur einer der beiden Substituenten $R^2$ oder $R^3$ jeweils für Alkoxy, Alkylthio oder $NHR^I$ steht,

R4

a) für Wasserstoff, $NO_2$ CN, $SR^{XI}$, $SO_2R^{XI}$,

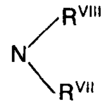 , 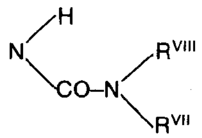

oder 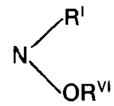

steht, wobei $R^I$, $R^{VI}$, $R^{VII}$, $R^{VIII}$ und $R^{IX}$ die unten angegebene Bedeutung haben,

b) für einen verzweigten oder unverzweigten Alkyl- oder Cycloalkylrest mit bis zu 8 C-Atomen steht, der gegebenenfalls substituiert ist durch Halogen, Hydroxy, Cyano, Alkoxy mit 1 bis 4 C-Atomen, Phenyloxy, $COOR^V$ oder

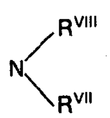

wobei $R^V$, $R^{VII}$ und $R^{VIII}$ die unten angegebene Bedeutung haben oder

c) für einen aromatischen Kohlenwasserstoffrest mit 6 bis 10 C-Atomen oder für einen 5- bis 7-gliedrigen gesättigten oder ungesättigten Heteroring mit 1 bis 3 gleichen oder verschiedenen Heterogliedern aus der Gruppe O, S, =N-, $NR^I$, wobei dieser Heteroring entweder über ein Kohlenstoffatom oder ein Stickstoffatom mit dem Dihydropyridinring verknüpft ist und wobei der aromatische Kohlenwasserstoffrest und die Heteroringe gegebenenfalls 1 oder 2 gleiche oder verschiedene Substituenten aus der Gruppe Halogen, Hydroxy, Cyano, $CF_3$, $NO_2$, Phenyl, Alkyl und Alkoxy mit je 1 bis 4 C-Atomen tragen,

oder

d) für den Rest

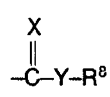

steht, wobei X Sauerstoff oder $NR^I$ und Y für eine einfache Bindung, Sauerstoff oder $NR^I$ steht und $R^8$ für für $R^1$ angegebene Bedeutung hat und mit dem Substituenten gleich oder von diesem verschieden ist,

oder

e) für den Rest

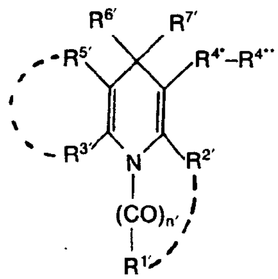

steht, wobei n', $R^{1'}$, $R^{2'}$, $R^{3'}$, $R^{5'}$, $R^{6'}$und $R^{7'}$die für n, $R^1$, $R^2$, $R^3$, $R^5$, $R^6$ und $R^7$ angegebene Bedeutung haben und mit diesen gleich oder von diesen verschieden sind, und

$R^{4*}$ und $R^{4**}$ gleich oder verschieden sind und jeweils für einen um ein Wasserstoff verminderten Rest der für $R^4$ unter a) bis d) angegebenen Substituenten stehen, oder

$R^2$ und $R^4$ gemeinsam einen verzweigten unverzweigten, gesättigten oder ungesättigten 5- bis 7-gliedrigen Ring bilden, der gegebenenfalls 1 oder 2 gleiche oder verschiedene Ringglieder aus der Gruppe O, CO, CS, C=NR$^I$, =N- oder NR$^I$ enthält und der gegebenenfalls substituiert ist durch Halogen oder Hydroxy,

wobei $R^1$ die unten angegebene Bedeutung hat,

$R^5$ die für $R^4$ angegebene Bedeutung besitzt und mit $R^4$ gleich oder von diesen verschieden ist oder

$R^5$ und $R^3$ gemeinsam einen Ring bilden, wie er für $R^2$ und $R^4$ definiert ist und mit diesem gleich oder von diesem verschieden ist,

$R^6$ für Wasserstoff oder Alkyl mit 1 bis 4 C-Atomen steht, welches gegebenenfalls durch Fluor, Chlor oder Brom substituiert ist und

$R^7$

a) für einen gesättigten, ungesättigten cyclischen, geradkettigen oder verzweigten aliphatischen Kohlenwasserstoffrest mit bis zu 8 C-Atomen steht, der gegebenenfalls substituiert ist durch Halogen, oder

b) für einen Phenyl- oder Heteroarylrest steht, der gegebenenfalls 1 bis 3 gleiche oder verschiedene Substituenten aus der Gruppe $NO_2$, CN, $N_3$, $CF_3$, Halogen, COR$^{IV}$ COR$^V$ OR$^{VI}$ SR$^{XI}$

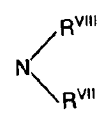

Phenyl, Alkyl mit 1 bis 4 C-Atomen, Benzyl oder Acyl mit 1 bis 4 C-Atomen enthält,

wobei in den vorgenannten Definitionen der Substituenten $R^1$ bis $R^8$

$R^I$ für Wasserstoff, Alkyl mit 1 bis 6 C-Atomen, Phenyl, Naphthyl, Benzyl, Phenethyl, Heteroaryl oder Acyl mit bis zu 4 C-Atomen steht,

$R^{II}$ und $R^{III}$ gleich oder verschieden sind und jeweils für Alkyl mit 1 bis 6 C-Atomen, Phenyl, Naphthyl, Benzyl oder Heteroaryl stehen,

$R^{IV}$, $R^V$ und $R^{VI}$ jeweils gleich oder verschieden sind und für Wasserstoff, Alkyl mit 1 bis 6 C-Atomen, Phenyl, Naphthyl, Benzyl oder Heteroaryl stehen,

$R^{VII}$ und $R^{VIII}$ die jeweils gleich oder verschieden sind und für Wasserstoff, Phenyl oder Benzyl stehen oder für Alkyl mit 1 bis 6 Kohlenstoffatomen stehen, welches gegebenenfalls durch O oder NR$^I$ unterbrochen ist, oder

gemeinsam mit dem Stickstoffatom einen 5- bis 7-gliedrigen Ring bilden, der 1 oder 2 gleiche verschiedene Heteroringglieder aus der Gruppe O, S oder NR$^I$ enthalten kann,

oder

wobei einer der Reste $R^{VII}$ oder $R^{VIII}$ für eine aliphatische Acylgruppe mit bis zu 6 Kohlenstoffatomen steht und

$R^{IX}$, $R^X$, $R^{XI}$, $R^{XII}$ und $R^{XIII}$ jeweils gleich oder verschieden sind und für Alkyl mit 1 bis 6 C-Atomen, Phenyl oder Benzyl stehen,

wobei als Heteroaryl folgende Substituenten genannt seien:

Thienyl, Furyl, Pyryl, Pyridyl, Chinolyl, Isochinolyl, Pyrimidyl, Pyridazinyl, Chinazolyl, Chinoxalyl, Benzothienyl, Pyrazolyl, Imidazolyl, Oxazolyl, Isoxazolyl, Thiazolyl, Triazolyl, Oxydiazolyl, Pyrazinyl, Oxazinyl, Thiazinyl, Indolyl, Benzofuranyl, Indazolyl, Benzothienyl, Benzimidazolyl, Benzoxazolyl, Benzisoxazolyl, Benzthiazolyl, Benztriazolyl, oder Benzoxadiazolyl.

Insbesondere seien Verbindungen der allgemeinen Formel (I) genannt, in welcher

n für 0 steht,

$R^1$

a) für Wasserstoff, einen aliphatischen Kohlenwasserstoffrest mit bis zu 6 C-Atomen, der gegebenenfalls ein Heterokettenglied aus der Gruppe O, CO, = N- oder $NR^I$ enthält und der gegebenenfalls substituiert ist durch Halogen, Nitro, Hydroxy oder Phenyl oder

b) für einen Phenyl- oder Pyridylrest steht, die gegebenenfalls substituiert sind durch Halogen, $NO_2$, $CF_3$, $OCF_3$, CN, $COOR^V$ oder $NR^{VII}R^{VIII}$,

$R^2$ für einen der unter $R^1$ angegebenen Substituenten steht, aber nicht mit diesem identisch sein muss oder für einen der Reste $NHR^I$ oder $N = CR^XR^{XI}$ steht,

$R^3$ für einen der unter $R^2$ angegebenen Substituenten steht und mit diesem gleich oder von diesem verschieden ist,

$R^4$

a) für Wasserstoff, $NO_2$, $NR^{VII}R^{VIII}$, NH-CO-$NR^{VII}R^{VIII}$ oder Halogen steht, oder

b) für einen Alkylrest mit 1 bis 4 C-Atomen steht, der gegebenenfalls substituiert ist durch Halogen, OH, CN, Alkoxy mit 1 bis 4 C-Atomen, $COOR^V$ oder $NR^{VII}R^{VIII}$ oder

c) für Phenyl, Pyridyl oder Thienyl steht, die gegebenenfalls substituiert sind durch Halogen, OH, CN, Alkyl oder Alkoxy mit jeweils 1 bis 4 C-Atomen oder durch $NR^{VII}R^{VIII}$ oder

d) für den Rest

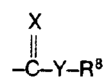

steht,

wobei X Sauerstoff bedeutet und Y für eine einfache Bindung, Sauerstoff oder $NR^I$ steht und $R^8$ die für $R^1$ angegebene Bedeutung hat und mit dem Substituenten $R^1$ gleich oder von diesem verschieden ist oder

e) für den Rest

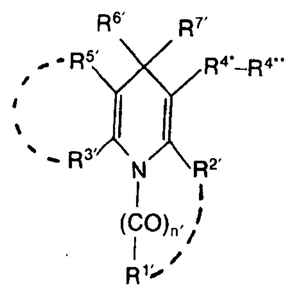

steht,

wobei n', $R^{1'}$, $R^{2'}$, $R^{3'}$, $R^{5'}$, $R^{6'}$ und $R^{7'}$ die für n, $R^1$, $R^2$, $R^3$, $R^5$, $R^6$ und $R^7$ angegebene Bedeutung haben und mit diesen gleich oder von diesen verschieden sind, und

$R^{4*}$ und $R^{4**}$ gleich oder verschieden sind und jeweils für einen um ein Wasserstoff verminderten Rest der für $R^4$ unter a) bis d) angegebenen Substituenten stehen, oder

$R^2$ und $R^4$ gemeinsam einen 5- bis 7-gliedrigen Ring bilden, der gegebenenfalls 1 oder 2 verschiedene Ringglieder aus der Gruppe O, CO, CS oder C = $NR^I$ enthält und der gegebenenfalls substituiert ist durch Halogen,

$R^5$ die für $R^4$ angegebene Bedeutung besitzt und mit $R^4$ gleich oder von diesem verschieden ist,

$R^6$ für Wasserstoff oder Alkyl mit 1 bis 4 C-Atomen steht und $R^7$ die oben angegebene Bedeutung besitzt, wobei in den vorgenannten Definitionen die mit römischen Ziffern gekennzeichneten Substituenten $R^I$ bis $R^{VIII}$ die oben angegebene Bedeutung besitzen.

Besonders hervorgehoben seien Verbindungen, in denen wenigstens einer der Substituenten $R^4$ und $R^5$ für $NO_2$ stehen,

und/oder

in denen $R^2$ und $R^4$ gemeinsam oder $R^3$ und $R^5$ gemeinsam einen Lactonring bilden.

Die Herstellung der erfindungsgemässen 1,4-Dihydropyridinderivate mit positiv inotroper Wirkung erfolgt nach üblichen Methoden, die für die Herstellung von 1,4-Dihydropyridinen bekannt sind (vgl. z.B. britisches Patent 1 305 793; britisches Patent 1 358 951; DE-OS 2 752 820; DE-OS 2 847 237; DE-OS 2 629 892; DE-OS 2 658 804).

Einige der erfindungsgemäss verwendbaren Verbindungen sind bereits aus dem Stand der Technik bekannt. Die Verbindungen der Herstellungsbeispiele 1 bis 10, 19 bis 23, 26, 28, 29, 30, 31, 32, 35 bis 38, 41, 42, 43, 45 und 46 bis 254 sind neu. Diese neuen Verbindungen werden z.T. durch allgemeine Substituentendefinitionen des Standes der Technik umfasst ohne jedoch bisher namentlich genannt worden zu sein.

Neu sind auch solche Verbindungen aus der allgemeinen Formel 1, in denen mindestens einer der Substituenten $R^4$ und $R^5$ für die Gruppe

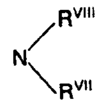

steht, wobei $R^{VIII}$ und $R^{VII}$ und die Substituenten $R^1$, $R^2$, $R^3$, $R^6$ und $R^7$ und n die oben angegebene Bedeutung haben, und solche Verbindungen aus der allgemeinen Formel I, in denen mindestens einer der Substituenten $R^4$ und $R^5$ für Methyl steht, wobei $R^1$, $R^2$, $R^3$, $R^6$ und $R^7$ und n die oben angegebene Bedeutung haben, und solche Verbindungen aus der allgemeinen Formel I, in denen einer der Reste $R^4$ oder $R^5$ Wasserstoff bedeutet, $R^1$ für Wasserstoff und n für 0 steht, und $R^2$, $R^3$, $R^6$ und $R^7$, sowie der andere Rest von $R^4$ oder $R^5$ die oben angegebene Bedeutung haben, und solche Verbindungen aus der allgemeinen Formel 1, in denen einer der Reste $R^4$ oder $R^5$ Halogen bedeutet, und $R^1$, $R^2$, $R^3$, $R^6$, $R^7$ und n, sowie der andere Rest von $R^4$ oder $R^5$ die oben angegebene Bedeutung haben.

Die vorliegende Erfindung betrifft auch die neuen Verbindungen unter der allgemeinen Formel (I), ihre Herstellung und ihre Verwendung in Arzneimitteln mit positiv inotroper Wirkung.

Verbindungen der allgemeinen Formel (I), in denen $R^2$ von $R^3$ oder $R^4$ von $R^5$ verschieden ist, können aufgrund der Anwesenheit eines asymmetrischen Kohlenstoffatoms in 4-Position des Dihydropyridinringes als racemische Mischungen oder in Form von optischen Isomeren erhalten werden. Einige der erfindungsgemässen Verbindungen die mindestens 2 asymmetrische Kohlenstoffatome enthalten, können in Form einzelner Diastereomeren oder in Form der Mischungen davon erhalten werden. Die diastereomere Mischung kann unter Anwendung konventioneller Verfahren aufgetrennt werden. Beispielsweise durch fraktionierte Umkristallisation oder durch chromatographische Verfahren. Racemische Mischungen lassen sich nach üblichen Methoden, beispielsweise durch Aufspaltung, durch fraktionierte Umkristallisation eines Salzes mit optisch aktiven Säuren in die jeweiligen optischen Isomeren auftrennen.

Durch die Definition der Substituenten der allgemeinen Formel (I) werden auch einige Verbindungen umfasst, die keine positiv inotrope Wirkung besitzen. Durch die oben angegebene Testmethode am isolierten Meerschweinchenvorhof ist es für den Fachmann jedoch unproblematisch, die erfindungsgemässen Verbindungen mit positiv inotroper Wirkung zu erkennen und von den evtl. umfassten Verbindungen mit negativ inotroper Wirkung zu unterscheiden. Die vorliegende Anmeldung ist auf solche Verbindungen gerichtet, die ab einer Konzentration von $10^{-5}$ g/ml im oben angegebenen Test am linken isolierten Vorhof des Meerschweinchenherzens eine Kontraktionsverstärkung um mindestens 25% bewirken.

Die folgende Tabelle zeigt beispielhaft die positiv inotrope bzw. kontraktionskraftverstärkende Wirkung um mindestens 25% einiger der erfindungsgemässen Verbindungen.

Tabelle

| Beispiel Nr. | Wirkstoffkonzentration in g/ml | Beispiel Nr. | Wirkstoffkonzentration in g/ml |
|---|---|---|---|
| 1 | $10^{-7}$ | 12 | $10^{-6}$ |
| 2 | $10^{-5}$ | 13 | $10^{-6}$ |
| 3 | $10^{-6}$ | 14 | $10^{-6}$ |
| 4 | $3 \times 10^{-7}$ | 15 | $10^{-5}$ |
| 5 | $10^{-5}$ | 16 | $10^{-6}$ |
| 6 | $10^{-7}$ | 17 | $10^{-7}$ |
| 7 | $10^{-7}$ | 18 | $3 \times 10^{-6}$ |
| 8 | $10^{-6}$ | 19 | $10^{-5}$ |
| 9 | $10^{-7}$ | 20 | $3 \times 10^{-7}$ |
| 10 | $10^{-7}$ | 21 | $10^{-7}$ |
| 11 | $10^{-5}$ | 22 | $10^{-6}$ |

Tabelle (Fortsetzung)

| Beispiel Nr. | Wirkstoff-konzentration in g/ml | Beispiel Nr. | Wirkstoff-konzentration in g/ml |
|---|---|---|---|
| 23 | $3 \times 10^{-7}$ | 148 | $10^{-7}$ |
| 24 | $10^{-5}$ | 149 | $10^{-7}$ |
| 25 | $10^{-6}$ | 152 | $10^{-7}$ |
| 26 | $10^{-7}$ | 156 | $3 \times 10^{-7}$ |
| 27 | $10^{-7}$ | 157 | $10^{-6}$ |
| 29 | $10^{-7}$ | 158 | $10^{-7}$ |
| 31 | $10^{-6}$ | 159 | $10^{-6}$ |
| 32 | $10^{-7}$ | 164 | $10^{-6}$ |
| 33 | $10^{-7}$ | 167 | $10^{-5}$ |
| 35 | $10^{-6}$ | 170 | $10^{-6}$ |
| 36 | $10^{-6}$ | 173 | $10^{-7}$ |
| 37 | $10^{-6}$ | 174 | $10^{-6}$ |
| 38 | $10^{-6}$ | 175 | $10^{-5}$ |
| 40 | $10^{-6}$ | 177 | $10^{-6}$ |
| 41 | $10^{-6}$ | 179 | $3 \times 10^{-7}$ |
| 42 | $10^{-5}$ | 181 | $10^{-6}$ |
| 43 | $10^{-6}$ | 186 | $10^{-7}$ |
| 44 | $10^{-6}$ | 187 | $10^{-7}$ |
| 45 | $10^{-6}$ | 189 | $3 \times 10^{-7}$ |
| 49 | $10^{-6}$ | 190 | $10^{-6}$ |
| 55 | $10^{-7}$ | 191 | $10^{-6}$ |
| 56 | $10^{-6}$ | 193 | $10^{-7}$ |
| 59 | $10^{-6}$ | 195 | $3 \times 10^{-6}$ |
| 60 | $3 \times 10^{-6}$ | 196 | $10^{-5}$ |
| 64 | $10^{-6}$ | 197 | $3 \times 10^{-6}$ |
| 65 | $10^{-6}$ | 198 | $3 \times 10^{-7}$ |
| 67 | $3 \times 10^{-7}$ | 199 | $3 \times 10^{-7}$ |
| 68 | $3 \times 10^{-7}$ | 200 | $10^{-7}$ |
| 69 | $10^{-7}$ | 202 | $10^{-7}$ |
| 72 | $10^{-7}$ | 203 | $10^{-6}$ |
| 73 | $10^{-7}$ | 208 | $10^{-7}$ |
| 77 | $10^{-6}$ | 210 | $3 \times 10^{-6}$ |
| 78 | $3 \times 10^{-6}$ | 211 | $10^{-6}$ |
| 79 | $10^{-7}$ | 212 | $3 \times 10^{-7}$ |
| 80 | $10^{-7}$ | 214 | $3 \times 10^{-6}$ |
| 86 | $10^{-6}$ | 215 | $3 \times 10^{-7}$ |
| 88 | $10^{-6}$ | 216 | $10^{-6}$ |
| 92 | $10^{-6}$ | 217 | $10^{-5}$ |
| 93 | $10^{-6}$ | 218 | $10^{-5}$ |
| 94 | $10^{-6}$ | 219 | $10^{-5}$ |
| 98 | $10^{-7}$ | 220 | $10^{-6}$ |
| 99 | $10^{-5}$ | 221 | $10^{-6}$ |
| 101 | $10^{-6}$ | 222 | $10^{-6}$ |
| 102 | $10^{-6}$ | 223 | $3 \times 10^{6}$ |
| 104 | $3 \times 10^{-7}$ | 224 | $10^{-6}$ |
| 105 | $10^{-7}$ | 225 | $10^{-6}$ |
| 108 | $10^{-5}$ | 226 | $10^{-6}$ |
| 111 | $3 \times 10^{-6}$ | 227 | $10^{-6}$ |
| 112 | $10^{-7}$ | 228 | $10^{-6}$ |
| 114 | $10^{-6}$ | 229 | $10^{-5}$ |
| 117 | $10^{-6}$ | 230 | $10^{-7}$ |
| 118 | $10^{-7}$ | 231 | $10^{-6}$ |
| 124 | $10^{-7}$ | 232 | $10^{-6}$ |
| 126 | $10^{-6}$ | 233 | $10^{-6}$ |
| 132 | $10^{-7}$ | 234 | $10^{-6}$ |
| 144 | $10^{-7}$ | 235 | $3 \times 10^{-}5$ |
| 145 | $10^{-7}$ | 236 | $10^{-6}$ |
| 146 | $10^{-6}$ | 239 | $3 \times 10^{-7}$ |
| 147 | $10^{-7}$ | 240 | $3 \times 10^{-6}$ |

Tabelle (Fortsetzung)

| Beispiel Nr. | Wirkstoff-konzentration in g/ml | Beispiel Nr. | Wirkstoff-konzentration in g/ml |
|---|---|---|---|
| 241 | $10^{-7}$ | 248 | $3 \times 10^{-7}$ |
| 242 | $3 \times 10^{-7}$ | 250 | $3 \times 10^{-7}$ |
| 245 | $3 \times 10^{-7}$ | 251 | $10^{-7}$ |
| 246 | $3 \times 10^{-7}$ | 257 | $3 \times 10^{-7}$ |
| 247 | $10^{-6}$ | | |

Die erfindungsgemässen Verbindungen zeigen ein nicht vorhersehbares und wertvolles pharmakologisches Wirkungsspektrum. Sie können als Cardiotonika zur Verbesserung der Herzkontraktilität dienen. Darüber hinaus können sie dadurch, dass sie den $Ca^{++}$-Einstrom in die Zellen erhöhten, als Antihypotonica, zur Senkung von Blutzukker, zur Abschwellung von Schleimhäuten und zur Beeinflussung des Salz- und Flüssigkeitshaushaltes eingesetzt werden.

Die erfindungsgemässen Verbindungen können in bekannter Weise in die üblichen Formulierungen überführt werden.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d. h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z. B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z. B. Erdölfraktionen), pflanzliche Öle (z. B. Erdnuss-/Sesamöl), Alkohole (z. B. Ethylalkohol, Glycerin), Glykole (z. B. Propylenglykol, Polyethylenglykol), feste Trägerstoffe, wie z. B. natürliche Gesteinsmehle (z. B. Kaoline, Tonerden, Talkum, Kreide), synthetische Gesteinsmehle (z. B. hochdisperse Kieselsäure, Silikate), Zucker (z. B. Rohr-, Milch- und Traubenzucker), Emulgiermittel (z. B. Polyoxyethylen-Fettsäure-Ester, Polyoxyethylen-Fettalkohol-Ether, Alkylsulfonate und Arylsulfonate), Dispergiermittel (z. B. Lignin, Sulfitablaugen, Methylcellulose, Stärke und Polyvinylpyrrolidon) und Gleitmittel (z. B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich ausser den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wässriger Suspensionen und/oder Elixieren, die für orale Anwendungen gedacht sind, können die Wirkstoffe ausser den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. deren Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation grösserer Mengen kann

es empfehlenswert sein, diese in mehrere Einzelgaben über den Tag zu verteilen. Für die Applikation in der Humanmedizin ist der gleiche Dosierungsspielraum vorgesehen. Sinngemäss gelten hierbei auch die obigen Ausführungen.

Herstellungsbeispiele

Beispiel 1

2-Methyl-4-(2-trifluormethylphenyl)-5-oxo-1,4-dihydro-5,7-dihydrofuro[3,4-b]pyridin-3-carbonsäureethyl ester

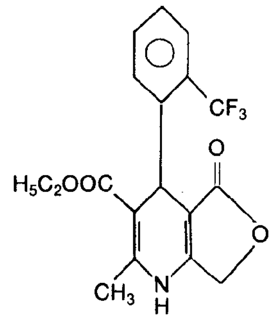

Je 50 mmol 4-Acetoxy-3-oxo-buttersäureethylester, 3-Aminocrotonsäureethylester und 2-Trifluormethylbenzaldehyd wurden in 100 ml Ethanol 24 h am Rückfluss gekocht, anschliessend mit 2 g Kaliumhydroxid versetzt und eine weitere Stunde gekocht. Nach Abkühlung wurde mit Wasser/Kochsalz-Gemisch ausgefällt und aus Methanol umkristallisiert.
Ausbeute: 45% der Theorie; Fp.: 195 °C.

Beispiel 2

2-Methyl-4-(3-methoxyphenyl)-5-oxo-1-4-dihydro-5,7-dihydrofuro[3,4-b]pyridin-3-carbonsaüreethylester

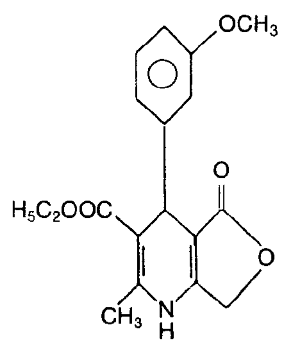

Darstellung analog Beispiel 1 mit 3-Methoxybenzaldehyd statt 2-Trifluormethylbenzaldehyd.
Ausbeute: 30% der Theorie; Fp.: 180 °C.

Beispiel 3

2-Methyl-4-(3-chlorphenyl)-5-oxo-1,4-dihydro-5,7-dihydrofuro[3,4-b]pyridin-3-carbonsäureethylester

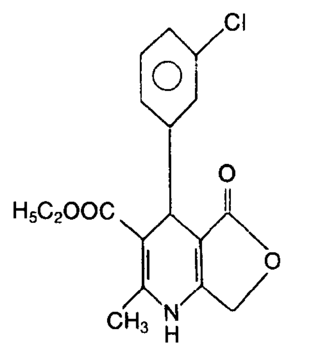

Darstellung analog Beispiel 1 mit 3-Chlorbenzaldehyd statt 2-Trifluormethylbenzaldehyd.
Ausbeute: 50% der Theorie; Fp.: 196 °C.

Beispiel 4

2-Methyl-4-(3-chlorphenyl)-5-oxo-1,4-dihydro-5,7-dihydrofuro[3,4-b]pyridin-3-carbonsäurepropylester

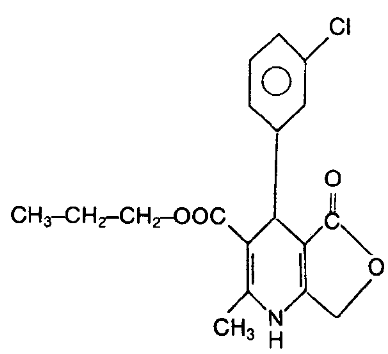

Darstellung analog Beispiel 1 3-Chlorbenzaldehyd statt 2-Trifluormethylbenzaldehyd und 3-Aminocrotonsäurepropylester statt 3-Aminocrotonsäureethylester.
Ausbeute: 42% der Theorie; Fp.: 166 °C.

Beispiel 5

2-Methyl-4-(3-nitrophenyl)-5-oxo-1,4-dihydropyridin-5,7-dihydrofuro[3,4-b]pyridin-3-carbonsäurepropylester

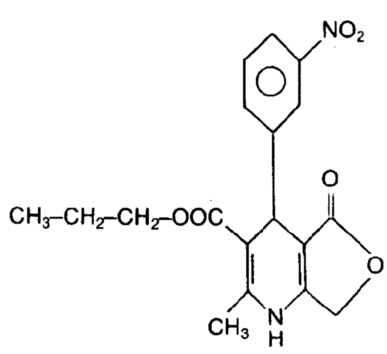

Darstellung analog Beispiel 1 mit 3-Nitrobenzaldehyd statt 2-Trifluormethylbenzaldehyd und 3-Aminocrotonsäurepropylester statt -ethylester.
Ausbeute: 50% der Theorie; Fp.: 196 °C.

Beispiel 6

2-Methyl-4-(3-trifluormethylphenyl)-5-oxo-1,4-dihydro-5,7-dihydrofuro[3,4-b]pyridin-3-carbonsäure-isopropylester

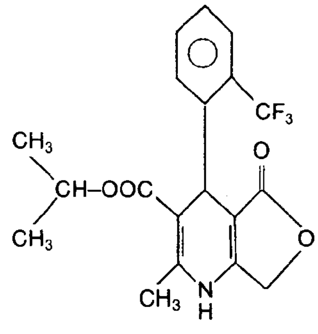

Darstellung analog Beispiel 1 mit 3-Aminocrotonsäureisopropylester statt -ethylester.
Ausbeute: 18% der Theorie; Fp.: 219-223 °C.

Beispiel 7

2-Methyl-4-(2-trifluormethylphenyl)-5-oxo-1,4-dihydro-5,7-dihydrofuro[3,4-b]pyridin-3-carbonsäurebutylester

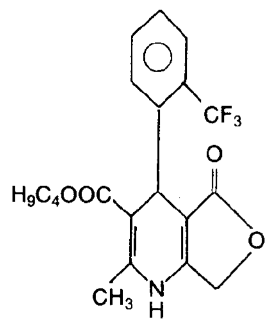

Darstellung analog Beispiel 1 mit 3-Aminocrotonsäurebutylester statt -ethylester.
Ausbeute: 10% der Theorie; Fp.: 194-195 °C.

Beispiel 8

2-Methyl-4-(2-trifluormethylphenyl)-5-oxo-1,4-dihydro-5,7-dihydrofuro[3,4-b]-pyridin-3-carbonsäure-(2-methoxy)-ethylester

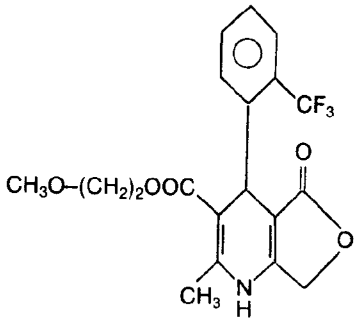

Darstellung analog Beispiel 1 mit 3-Aminocrotonsäure-(2-methoxy)-ethylester statt 3-Aminocrotonsäureethylester.
Ausbeute: 16% der Theorie; Fp.: 196-197 °C.

Beispiel 9

2-Methyl-4-(2-benzylthiophenyl)-5-oxo-1,4-dihydro-5,7-dihydrofuro[3,4-b]pyridin-3-carbonsäureethylester

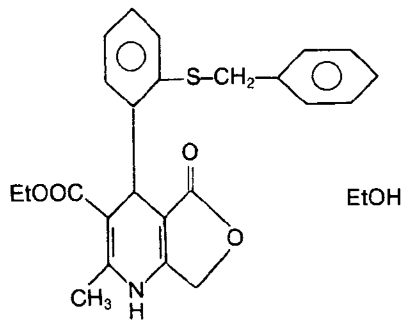

EtOH

Darstellung analog Beispiel 1 mit 2-Benzylthiobenzaldehyd anstatt 2-Trifluormethylbenzaldehyd.
Ausbeute: 31% der Theorie; Fp.: 189-190 °C (aus EtOH ).

Beispiel 10

2-Methyl-4-(2-methylphenyl)-5-oxo-1,4-dihydro-5,7-dihydrofuro[3,4-b]pyridin-3-carbonsäureethylester

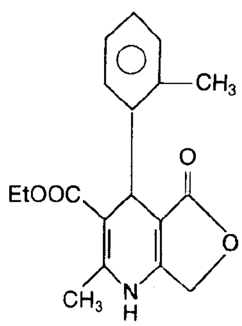

Darstellung analog Beispiel 1 mit 2-Methylbenzaldehyd anstatt 2-Trifluormethylbenzaldehyd.
Ausbeute: 45%, Fp. 196-198 °C.

Beispiel 11

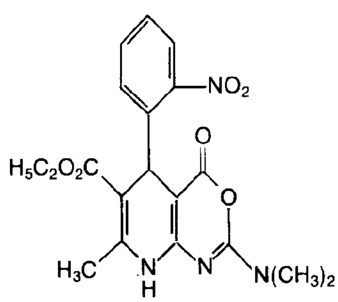

33,8 g (90 mmol) 2-Amino-1,4-dihydro-2,6-dimethyl-4-(2-nitrophenyl)-pyridin-3,5-dicarbonsäurediethylester wurden zusammen mit 14,6 g (90 mmol) Dichlormethylen-dimethyl-ammoniumchlorid in 200 ml Chlorbenzol 2 Stunden bei 80 °C gerührt. Anschliessend wurde mit Eis gekühlt, das ausgefallene Produkt abgesaugt und in einer kalten 10%igen Natriumbicarbonatlösung aufgenommen. Die wässrige Phase wurde mit Methylenchlorid

mehrmals extrahiert. Nach Trocknen der Extrakte über $Na_2SO_4$ und Abdestillation des Lösungsmittels resultierten 16,6 g (46% der Theorie) des Reaktionsproduktes vom Schmelzpunkt Fp.: 216-218 °C.

Beispiel 12

1,4-Dihydro-2-methyl-5-oxo-7,7-pentamethylen-4-(2′-nitrophenyl)-7H-pyrano[4,3-b]pyridin-3-carbonsäuremethylester

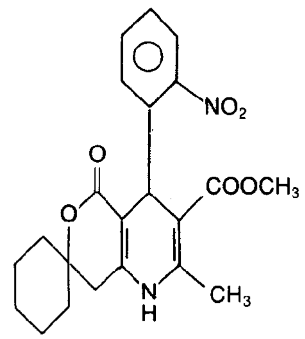

4,6 g 6,6-Pentamethylentetrahydropyrandion-2,4 3,8 g 2-Nitrobenzaldehyd und 2,9 g 3-Aminocrotonsäuremethylester werden in 100 ml Ethanol/Eisessig (5: 1) 10 Stunden unter Rückfluss gekocht. Man kühlt auf Raumtemperatur und saugt den Niederschlag ab. Aus Ethanol umkristallisiert werden 6 g (58% der Theorie) vom Fp.: 228 °C erhalten.

Beispiel 13

1,4-Dihydro-2,7-dimethyl-4-(2′-methylphenyl)-5-oxo-7H-pyrano[4,3-b]pyridin-3-carbonsäureethylester

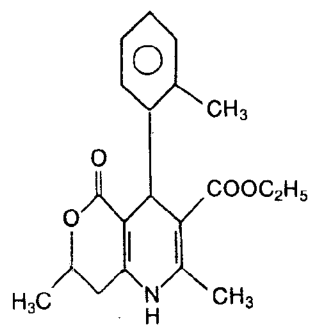

Eine Lösung von 4,3 g 6-Methyltetrahydropyrandion-2,4, 4,0 g 2-Methylbenzaldehyd und 4,3 g 3-Aminocrotonsäureethylester in 80 ml Ethanol/Eisessig (5: 1) wird 10 Stunden unter Rückfluss gekocht. Man engt im Vakuum ein und kristallisiert den Rückstand aus Ethanol um: 5,3 g (50% der Theorie) vom Fp.: 213 °C.
Analog wurden erhalten:

Beispiel 14

1,4-Dihydro-2,7-dimethyl-4-(3′-trifluormethylphenyl)-5-oxo-7H-pyrano[4,3-b]pyridin-3-carbonsäureethylester vom Fp.: 210 °C

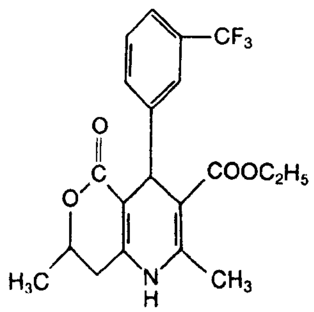

Beispiel 15

1,4-Dihydro-2,7-dimethyl-4-(2'-chlorphenyl)-5-oxo-7H-pyrano[4,3-b]pyridin-4-carbonsäuremethylester vom Fp.: 252-254 °C

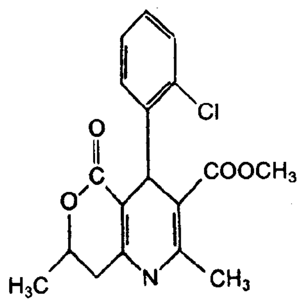

Beispiel 16

1,4-Dihydro-2,6-dimethyl-3-nitro-4-(3-nitrophenyl)-pyridin-5-carbonsäurecyclopentylester

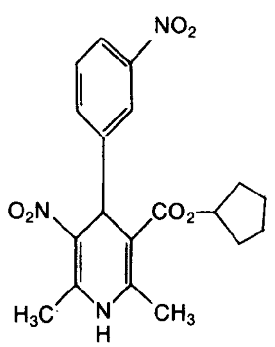

15,1 g (0,1 Mol) 3-Nitrobenzaldehyd werden zusammen mit 16,9 g (0,1 Mol) β-Aminocrotonsäurecyclopentylester und 10,3 g (0,1 Mol) Nitroaceton in 150 ml Ethanol 6 Stunden zum Rückfluss erhitzt. Nach dem Erkalten der Reaktionsmischung wird das Lösungsmittel im Vakuum abdestilliert, der ölige Rückstand in wenig Chloroform aufgenommen und auf einer Kieselgelsäule mit Chloroform unter Zusatz von Methanol chromatographiert. Die das Reaktionsprodukt enthaltenden Fraktionen werden eingeengt, der Rückstand in wenig Isopropanol, das Nitrodihydropyridin kristallisierte in gelben Kristallen vom Schmp. 174 °C.
Ausbeute: 37% der Theorie.

Beispiel 17

1,4-Dihydro-2,6-dimethyl-3-nitro-4-(3-nitrophenyl)-pyridin-5-carbonsäure-(β-n-propoxyethyl)-ester

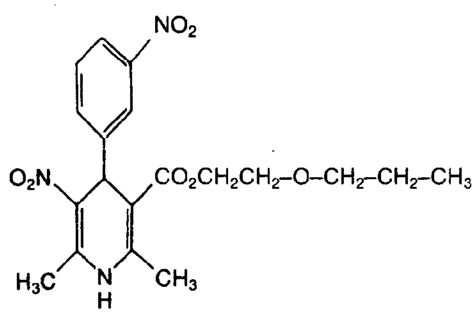

15,1 g (0,1 Mol) 3-Nitrobenzaldehyd werden zusammen mit 18,8 g (0,1 Mol) Acetessigsäure-(β-n-propoxy-ethyl)-ester und 10,2 g (0,1 Mol) 2-Amino-1-nitro-1-propen in 150 ml Ethanol 6 Stunden zum Rückfluss erhitzt. Nach dem Erkalten wird das Lösungsmittel im Vakuum abdestilliert, der ölige Rückstand in wenig Chloroform aufgenommen und auf einer Kieselgelsäule mit Chloroform unter Zusatz von Methanol chromatographiert. Die das Produkt enthaltenden Fraktionen werden eingeengt, der Rückstand in wenig Isopropanol aufgenommen, das Nitrodihydropyridin kristallisierte in gelben Kristallen vom Schmp. 161 °C.
Ausbeute: 41 % der Theorie.

Beispiel 18

1,4-Dihydro-2,6-dimethyl-3,5-dinitro-4-(3-nitrophenyl)-pyridin

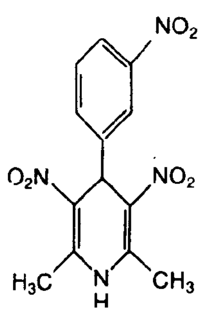

23,6 g (0,1 Mol) 2-Nitro-1-(3-nitrophenyl)-buten-1-on-3 und 10,2 g (0,1 Mol) 2-Amino-1-nitro-1-propen werden in 150 ml Ethanol 12 Stunden zum Rückfluss erhitzt. Nach dem Erkalten wird das Lösungsmittel im Vakuum abdestilliert, der ölige Rückstand wird in Chloroform aufgenommen und auf einer Kieselgelsäule mit Chloroform unter Zusatz von Methanol chromatographiert. Das Produkt bildet in Isopropanol gelbe Kristalle vom Schmp. 237-240 °C (Zers.).
Ausbeute: 38% der Theorie.

Beispiel 19

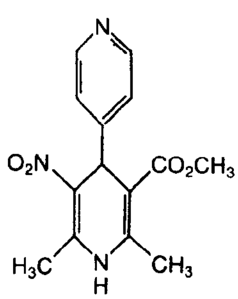

Analog Beispiel 16 wurde durch Umsetzung von 50 mmol Pyridin-4-aldehyd mit 50 mmol Acetessigsäuremethylester und 50 mmol 2-Amino-1-nitro-1-propen in Ethanol 1,4-Dihydro-2,6-dimethyl-4-(4-pyridyl)-3-nitropyridin-5-carbonsäuremethylester vom Schmp. 210 °C (Zers.) (Isopropanol) erhalten.

Ausbeute: 19% der Theorie.

Beispiel 20

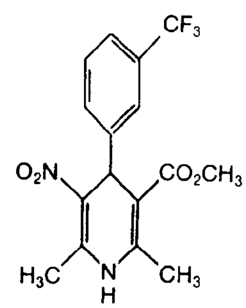

Analog Beispiel 16 wurde durch Umsetzung von 50 mmol 3-Trifluormethylbenzaldehyd mit 50 mmol Nitroaceton und 50 mmol β-Aminocrotonsäuremethylester
1,4-Dihydro-2,6-dimethyl-3-nitro-4-(3-trifluormethylphenyl)-pyridin-5-carbonsäuremethylester vom Schmp. 175 °C (Isopropanol) erhalten.
Ausbeute: 42% der Theorie.

Beispiel 21

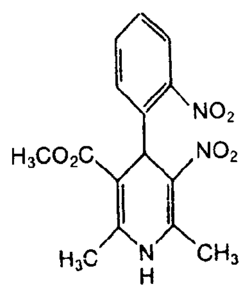

Analog Beispiel 16 wurde durch Umsetzung von 50 mmol 2-Nitrobenzaldehyd mit 50 mmol Nitroaceton und 50 mmol β-Aminocrotonsäuremethylester
1,4-Dihydro-2,6-dimethyl-3-nitro-4-(2-nitro-phenyl)-pyridin-5-carbonsäuremethylester vom Schmp. 190 °C (Isopropanol) als sehr lichtempfindliche Verbindung erhalten.
Ausbeute: 12% der Theorie.

Beispiel 22

1,4-Dihydro-2,6-dimethyl-3-nitro-4-(4-nitrophenyl)-pyridin-5-carbonsäure-(β-cyanoethyl)-ester

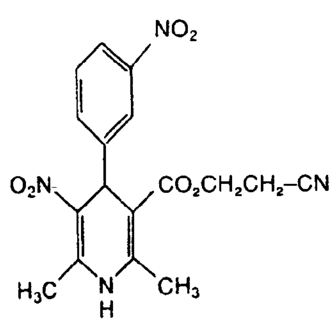

Analog Beispiel 18 wurde durch Umsetzung von 50 mmol 2-Nitro-1-(3-nitrophenyl)-buten-1-on-3 mit 50 mmol β-Aminocrotonsäure-β-cyanoethylester in 100 ml Ethanol
1,4-Dihydro-2,6-dimethyl-3-nitro-4-(3-nitrophenyl)-pyridin-5-carbonsäure-(β-cyanoethyl)-ester vom Schmp. 210 °C (Isopropanol) erhalten.

Ausbeute: 33% der Theorie.

Beispiel 23

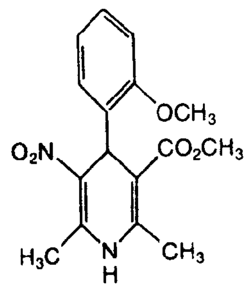

Analog Beispiel 18 wurde durch Umsetzung von 50 mmol 2-Methoxybenzylidenacetessigsäuremethylester und 50 mmol 2-Amino-1-nitrol-propen in Ethanol 1,4-Dihyro-2,6-dimethyl-4-(2-methoxyphenyl)-3-nitro-pyridin-5-carbonsäuremethylester vom Schmp. 206 °C (Ethanol) erhalten.
Ausbeute: 44% der Theorie.

Beispiel 24

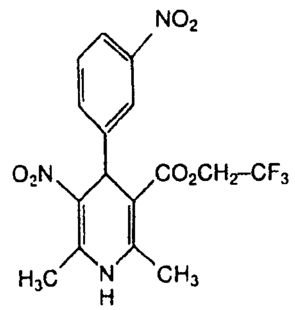

Analog Beispiel 18 wurde durch Umsetzung von 3-Nitro-benzylidenacetessigsäure-(β-trifluorethyl)-ester mit 2-Amino-1-nitro-1-propen in Ethanol 1,4-Dihydro-2,6-dimethyl-3-nitro-4-(3-nitrophenyl)-pyridin-5-carbonsäure-(β-trifluorethyl)-ester vom Schmp. 196 °C (Ethanol) erhalten.
Ausbeute: 28% der Theorie.

Beispiel 25

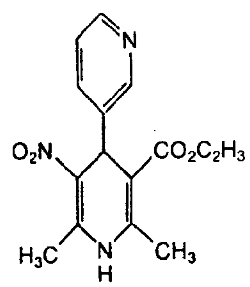

Analog Beispiel 16 wurde durch Umsetzung von Pyridin-3-aldehyd mit β-Aminocrotonsäureethylester und Nitroaceton in Ethanol 1,4-Dihydro-2,6-dimethyl-3-nitro-4-(pyridyl-3)-pyridin-5-carbonsäureethylester vom Schmp. 264 °C (Isopropanol) erhalten.
Ausbeute: 34% der Theorie.

Beispeil 26

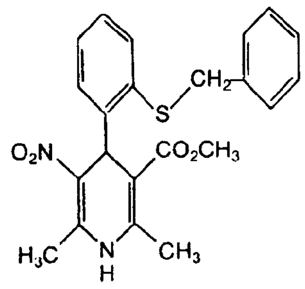

Analog Beispiel 17 wurde durch Umsetzung von 2-Benzylthiobenzaldehyd mit 2-Amino-1-nitro-1-propen und Acetessigsäuremethylester in Ethanol 4-((2-Benzylthiophenyl)-1,4-dihydro-2,6-dimethyl-3-nitropyridin-5-carbonsäuremethylester
vom Schmp. 173 °C (Isopropanol) erhalten.
Ausbeute: 21 % der Theorie.

Beispiel 27

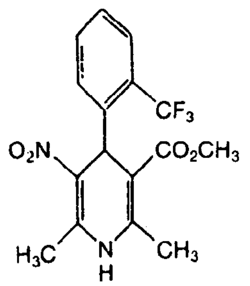

Analog Beispiel 16 wurde durch Umsetzung von 2-Trifluormethylbenzaldehyd mit β-Aminocrotonsäuremethylester und Nitroaceton in Ethanol 1,4-Dihydro-2,6-dimethyl-3-nitro-4-(2-trifluormethylphenyl)-pyridin-5-carbonsäuremethylester vom Schmp. 176 °C (Ethanol) erhalten.
Ausbeute: 36% der Theorie.

Beispiel 28

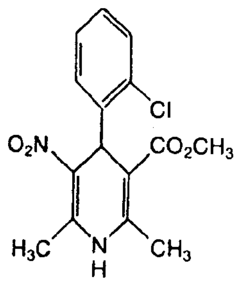

Analog Beispiel 16 wurde durch Umsetzung von 2-Chlorbenzaldehyd mit β-Aminocrotonsäuremethylester und Nitroaceton in Ethanol 4-(2-Chlorphenyl)-1,4-dihydro-2,6-dimethyl-3-nitro-pyridin-5-carbonsäuremethylester vom Schmp. 167 °C (Isopropanol) erhalten.
Ausbeute: 42% der Theorie.

Beispiel 29

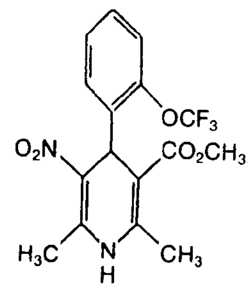

Analog Beispiel 18 wurde durch Umsetzung von 50 mmol 2-Trifluormethoxybenzylidenacetessigsäuremethylester mit 50 mmol 2-Amino-1-nitro-1-propen in Ethanol 1,4-Dihyro2,6-dimethyl-3-nitro-4-(2-trifluormethoxyphenyl)-pyridin-5-carbonsäuremethylester vom Schmp. 175 °C(Isopropanol) erhalten.
Ausbeute: 39% der Theorie.

Beispiel 30

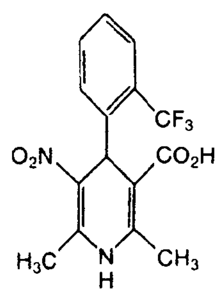

Die Behandlung von 1,4-Dihydro-2,6-dimethyl-3-nitro-4-(2-trifluormethylphenyl)-pyridin-5-carbonsäure-(β-cyanoethyl)-ester in 1,2 Äquivalenten Kaliumhydroxid in wässrigem Ethylenglykoldimethylether bei Raumtemperatur ergibt nach Ansäuern mit verd. HCl 1,4-Dihydro-2,6-dimethyl-3-nitro-4-(2-trifluormethylphenyl)-pyridin-5-carbonsäure vom Schmp. 183 °C (Zersetzung) (Methanol).
Ausbeute: 89% der Theorie.

Beispiel 31

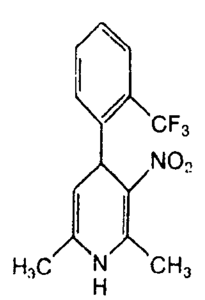

Beim Erhitzen in Ethanol unter Zusatz einer Spur Schwefelsäure decarboxyliert 1,4-Dihydro-2,6-dimethyl-3-nitro-4-(trifluormethylphenyl)-pyridin-5-carbonsäure (Beispiel 30) zu 1,4-Dihydro-2,6-dimethyl-3-nitro-4-(trifluormethylphenyl)-pyridin mit quantitativer Ausbeute.
Schmp. 201 °C.

Beispiel 32

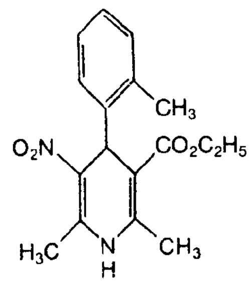

Analog Beispiel 18 wurde durch Umsetzung von 2-Methylbenzyliden-acetessigsäureethylester mit 2-Amino-1-nitro-1-propen in Ethanol 1,4-Dihydro-2,6-dimethyl-4-(2-tolyl)-3-nitro-pyridin-5-carbonsäureethylester vom Schmp.155 °C (Isopropanol) erhalten.
Ausbeute: 42% der Theorie.

Beispiel 33

2-Acetylamino-4,6-dimethyl-1,4-dihydropyridin-3,5-dicarbonsäurediethylester

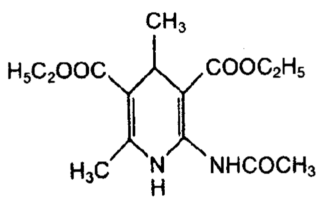

8 g (0,03 Mol) 2-Amino-4,6-dimethyl-1,4-dihydropyridin-3,5-dicarbonsäurediethylester werden in 100 ml Acetanhydrid 4 Stunden unter Rückfluss gekocht. Man engt im Vakuum zum Trockne ein und kristallisiert den Rückstand aus Ethanol um. Ausbeute: 5,4 g (58% der Theorie) vom Fp.: 105 °C.

Beispiel 34

2-Amino-6-methyl-1,4-dihydro-4-(2′-chlorphenyl)-pyridin-3-carbonsäureethylester

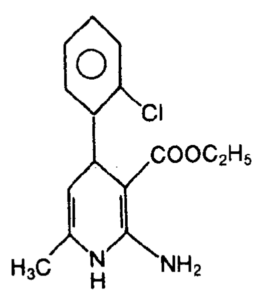

Nach 2-stündigem Kochen einer Lösung von 18,1 g 2′-Chlorbenzylidenaceton und 13,0 g Amidinoessigsäureethylester in 150 ml Ethanol wird das Lösungsmittel im Vakuum abgezogen. Der Rückstand wird aus Ethanol umkristallisiert, Ausbeute 62% der Theorie, Fp.: 171 °C.

Beispiel 35

1,4-Dihydro-4-phenyl-2,3,6-trimethylpyridin-5-carbonsäuremethylester

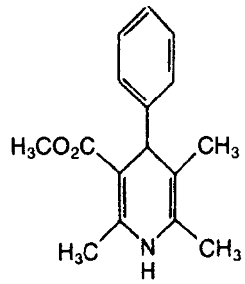

24,1 g (80 mmol) 1,4-Dihydro-2,6-dimethyl-4-phenyl-pyridin-3,5-dicarbonsäuremethylester werden bei 60 °C unter Rühren portionsweise in eine Lösung von 12,2 g (320 mmol) $LiAlH_4$ in 400 ml abs. Tetrahydrofuran eingetragen ($N_2$-Atm) und 6 bis 7 Stunden bei 60 °C nachgerührt. Nach dem Abkühlen werden nacheinander 19,7 ml Essigester, 15,9 ml Wasser, 19,6 ml N NaOH und 15,9 ml Wasser zugetropft. Anschliessend wird abgesaugt, gut mit Ether nachgewaschen und das Filtrat im Vakuum eingedampft. Der sirupöse Rückstand wird in 50 ml Ether gelöst und in der Kälte zur Kristallisation gestellt, 7,4 g d.s. 36% der Theorie. Nach Umkristallisation aus Acetonitril farblose Prismen; Schmp. 120 °C.

Beispeil 36

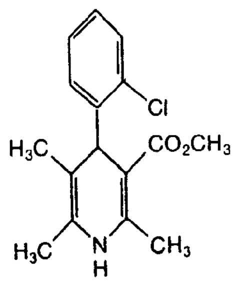

Analog Beispiel 35 wurde am 4-(2-Chlorphenyl)-1,4-hydro-2,6-dimethyl-pyridin-3,5-dicarbonsäuremethylester durch Reduktion mit $LiAlH_4$ in THF 4-(2-Chlorphenyl)-1,4-dihydro-2,3,6-trimethyl-pyridin-5-carbonsäuremethylester vom Schmp. 164 °C (Acetonitril) erhalten.
Ausbeute: 30% der Theorie.

Beispeil 37

1,4-Dihydro-2,6-dimethyl-4-(3-nitrophenyl)-pyridin-3,5-dicarbonsäure-isopropyl-(2,2,2- trichlorethyl)-ester

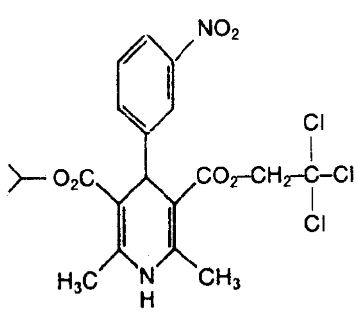

Eine Lösung von 30,2 g (0,2 Mol) 3-Nitrobenzaldehyd, 46,6 g (0,2 Mol) Acetessigsäure-(2,2,2-trichlorethyl)-ester und 28,6 g (0,2 Mol) 3-Aminocrotonsäureisopropylester in 200 ml Ethanol wurde 12 Stunden unter Stickstoff zum Sieden erhitzt. Das Lösungsmittel wurde im Vakuum eingeengt, der feste Rückstand aus Ethanol umkristallisiert. Schmp.: 192 °C; Ausbeute: 46 g (47% er Theorie).

Beispiel 38

1,4-Dihydro-2,6-dimethyl-4-(3-nitrophenyl)-pyridin-3,5-dicarbonsäure (n) hexylisopropylester

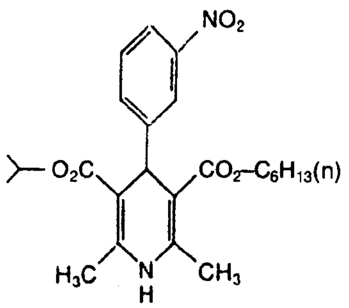

Wurde analog Bespiel 32 hergestellt aus 0,2 Mol 3-Nitrobenzaldehyd, 0,2 Mol Acetessigsäure-(n-hexyl)-ester und 0,2 Mol 3-Aminocrotonsäureisopropylester. Schmp.: 87 °C; Ausbeute: 75%.

Beispiel 39

1,4-Dihydro-2,6-dimethyl-4-(pyridyl-3)-pyridin-3,5-dicarbonsäuremonoethylester

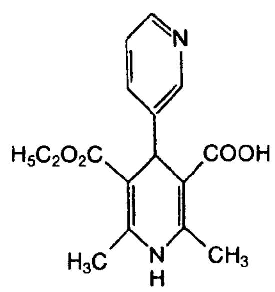

Hergestellt gemäss DE-OS 2 847 237 (Beispiel Nr. 14). Schmp.: 205 °C (Zers.); Ausbeute: 32% der Theorie.

Beispiel 40

3-Cyano-1,4-dihydro-2,6-dimethyl-4-(2-trifluormethylphenyl)-pyridin-5-carbonsäuremethylester

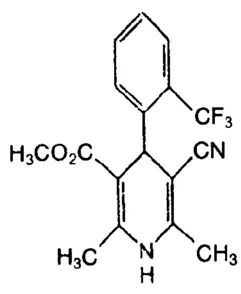

Hergestellt gemäss DE-OS 2 658 804 (Beispiel Nr. 17). Schmp.: 171 °C; Ausbeute: 42% der Theorie.

Beispiel 41

2,6-Di-(4-nitrophenyl)-4-(4-ethoxycarbonylmethoxy-1-methyl-1,4-dihydropyridin-3,5-dicarbonsäurediethyl-ester

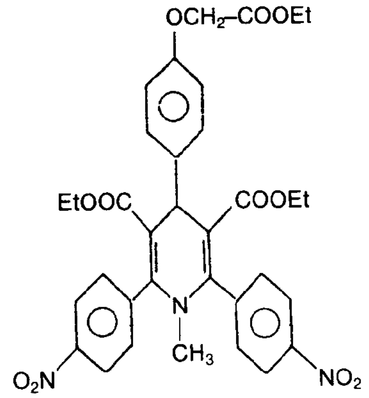

100 mmol 4-(Ethoxycarbonylmethoxy)-benzaldehyd wurden mit 200 mmol 4-Nitrobenzoylessigester, 100 mmol Methylamin-hydrochlorid in 60 ml Pyridin 5 Stunden auf 100 °C erhitzt. Es wurde auf Eiswasser gegossen und abgesaugt.
Ausbeute: 11% der Theorie; Fp.: 136 °C.

Beispiel 42

2,6-Dimethyl-4-(2-trifluormethylphenyl)-1-methyl-1,4-dihydropyridin-3,5-dicarbonsäurediallylester

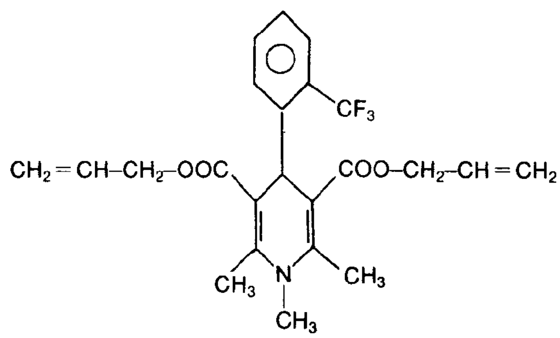

100 mmol 2-Trifluormethylbenzaldehyd, 200 mmol Acetessigsäureallylester und 120 mmol Methylaminhydrochlorid wurden in 50 ml Pyridin 5 Stunden auf 110 °C erhitzt, auf Wasser gekippt und abgesaugt.
Ausbeute: 5% der Theorie; Fp.: 90 bis 91 °C.

Beispiel 43

2,6-Diphenyl-3-5-di(phenylcarbonyl)-1-methyl-4-(3-pyridiyl)-1,4-dihydropyridin

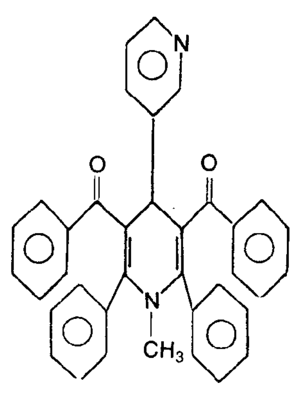

1,3 ml Pyridin-3-aldehyd, 6 g Dibenzoylmethan und 1 g Methylaminhydrochlorid werden in 10 ml Pyridin über

Nacht auf 100 °C erwärmt, anschliessend auf Eiswasser gegossen, abgesaugt und aus Methanol umkristallisiert.
Ausbeute: 15% der Theorie; Fp.: 238 °C.

Beispiel 44

Bis-[2,6-dimethyl-5-ethoxycarbonyl-4-(3-nitrophenyl)-1,4-dihydropyridin-3-carbonsäure]-1,6-hexandiylester

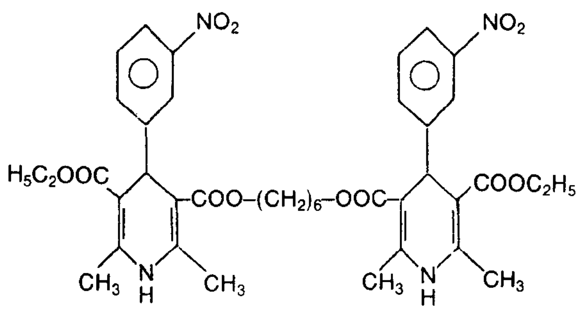

25 mmol Bis-(3-aminocrotonsäure)-1,6-hexandiylester werden zusammen mit 50 mmol 3-Nitrobenzylidenacetessigsäureethylester in 100 ml Ethanol 14 Stunden unter Rückfluss gekocht. Nach Erkalten wird das Lösungsmittel im Vakuum abdestilliert und mit 50%igem wässrigem Ethanol aufgenommen. Der halbfeste Rückstand wurde aus Methanol umkristallisiert.
Ausbeute: 37% der Theorie; Fp.: 177 bis 179 °C.

Beispiel 45

Bis-[2,6-dimethyl-5-ethoxycarbonyl-4-(3-nitrophenyl)-1,4-dihydropyridin-3-carbonsäure]-1,12-dodecandiylester

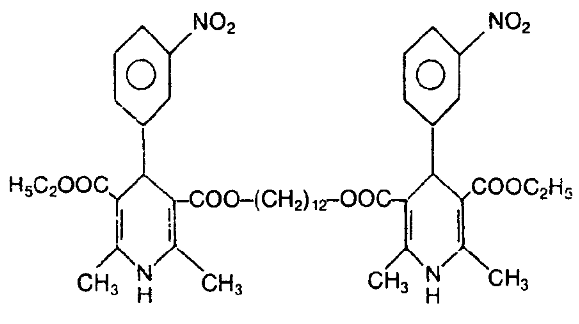

Darstellung analog Beispiel 44 mit Bis-(3-aminocrotonsäure)-1,12-dodecandiylester anstatt Bis-(3-aminocrotonsäure)-1,6-Hexandiylester.
Ausbeute: 10% der Theorie; Fp.: 103 bis 120 °C.

Beispiel 46

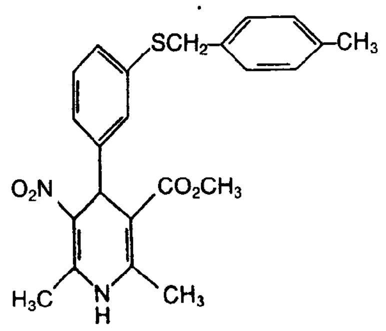

Analog Beispiel 17 wurde durch Umsetzung von 2-(4-Methylbenzylthio)-benzaldehyd mit 2-Amino-1-nitro-1-propen und Acetessigsäuremethylester in Ethanol
1,4-Dihydro-2,6-dimethyl-4-[2-(4-methylbenzylthio)-phenyl]-3-nitropyridin-5-carbonsäuremethylester vom Schmp. 160 °C (Isopropanol) erhalten.
Ausbeute: 28% der Theorie.

Beispiel 47

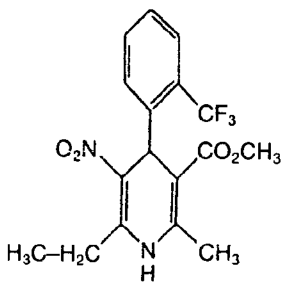

Analog Bespiel 16 wurde durch Umsetzung von 2-Trifluormethylbenzaldehyd mit β-Aminocrotonsäuremethylester und
1-Nitrobutanon-2 in Ethanol 1,4-Dihydro-2-ethyl-6-methyl-3-nitro-4-(2-trifluormethylphenyl)-pyridin-5-carbonsäure-methylester vom Schmp. 199 °C (Iropropanol) erhalten.
Ausbeute: 31 % der Theorie.

Beispiel 48

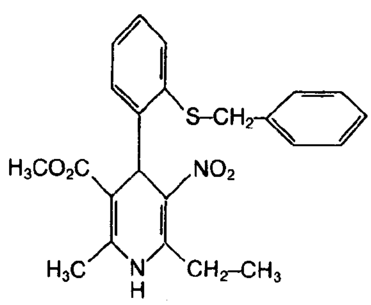

Analog Beispiel 16 wurde durch Umsetzung von 2-Benzylthiobenzaldehyd mit β-Aminocrotonsäuremethylester und 1-Nitrobutanon-2 in Ethanol 4-(2-Benzylthiophenyl)-1,4-dihydro-2-ethyl-6-methyl-3-nitro-pyridin-5-carbonsäuremethylester vom Schmp. 122 °C (Isopropanol) erhalten.
Ausbeute: 21 % der Theorie.

Beispiel 49

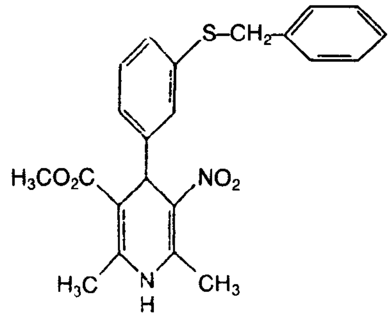

Analog Beispiel 16 wurde durch Umsetzung von 3-Benzylthiobenzaldehyd mit β-Aminocrotonsäuremethylester und Nitroaceton in Ethanol 4-(3-Benzylthiophenyl)-1,4-dihydro-2,6-dimethyl-3-nitropyridin-5-carbonsäuremethyl-ester vom Schmp. 155 °C (Ethanol) erhalten.
Ausbeute: 35% der Theorie.

Beispiel 50

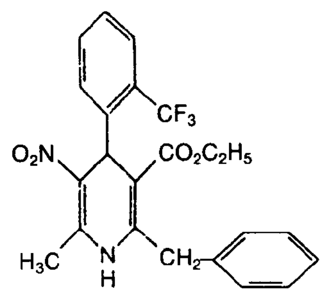

Analog Beispiel 17 wurde durch Umsetzung von 2-Trifluormethylbenzaldehyd mit γ-Phenylacetessigsäureethylester und 2-Amino-1-nitro-1-propen in Ethanol 2-Benzyl-1,4-dihydro-6-methyl-3-nitro-4-(2-trifluormethylphenyl)-pyridin-5-carbonsäureethylester
vom Schmp. 168 °C erhalten.
Ausbeute: 12% der Theorie.

Beispiel 51

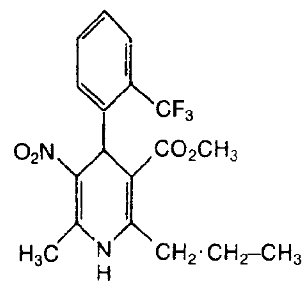

Analog Beispiel 17 wurde durch Umsetzung von 2-Trifluormethylbenzaldehyd mit 2-Amino-1-nitro-1-propen und 3-Oxo-hexansäuremethylester in Etanol
1,4-Dihydro-2-methyl-3-nitro-6-propyl-4-(2-trifluormethyl-phenyl)-pyridin-5-carbonsäuremethylester
vom Schmp. 168 °C (Isopropanol) erhalten.
Ausbeute: 19% der Theorie.

Beispiel 52

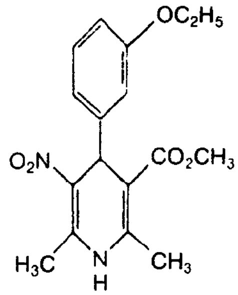

Analog Beispiel 16 wurde durch Umsetzung von 3-Ethoxybenzaldehyd mit Nitroaceton und β-Aminocrotonsäuremethylester in Ethanol 1,4-Dihydro-2,6-dimethyl-4-(3-ethoxyphenyl)-3-nitropyridin-5-carbonsäuremethylester vom Schmp. 127 °C erhalten.
Ausbeute: 42% der Theorie.

Beispiel 53

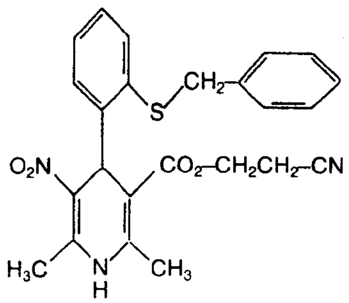

Analog Beispiel 16 wurde durch Umsetzung von 2-Benzyl-thiobenzaldehyd mit Nitroaceton und β-Aminocrotonsäure-(β-cyanoethyl)ester in Ethanol-4-(2-Benzylthiophenyl)-1,4-dihydro-2,6-dimethyl-3-nitropyridin-5-carbonsäure-(β-cyanoethyl)ester
vom Schmp. 173 °C (Isopropanol) erhalten.
Ausbeute: 28% der Theorie.

Beispiel 54

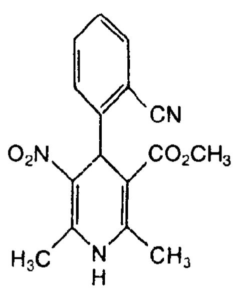

Analog Beispiel 16 wurde durch Umsetzung von 2-Cyanobenzaldehyd mit Nitroaceton und β-Aminocrotonsäuremethylester in Ethanol 4-(2-Cyanophenyl)-1,4-dihydro-2,6-dimethyl-3-nitropyridin-5-carbonsäuremethylester vom Schmp. 182 °C (Isopropanol) erhalten.
Ausbeute: 28% der Theorie.

Beispiel 55

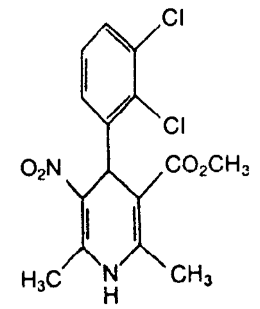

Analog Beispiel 16 wurde durch Umsetzung von 2,3-Dichlorbenzaldehyd mit Nitroaceton und β-Aminocrotonsäuremethylester in Ethanol 4-(2,3-Dichlorphenyl)-1,4-dihydro-2,6-dimethyl-3-nitropyridin-5-carbonsäuremethylester
vom Schmp. 184 °C erhalten.
Ausbeute: 39% der Theorie.

Beispiel 56

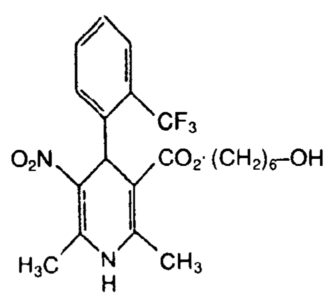

Analog Beispiel 16 wurde durch Umsetzung von 2-Trifluormethylbenzaldehyd mit Nitroaceton und β-Aminocrotonsäure-(6-hydroxyhexyl)ester in Ethanol 1,4-Dihydro-2,6-dimethyl-3-nitro-4-(2-trifluormethylphenyl)-pyridin-5-carbonsäure-(6-hydroxyhexyl)ester
vom Schmp. 174 °C erhalten.
Ausbeute: 32% der Theorie.

Beispiel 57

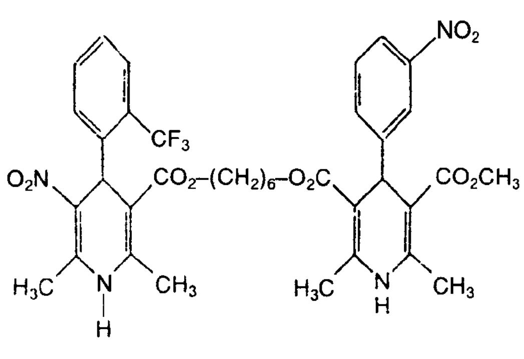

Durch Umsetzung von 1 Äquivalent 1,4-Dihydro-2,6-dimethyl-3-nitro-4-(2-trifluormethylphenyl)-pyridin-5-carbonsäure-(6-hydroxyhexyl)-ester mit 1 Äquivalent 1,4-Dihydro-2,6-dimethyl-4-(3-nitrophenyl)-3-carboxymethylpyridin-5-carbonsäure und 1,1 Äquivalenten Dicyclohexylcarbodiimid im Methylenchlorid wird 5-6-[2,6-Dimethyl-5-nitro-4-(2-trifluormethylphenyl)-1,4-dihydro-pyrid-3-ylcarboxy]-hexyloxycarbonyl-2,6-dimethyl-4-(3-nitrophenyl)-1,4-dihydropyridin-3-carbonsäuremethylester als amorphe, gelbe Substanz erhalten.
Ausbeute: 68%

m/e: 756 ≙ $M^{\oplus}$

| Elementaranalyse | C | H | N | F |
|---|---|---|---|---|
| ber. | 58,7% | 5,2% | 7,4% | 7,5% |
| gef. | 58,5% | 5,2% | 7,2% | 7,0% |

Beispiel 58

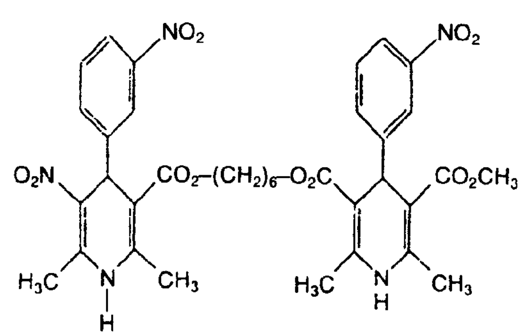

Analog dem vorhergehenden Beispiel erhält man durch Umsetzung von 1,4-Dihydro-2,6-dimethyl-3-nitro-4-(3-nitrophenyl)-pyridin-5-carbonsäure-(6-hydroxyhexylester) mit 1,4-Dihydro-2,6-dimethyl-4-(3-nitrophenyl)-3-carboxymethyl-pyridin-5-carbonsäure und Dicyclohexylcarbodiimid in Methylenchlorid wird
5- 6-[2,3-Dimethyl-5-nitro-4-(3-nitrophenyl)-1,4-dihydro-pyrid-3-ylcarboxy]-hexyloxycarbonyl-2,6-dimethyl-4-(3-nitrophenyl)-1,4-dihydropyridin-3-carbonsäuremethylester
als amorphe, gelbe Substanz erhalten.
Ausbeute: 73% m/e: 733 ≙ M $^{\oplus}$

| Elementaranalyse | C | H | N |
|---|---|---|---|
| ber. | 58,9% | 5,3% | 9,5% |
| gef. | 58,7% | 5,2% | 9,6% |

Beispiel 59

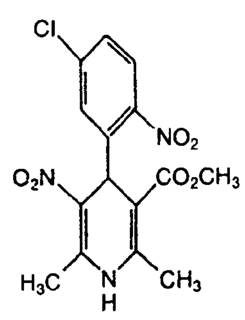

Analog Beispiel 16 wurde durch Umsetzung von 5-Chlor-2-nitrobenzaldehyd mit Nitroaceton und β-Aminocrotonsäuremethylester in Ethanol 4-(5-Chlor-2-nitrophenyl)-2,4-dihydro-2,6-dimethyl-3-nitro-pyridin-5-carbonsäure-methylester vom Schmp. 221 °C (Isopropanol) erhalten.
Ausbeute: 43% der Theorie.

Beispiel 60

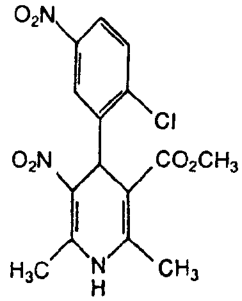

Analog Beispiel 16 wurde durch Umsetzung von 2-Chlor-5-nitrobenzaldehyd mit Nitroaceton und β-Aminocrotonsäuremethylester in Ethanol 4-(2-Chlor-5-nitrophenyl)-1,4-dihydro-2,6-dimethyl-3-nitro-pyridin-5-carbonsäure-methylester vom Schmp. 219 °C erhalten.
Ausbeute: 39% der Theorie.

Beispiel 61

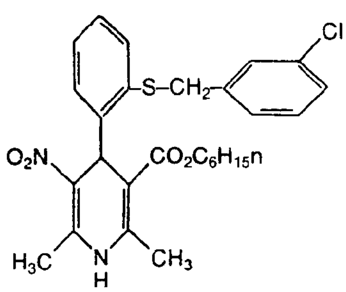

Analog Beispiel 16 wurde durch Umsetzung von 2-(3-Chlorbenzylthio)-benzaldehyd mit Nitroaceton und β-Aminocrotonsäure-n-hexylester in Ethanol 4-(3-Chlorbenzylthiophenyl)-1,4-dihydro-2,6-dimethyl-3-nitropyridin-5-carbonsäure-n-hexylester nach Chromatographie erhalten.
Ausbeute: 21% der Theorie.

Elementaranalyse

| | C | H | Cl | N | S |
|---|---|---|---|---|---|
| ber. | 62,9% | 6,2% | 6,9% | 5,4% | 6,2% |
| gef. | 62,7% | 6,1% | 6,4% | 5,3% | 6,1% |

Beispiel 62

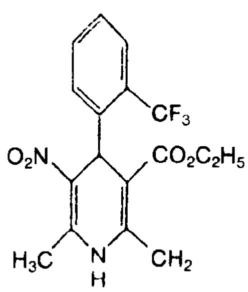

Durch Umsetzung von je 10 mmol 2-Trifluormethylbenzaldehyd, Nitroaceton, Amidinoessigesterhydrochlorid und Natriummethylat in 60 ml siedendem Ethanol wurde 6-Amino-1,4-dihydro-2-methyl-3-nitro-4-(2-trifluormethylphenyl)-pyridin-5-carbonsäuremethylester vom Schmp. 210 °C (Isopropanol) erhalten.

Ausbeute: 36% der Theorie.

Beispiel 63

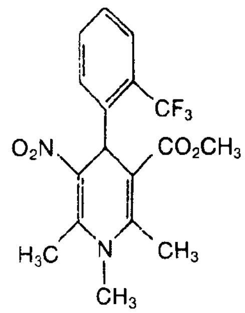

Durch Umsetzung von je 10 mmol 2-Trifluormethylbenzaldehyd, Acetessigsäuremethylester, Nitroaceton und Methylaminhydrochlorid in 50 ml Eisessig bei 60 °C für 12 Sunden wurde 1,4-Dihydro-3-nitro-4-(2-trifluormethylphenyl)-1,2,6-trimethylpyridin-5-carbonsäuremethylester vom Schmp. 156 °C (Isopropanol) erhalten.
Ausbeute: 15% der Theorie.

Beispiel 64

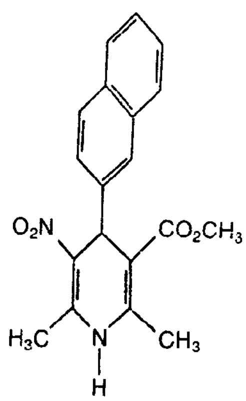

Analog Beispiel 16 wurde durch Umsetzung von β-Naphthylaldehyd, β-Aminocrotonsäuremethylester und Nitroaceton in Ethanol 1,4-Dihydro-2,6-dimethyl-4-β-naphthyl-3-nitropyridin-5-carbonsäuremethylester vom Schmp. 163 °C (Isopropanol) erhalten.
Ausbeute: 42% der Theorie.

Beispiel 65

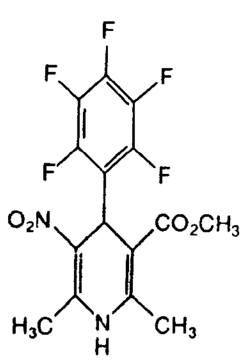

Analog Beispiel 16 wurde durch Umsetzung von Pentafluorbenzaldehyd mit Nitroaceton und β-Aminocrotonsäuremethylester in Ethanol 1,4-Dihydro-2,6-dimethyl-3-nitro-4-pentafluorphenylpyridin-5-carbonsäuremethylester
vom Schmp. 231 °C erhalten.
Ausbeute: 38% der Theorie.

Beispiel 66

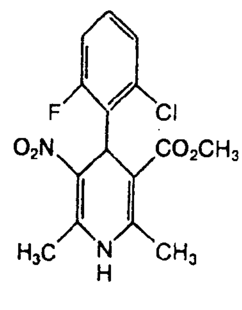

Analog Beispiel 16 wurde durch Umsetzung von 2-Chlor-6-fluorbenzaldehyd mit Nitroaceton und β-Aminocrotonsäuremethylester
4-(2-Chlor-6-fluorphenyl)-1,4-dihydro-2,6-dimethyl-3-nitropyridin-5-carbonsäuremethylester vom Schmp. 190 °C erhalten.
Ausbeute: 28% der Theorie.

Beispiel 67

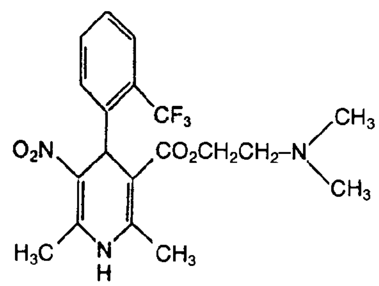

Durch Umsetzung von je 1 Äquivalent 1,4-Dihydro-2,6-dimethyl-3-nitro-4-(2-trifluormethylphenyl)-pyridin-5-carbonsäure mit Dimethylaminoethanol und DCC im Methylenchlorid erhält man
1,4-Dihydro-2,6-dimethyl-3-nitro-4-(2-trifluormethylphenyl)-pyridin-5-carbonsäure-β-dimethylaminoethylester
vom Schmp. 132 °C.
Ausbeute: 84% der Theorie.

Beispiel 68

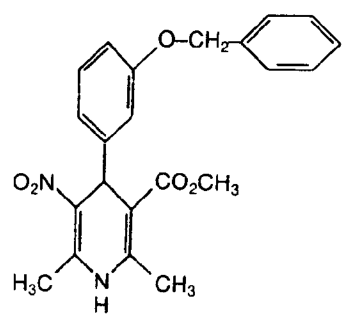

Analog Beispiel 16 erhält man durch Umsetzung von 3-Benzyloxybenzaldehyd mit Nitroaceton und β-Ami-

nocrotonsäuremethylester
4-(3-Benzyloxyphenyl)-1,4-dihydro-2,6-dimethyl-3-nitropyridin-5-carbonsäuremethylester vom Schmp. 181 °C (Isopropanol).
Ausbeute: 34% der Theorie.

Beispiel 69

1,4-Dihydro-2,6-dimethyl-4-[2-(4-methylbenzyloxy)-phenyl]-pyridin-3-carbonsäure-methylester

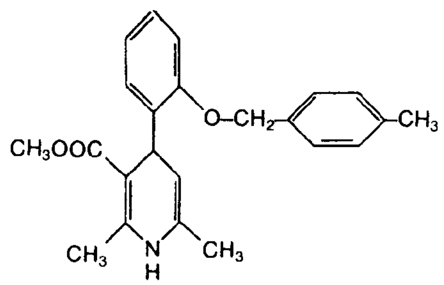

A) Herstellung von α-Acetyl-2-(4-methylbenzyloxy)-zimtsäuremethylester

25 g (110,6 mmol) 2-(4-Methylbenzyloxy)-benzaldehyd werden mit 11,9 ml (110,6 mmol) Acetessigsäuremethylester in 66 ml Isopropanol verrührt und mit einer frisch zubereiteten Lösung von 0,64 ml Piperidin und 0,38 ml Eisessig in 5,5 ml Isopropanol versetzt. Es wird 1 Stunde bei 60 °C und 4 Stunden bei 40 °C gerührt, abgekühlt und eingeengt. Der Eindampfrückstand wird in Ether gelöst und nacheinander mit ca. 100 ml 1N Salzsäure, zweimal mit Wasser, mit gesättigter Natriumhydrogencarbonatlösung und wieder zweimal mit Wasser gewaschen. Die Etherphase wird getrocknet, filtriert und eingeengt. Man erhält 35,3 g (98,51 % der Theorie) eines dunkelgelben Öles, welches roh weiter umgesetzt wird.

B) 1,4-Dihydro-2,6-dimethyl-4-[2-(4-methylbenzyloxy)-phenyl]-pyridin-3,5-dicarbonsäuremethyl-(2-cyanoethyl)-ester

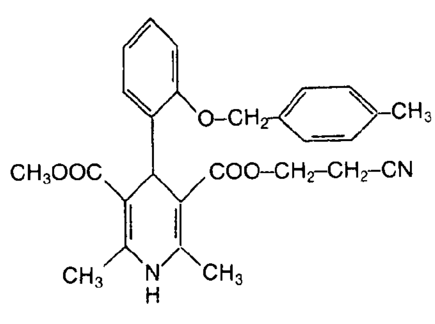

35,3 g (108,35 mmol) α-Acetyl-2-(4-methylbenzyloxy)-zimtsäuremethylester werden mit 16,8 g (108,95 mmol) β-Aminocrotonsäure-2-cyanoethylester in 160 ml Ethanol 18 Stunden zum Rückfluss gekocht. Es wird eingeengt, der Eindampfrückstand wird in Essigester aufgenommen und zweimal mit Wasser geschüttelt. Die Essigesterphase wird getrocknet, filtriert und eingeengt. Der erhaltene Eindampfrückstand kristallisiert beim Verrühren mit Methanol. Es wird abgesaugt und mit Methanol gewaschen. Man erhält 20,2 g (40,53% der Theorie) eines leicht gelbgefärbten Produktes vom Schmp. 165 °C unter Zersetzung.

C) 1,4-Dihydro-2,6-dimethyl-4-[2-(4-methylbenzyloxy)-phenyl]-pyridin-3,5-dicarbonsäuremonomethylester

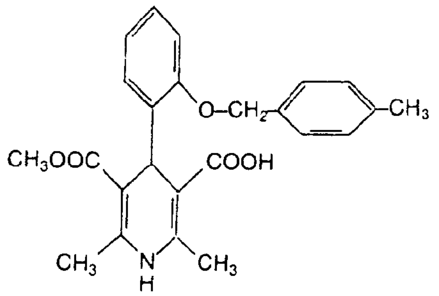

17,5 g (38 mmol) 1,4-Dihydro-2,6-dimethyl-4-[2-(4-methylbenzyloxy)-phenyl]-pyridin-3,5-dicarbonsäure-methyl-(2-cyanoethyl)-ester werden in einer Lösung von 4,6 g (115 mmol) Natriumhydroxid in 115 ml Wasser suspendiert und mit 57,5 ml Dimethoxyethan versetzt. Man erhält allmählich eine klare Lösung. Es wird 20 Stunden gerührt, mit 100 ml Wasser versetzt und dreimal mit Methylenchlorid extrahiert. Die wässrige Phase wird unter Rühren mit konz. Salzsäure tropfenweise sauer gestellt, wobei die Säure ausfällt. Es wird abgesaugt, mit Wasser gewaschen und getrocknet. Man erhält 12,2 g (78,9% der Theorie) eines beigefarbenen Festproduktes vom Schmp. ab 169 °C Zersetzung.

D) 3 g 1,4-Dihydro-2,6-dimethyl-4-[2-(4-methylbenzyloxy)-phenyl]-pyridin-3,5-dicarbonsäuremonomethylester

werden in 30 ml Diethylenglykol suspendiert und unter Rühren auf 170 bis 180 °C erwärmt, wobei unter Gasentwicklung eine klare Lösung entsteht. Es wird ca. 5 Minuten bei 180 °C gerührt und abgekühlt. Die viskose Lösung wird unter starkem Schütteln in einem Ether/Wasser-Gemisch gelöst, es wird getrennt, die wässrige Phase mit Ether extrahiert und die vereinten Etherphasen mit 1N Natronlauge und zweimal mit Wasser gewaschen. Es wird getrocknet, filtriert und eingeengt. Der erhaltene sirupöse Rückstand wird mit oder Acetonitril im Eisbad kristallisiert und abgesaugt. Es werden 1,6 g 1,4-Dihydro-2,6-dimethyl-4-[2-(4-methylbenzyloxy)-phenyl]-pyridin-3-carbonsäuremethylester vom Schmp. 107 °C bis 109 °C erhalten.

Analog wurden hergestellt:

Beispiel 70

1,4-Dihydro-2,6-dimethyl-4-(2-trifluormethylphenyl)-pyridin-3-carbonsäureethylester vom Schmp. 125 bis 128 °C.

Beispiel 71

1,4-Dihydro-2,6-dimethyl-4-(3-nitrophenyl)-pyridin-3-carbonsäureisopropylester, isoliert als Öl. $R_f$-Wert: 0,525; DC-Alurolle, Schichtdicke: 0,2 mm, Kieselgel: 60 F 254, Merck; Fliessmittel: Petrolether/Essigester im Volumenverhältniss 2 : 1.

Beispiel 72

1,4-Dihydro-1,2,6-trimethyl-4-(2-chlorphenyl)-pyridin-3-carbonsäuremethylester
vom Schmp. 142 bis 144 °C.

Beispiel 73

1,4-Dihydro-2,6-dimethyl-4-(3-chlorphenyl) pyridin-3-carbonsäuremethylester vom Schmp. 1299 bis 133 °C.

Beispiel 74

1,4-Dihydro-2,6-dimethyl-4-(2-methylphenyl)-pyridin-3-carbonsäuremethylester
vom Schmp. 127 bis 130 °C.

Beispiel 75

1,4-Dihydro-2,6-dimethyl-4-(2-chlorphenyl)-pyridin-3-carbonsäuremethylester vom Schmp. 143-146 °C.

Beispiel 76

1,4-Dihydro-2,6-dimethyl-4-(3-nitrophenyl)-pyridin-3-carbonsäuremethylester vom Schmp. 110 bis 112 °C.

Beispiel 77

1,4-Dihydro-2,6-dimethyl-4-(2-phenylthiomethyloxy-phenyl)-pyridin-3-carbonsäuremethylester als Öl.

NMR-Spektrum bei 250 MHz in $CDCl_3$

| | | | |
|---|---|---|---|
| Singulett | bei $\delta =$ | 1,6 ppm | (3H, 6–$CH_3$) |
| Singulett | bei $\delta =$ | 2,38 ppm | (3H, 2–$CH_3$) |
| Singulett | bei $\delta =$ | 3,42 ppm | (3H, $COOCH_3$) |
| 2 Dupletts | bei $\delta =$ | 4,65 und 4,96 ppm | (je 1H für 5H und 4H) |
| Singulett | bei $\delta =$ | 5,16 ppm | (1H für 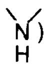) |
| AB-System | bei $\delta =$ | 5,5 bis 5,64 ppm | (2H für –O–$CH_2$–S–) |
| Multiplett | bei $\delta =$ | 6,9 bis 7,65 ppm | (9H, Aromaten) |

Beispiel 78

1,4-Dihydro-2,6-dimethyl-4-(2-benzyloxyphenyl)-pyridin-3-carbonsäuremethylester vom Schmp. 81 bis 83 °C.

Beispiel 79

1,4-Dihydro-2,6-dimethyl-4-[2-(4-chlorbenzyloxy)-phenyl]-pyridin-3-carbonsäuremethylester vom Schmp. 90 bis 94 °C.

Beispiel 80

1,4-Dihydro-2,6-dimethyl-4-[2-(2-chlorbenzyloxy)-phenyl]-pyridin-3-carbonsäuremethylester vom Schmp. 75 bis 77°C.

Beispiel 81

1,4-Dihydro-2,6-dimethyl-4-[2-(2,6-dichlorbenzyloxy)-phenyl]-pyridin-3-carbonsäuremethylster vom Schmp. 125 bis 128 °C.

Beispiel 82

1,4-Dihydro-2,6-dimethyl-4-[2-(3,4-dichlorphenyloxy)-phenyl]-pyridin-3-carbonsäuremethylester, isoliert als Schaum. $R_f$-Wert: 0,65, DC-Fertigplatten Kieselgel 60 F 254, Fliessmittel: Chloroform/Essigester im Volumenverhältnis 5 : 1.

Beispiel 83

1,4-Dihydro-2,6-dimethyl-4-[2-(4-methylbenzyloxy)-naphthyl]-pyridin-3-carbonsäuremethylester, isoliert als Schaum.

Massenspektrum:

Die wichtigsten Peaks findet man bei m/e = 413 (Molpeak); m/e = 308; m/e = 248; m/e = 166.

Beispiel 84

1,4-Dihydro-2,6-dimethyl-4-[2-(4-methylbenzyloxy)-phenyl]-pyridin-3-carbonsäureisopropylester vom Schmp. ab 99 °C.

Beispiel 85

1,4-Dihydro-2,6-dimethyl-4-[2-(3-trifluormethylbenzylthio)-phenyl]-pyridin-3-carbonsäuremethylester, isoliert als Öl. $R_f$-Wert: 0,84, DC-Alurolle, Schichtdicke: 0,2 mm, Kieselgel 60 F 254, Fliessmittel: Choroform/Essigester im Volumenverhältnis 5 : 1.

Beispiel 86

1,4-Dihydro-2,6-dimethyl-4-[2-(4-methylbenzyloxy)-phenyl]-pyridin-3-carbonsäureisobutylester vom Schmp. ab 57 °C.

Beispiel 87

1,4-Dihydro-2,6-dimethyl-4-naphthyl-pyridin-3-carbonsäuremethylester, isoliert als Schaum.

Massenspektrum:

Die wichtigsten Massenpeaks sind m/e = 293 (Molpeak); m/e = 166 (M - naphthyl).

Beispiel 88

1,4-Dihydro-2,6-dimethyl-4-[2-(3-trifluormethylbenzyloxy)-phenyl]-pyridin-3-carbonsäuremethylester vom Schmp. 134 °C.

Beispiel 89

1,4-Dihydro-2,6-dimethyl-4-[2-(4-methylbenzyloxy)-phenyl]-pyridin-3-carbonsäure-sec.-butylser vom Schmp. ab 78 °C.

Beispiel 90

1,4-Dihydro-2,6-dimethyl-4-(3-benzyloxyphenyl)-pyridin-3-carbonsäuremethylester, isoliert als Öl. $R_f$-Wert: 0,69, DC-Alurolle, Schichtdicke 0,2 mm, Kieselgel 60 G 254, Merck; Fliessmittel: Cloroform/Essigester im Volumenverhältnis 7 : 1.

Beispiel 91

1,4-Dihydro-2,6-dimethyl-4-[2-(3-trifluormethylbenzyloxy)-phenyl]-pyridin-3-carbonsäureisobutylester, isoliert als Öl.

Massenspektrum:

Die wichtigsten Massenpeaks sind:

m/e = 459 (Molpeak);

m/e = 402 $(M-CH_2-CH$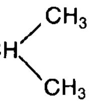 ) ,

m/e = 208 $(M-C_{14}H_{10}F_3O)$.

Beispiel 92

1,4-Dihydro-2,6-dimetyl-4-[2-(3-nitrobenzyloxy)-phenyl]-carbonsäuremethylester vom Schmp. 117 bis 118 °C.

Beispiel 93

1,4-Dihydro-2,6-dimethyl-4-[2-(3-nitrobenzyloxy)-phenyl]-pyridin-3-carbonsäureethylester vom Schmp. ab 107 bis 109 °C.

Beispiel 94

1,4-Dihydro-2,6-dimethyl-4-[2-(4-fluorbenzyloxy)-phenyl]-pyridin-3-carbonsäuremethylester vom Schmp. 68 bis 70 °C.

Beispiel 95

1,4-Dihydro-2,6-dimethyl-4-[2-(3-trifluormethylbenzyloxy)-phenyl]-pyridin-3-carbonsäure-sec.-butylester, isoliert als Öl.

Massenspektrum:

Die wichtigsten Massenpeaks sind:
m/e = 459 (Molpeak); m/e = 402 (M-57).

Beispiel 96

1,4-Dihydro-2,6-dimethyl-4-[2-(4-fluorbenzyloxy)-phenyl]-pyridin-3-carbonsäureethylester vom Schmp. 122 bis 125 °C.

Beispiel 97

1,4-Dihydro-2,6-dimethyl-4-[2-(3,5-dimethylbenzyloxy)-phenyl]-pyridin-3-carbonsäuremethylester vom Schmp. 127 bis 129 °C (kristallisiert mit 1 Mol Acetonitril).

Beispiel 98

1,4-Dihydro-2,6-dimethyl-4-(3-nitrophenyl)-pyridin-3-carbonsäureethylester vom Schmp. 107 bis 108 °C.

Beispiel 99

1,4-Dihydro-2,6-dimethyl-4-[2-(3,5-dimethylbenzyloxy)-phenyl]-pyridin-3-carbonsäure-n-butylester, isoliert als Öl.

Massenspektrum:

Die wichtigsten Peaks liegen bei:
m/e = 417 (M-2); m/e = 300 (M-119) und
m/e = 208.

Beispiel 100

1,4-Dihydro-2,6-dimethyl-4-[2-(3-methylbenzyloxy)-phenyl]-pyridin-3-carbonsäure-n-butylester vom Schmp. 104 bis 10 °C.

Beispiel 101

1,4-Dihydro-2,6-dimethyl-4-[2-(3-methylbenzyloxy)-phenyl]-pyridin-3-carbonsäuremethylester vom Schmp. 58 bis 62 °C, kristallisiert mit 1 Mol Acetonitril.

Beispiel 102

1,4-Dihydro-2,6-dimethyl-4-[2-(3-fluorbenzyloxy)-phenyl]-pyridin-3-carbonsäuremethylester vom Schmp. 109 bis 10 °C.

Beispiel 103

1,4-Dihydro-2,6-dimethyl-4-[2-(3-fluorbenzyloxy)-phenyl]-pyridin-3-carbonsäure-n-butylester vom Schmp. 105 bis 106 °C.

Beispiel 104

1,4-Dihydro-2,6-dimethyl-4-(2-phenylpropylmercaptophenyl)-pyridin-3-carbonsäuremethylester, isoliert als Öl.

$R_f$-Wert: 0,46, DC-Alurolle, Schichtdicke: 0,2 mm, Kieselgel 60 F 254 Merck, Fliessmittel: Chloroform.

Beispiel 105

1,4-Dihydro-2,6-dimethyl-4-[2-(3-methoxybenzyloxy)-phenyl]-pyridin-3-carbonsäuremethylester vom Schmp. ab 53 °C.

Beispiel 106

1,4-Dihydro-2,6-dimethyl-4-(2-benzylthiophenyl)-pyridin-3-carbonsäuremethylester.

Massenspektrum:

Die wichtigsten Peaks findet man bei
m/e = 365 (Molpeak), m/e = 350 (M-15),
m/e = 306 (M-59), m/e = 274, m/e = 166.

Beispiel 107

1,4-Dihydro-2,6-dimethyl-4-[2-(3-methoxybenzyloxy)-phenyl]-pyridin-3-carbonsäure-n-butylester vom Schmp. 144 bis 146 °C.

Beispiel 108

1,4-Dihydro-2,6-dimethyl-4-(2-chlorphenyl)-5-phenyl-ureidopyridin-3-carbonsäuremethylester

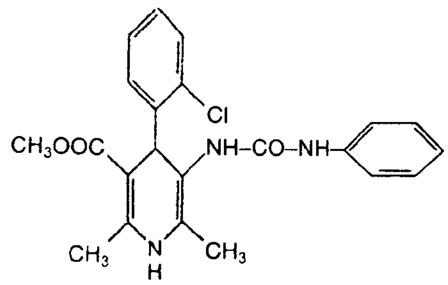

9,65 g (30 mmol) 1,4-Dihydro-2,6-dimethyl-4-(2-chlorphenyl)-3,5-dicarbonsäuremonoethylester werden mit 6,4 Mol (30 mmol) Diphenylphosphorylazid und 4,2 ml (30 mmol) Triethylamin 1 Stunde zum Rückfluss erhitzt. Es wird abgekühlt, mit 2,8 ml (30 mmol) Anilin versetzt und 1 Stunde gekocht. Es wird eingeengt, der feste Eindampfrückstand wird in warmem Essigester gelöst, es wird mit 1N Salzsäure, Wasser, 2N Natronlauge und zweimal mit Wasser gewaschen, getrocknet und eingeengt. Der feste Eindampfrückstand wird mit 100 ml heissem Essigester versetzt, es wird unter Rühren abgekühlt, abgesaugt und mit Essigester gewaschen. Man erhält 4,6 g 1,4-Dihydro-2,6-dimethyl-4-(2-chlorphenyl)-5-phenyl-ureido-pyridin-3-carbonsäuremethylester vom Schmp. 203 bis 205 °C.

Analog wurden hergestellt:

Beispiel 109

1,4-Dihydro-2,6-dimethyl-4-[2-(4-methylbenzyloxy)-phenyl]-5-n-butylureido-pyridin-3-carbonsäuremethylester vom Schmp. 194 bis 196 °C.

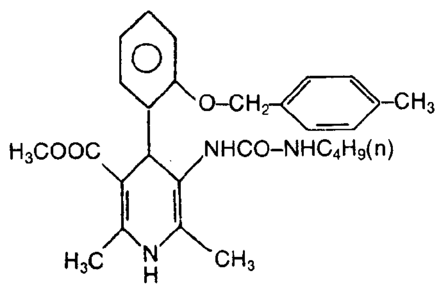

Beispiel 110

1,4-Dihydro-2,6-dimethyl-4-(2-chlorphenyl)-5-N-morpholinyl)-carbonylamino-pyridin-3-carbonsäuremethylester vom Schmp. 245 bis 249 °C.

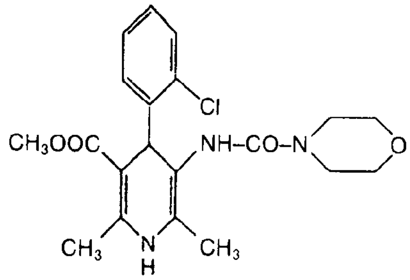

Beispiel 111

1,4-Dihydro-2,6-dimethyl-4-(2-chlorphenyl)-5-(4-N-methylpiperazinyl-carbonylamino)-pyridin-3-carbonsäuremethylester vom Schmp. 231 °CC.

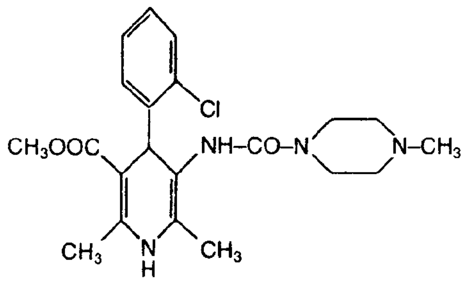

Beispiel 112

1,4-Dihydro-2,6-dimethyl-4-(2-benzyloxyphenyl)-pyridin-3,5-dicarbonsäure-monomethylester

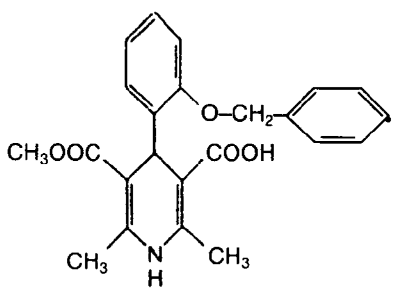

10,6 g (24 mmol) 1,4-Dihydro-2,6-dimethyl-4-(2-benzyloxyphenyl)-pyridin-3,5-dicarbonsäuremethyl-(β-cyanoethyl)-ester werden in einer Lösung von 2,88 g (72 mmol) Natriumhydroxid in 72 ml Wasser suspendiert und mit 45 ml Dimethoxyethan versetzt. Es wird über Nacht gerührt, mit 100 ml Wasser versetzt und dreimal mit Ether extrahiert. Die wässrige Phase wird am Rotationsverdampfer kurz andestilliert und anschliessend unter Rühren tropfenweise mit konzentrierter Salzsäure angesäuert. Das ausgefallene Produkt wird abgesaugt, mit Wasser gewaschen und getrocknet. Man erhält 6,2 g eines beigegefärbten Produktes voom Schmp. 160 °C unter Zersetzung. Analog Beispiel 112 wurden hergestellt:

Beispiel 113

1,4-Dihydro-2,6-dimethyl-4-[2-(4-methylbenzyloxy)-phenyl]-pyridin-3,5- dicarbonsäure-monomethylester vom Schmp. 169 °C unter Zersetzung.

Beispiel 114

1,4-Dihydro-2,6-dimethyl-4-[2-(4-chlorbenzyl)-phenyl]-pyridin-3,5-dicarbonsäure-monomethylester vom Schmp. 156 °C unter Zersetzung.

Beispiel 115

1,4-Dihydro-2,6-dimethyl-4-[2-(2-chlorbenzyloxy)-phenyl]-pyridin-3,5-dicarbonsäure-monomethylester vom Schmp. 153 °C unter Zersetzung.

Beispiel 116

1,4-Dihydro-2,6-dimethyl-4-(2-phenylmercaptomethoxyphenyl)-pyridin-3,5-dicarbonsäure-monomethylester vom Schmp. 95 °C unter Zersetzung.

Beispiel 117

1,4-Dihydro-2,6-dimethyl-4-(2-phenethyloxyphenyl)-pyridin-3,5-dicarbonsäure-monomethylester vom Schmp. 100 bis 103 °C unter Zersetzung.

Beispiel 118

1,4-Dihydro-2,6-dimethyl-4-[2-(2,6-dichlorbenzyloxy)-phenyl]-pyridin-3,5-dicarbonsäuremonomethylester vom Schmp. 198 bis 201 °C unter Zersetzung.

Beispiel 119

1,4-Dihydro-2,6-dimethyl-4-[2-(3,4-dichlorbenzyloxy)-phenyl]-pyridin-3,5-dicarbonsäuremonomethylester vom Schmp. 138 bis 140 °C unter Zersetzung.

Beispiel 120

1,4-Dihydro-2,6-dimethyl-4-(3-propoxyphenyl)-pyridin-3,5-dicarbonsäure-monomethylester vom Schmp. 175 bis 177°C unter Zersetzung.

Bespiel 121

1,4-Dihydro-2,6-dimethyl-4-(3-benzyloxyphenyl)-pyridin-3,5-dicarbonsäure-monomethylester vom Schmp. 148 °C unter Zersetzung.

Beispiel 122

1,4-Dihydro-2,6-dimethyl-4-[2-(4-methylbenzyloxy)-naphthyl]-pyridin-3,5-dicarbonsäure-monomethylester vom Schmp. 171 bis 179 °C unter Zersetzung.

Bespiel 123

1,4-Dihydro-2,6-dimethyl-4-naphthyl-pyridin-3,5-dicarbonsäure-monomethylester vom Schmp. 175 bis 179 °C unter Zersetzung.

Beispiel 124

1,4-Dihydro-2,6-dimethyl-4-[2-(4-methylbenzyloxy)-phenyl]-pyridin-3,5-dicarbonsäure-monoisopropylester vom Schmp. 101 °C unter Zersetzung.

Beispiel 125

1,4-Dihydro-2,6-dimethyl-4-[2-(3-trifluormethylbenzylthio)-phenyl]-pyridin-3,5-dicarbonsäuremonomethylester vom Schmp. 90 °C unter Zersetzung.

Beispiel 126

1,4-Dihydro-2,6-diphenyl-4-[2-(4-methylbenzyloxy)-phenyl]-pyridin-3,5-dicarbonsäure-monoisobutylester vom Schmp. 85 °C unter Zersetzung.

Beispiel 127

1,4-Dihydro-2,6-dimethyl-4-[2-(4-methylbenzyloxy)-phenyl]-pyridin-3,5-dicarbonsäure-monosec.-butylester vom Schmp. 133 bis 140 °C unter Zersetzung.

Beispiel 128

1,4-Dihydro-2,6-dimethyl-4-[2-(3-trifluormethylbenzyloxy)-phenyl]-pyridin-3,5-dicarbonsäuremonomethylester

vom Schmp. 182 °C unter Zersetzung.

Beispiel 129

1,4-Dihydro-2,6-dimethyl-4-[2-(3-trifluormethylbenzyloxy)-phenyl]-pyridin-3,5-dicarbonsäuremonoisobutylester vom Schmp. 150 bis 153 °C unter Zersetzung.

Beispiel 130

1,4-Dihydro-2,6-dimethyl-4-[2-(3-trifluormethylbenzyloxxy)-phenyl]-pyridin-3,5-dicarbonsäuremono-sec.-butylester
vom Schmp. 144 °C unter Zersetzung.

Beispiel 131

1,4-Dihydro-2,6-dimethyl-4-[2-(3-nitrobenzyloxy)-phenyl]-pyridin-3,5-dicarbonsäure-monomethylester vom Schmp. 167 °C unter Zersetzung.

Beispiel 132

1,4-Dihydro-2,6-dimethyl-4-[2-(3-nitrobenzyloxy)-phenyl]-pyridin-3,5-dicarbonsäure-monoethylester vom Schmp. 172 °C unter Zersetzung.

Beispiel 133

1,4-Dihydro-2,6-dimethyl-4-[2-(4-fluorbenzyloxy)-phenyl]-pyridin-3,5-dicarbonsäure-monoethylester vom Schmp. 154 bis 156 °C unter Zersetzung.

Beispiel 134

1,4-Dihydro-2,6-dimethyl-4-[2-(4-fluorbenzyloxy)-phenyl]-pyridin-3,5-dicarbonsäure-monomethylester vom Schmp. 181 bis 186 °C unter Zersetzung.

Bespiel 135

1,4-Dihydro-2,6-dimethyl-4-(2-phenylpropylmercaptophenyl)-pyridin-3,5-dicarbonsäuremonomethylester vom Schmp. ab 94 °C unter Zersetzung.

Beispiel 136

1,4-Dihydro-2,6-dimethyl-4-[2-(3,5-dimethylbenzyloxy)-phenyl]-pyridin-3,5-dicarbonsäuremonomethylester vom Schmp. ab 98 °C unter Zersetzung.

Beispiel 137

1,4-Dihydro-2,6-dimethyl-4-[2-(3,5-dimethylbenzyloxy)-phenyl]-pyridin-3,5-dicarbonsäuremono-n-butylester vom Schmp. ab 85 °C unter Zersetzung.

Beispiel 138

1,4-Dihydro-2,6-dimethyl-4-[3-methylbenzyloxy)-phenyl]-pyridin-3,5-dicarbonsäure-monomethylester vom Schmp. 140 bis 145 °C unter Zersetzung.

Beispiel 139

1,4-Dihydro-2,6-dimethyl-4-[2-(3-methylbenzyloxy)-phenyl]-pyridin-3,5-dicarbonsäure-mono-n-butylester vom Schmp. 132 bis 139 °C unter Zersetzung.

Beispiel 140

1,4-Dihydro-2,6-dimethyl-4-[2-(3-fluorbenzyloxy)-phenyl]-pyridin-3,5-dicarbonsäure-monomethylester vom Schmp. 146 bis 149 °C unter Zersetzung.

Beispiel 141

1,4-Dihydro-2,6-dimethyl-4-[2-(3-fluorbenzyloxy)-phenyl]-pyridin-3,5-dicarbonsäure-mono-n-butylester vom Schmp. 141 bis 144 °C unter Zersetzung.

Beispiel 142

1,4-Dihydro-2,6-dimethyl-4-[2-(3-methoxybenzyloxy)-phenyl]-pyridin-3,5-dicarbonsäuremonomethylester vom Schmp. 155 bis 160 °C unter Zersetzung.

Beispiel 143

1,4-Dihydro-2,6-dimethyl-4-[2-(3-methoxybenzyloxy)-phenyl]-pyridin-3,5-dicarbonsäuremono-n-butylester vom Schmp. 145 bis 149 °C unter Zersetzung.

Beispiel 144

1-Ethyl-2-methyl-4-[2-(3-trifluormethylbenzylthio)-phenyl]-5-oxo-1,4,5,7-tetrahydrofuro[3,4-b]-pyridin-3-carbonsäureethylester.
Fp.: 117 bis 119 °C.

Beispiel 145

2-Methyl-4-[2-(3-trifluormethylbenzylthio)-phenyl]-5-oxo-1,4,5,7-tetrahydrofuro[3,4-b]-pyridin-3-carbonsäure-2-methoxyethylester.
Fp.: 124 bis 125 °C.

Beispiel 146

2-Methyl-4-[2-(3-trifluormethylbenzylthio)-phenyl]-5-oxo-1,4,5,7-tetrahydrofuro[3,4-b]-pyridin-3-carbonsäurebutylester.
Fp.: 112 bis 120 °C.

Beispiel 147

2-Methyl-4-[2-(3-trifluormethylbenzyloxy)-phenyl]-5-oxo-1,4,5,7-tetrahydrofuro[3,4-b]-pyridin-3-carbonsäureethylester.
FFp.: 180 bis 183 °C.

Beispiel 148

2-Methyl-4-[2-(3-nitrobenzylthio)phenyl]-5-oxo-1,4,5,7-tetrahydrofuro[3,4-b]pyridin-3-carbonsäureethylester.
Fp.: 164 bis 166 °C.

Beispiel 149

2-Methyl-4-[2-(3-nitrobenzylthio)phenyl]-5-oxo-1,4,5,7-tetrahydrofuro[3,4-b]pyridin-3-carbonsäure-n-butylester.
Fp.: 137 bis 139 °C.

Beispiel 150

2-Methyl-4-[2-(3-methylbenzylsulfinyl)phenyl]-5-oxo-1,4,5,7-tetrahydrofuro[3,4-b]pyridin-3-carbonsäure-

ethylester.
Fp.:243-245 °C.

Beispiel 151

4-[2-(Benzylthio)phenyl]-2-methyl-5-oxo-4,5,6,7-tetrahydro-1H-pyrrolo[3,4-b]pyridin-3-carbonsäureethylester.
Fp.: 243 bis 246 °C.

Beispiel 152

4-[2-(Benzylsulfonyl)phenyl]-2-methyl-5-oxo-1,4,5,7-tetrahydrofuro[3,4-b]pyridin-3-carbonsäureethylester.
Fp.: 150 bis 152 °C.

Beispiel 153

2-Methyl-4-[2-(3-trifluormethylbenzylthio)phenyl]-5-oxo-1,4,5,7-tetrahydrofuro[3,4-b]-3-carbonsäuremethylester.
Fp.: 178 bis 181 °C.

Beispiel 154

4-[2-(4-tert.-Butylbenzylthio)phenyl]-2-methyl-5-oxo-1,4,5,7-tetrahydrofuro[3,4-b]pyridin-3-carbonsäurebutylester.
Fp.: 103 °C.

Beispiel 155

2-Methyl-4-[2-(3-trifluormethylbenzyl)phenyl]-5-oxo-1,4,5,7-tetrahydrofuro[3,4-b]pyridin-3-carbonsäureethylester.
Fp.: 189 °C.

Beispiel 156

2-Methyl-4-[2-(3-methylbenzylthio)phenyl]-5-oxo-1,4,5,7-tetrahydrofuro[3,4-b]pyridin-3-carbonsäureethylester.
Fp.: 150 bis 155 °C.

Beispiel 157

4-[2-(3-Chlorbenzylthio)phenyl]-2-methyl-5-oxo-1,4,5,7-tetrahydrofuro[3,4-b]pyridin-3-carbonsäurebutylester.
Fp.: 167 bis 170 °C.

Beispiel 158

4-[2-(4-Ethoxycarbonylbenzylthio)phenyl]-2-methyl-5-oxo-1,4,5,7-tetrahydrofuro[3,4-b]-pyridin-3-carbonsäureethylester.
Fp.: 157 bis 160 °C.

Beispiel 159

4-[2-(Benzylthio)phenyl]-2-propyl-5-oxo-1,4,5,7-tetrahydrofuro[3,4-b]pyridin-3-carbonsäureethylester.
Fp.: 167 bis 169 °C.

Beispiel 160

4-[2-(Chlorbenzylthio)phenyl]-2-methyl-5-oxo-1,4,5,7-tetrahydrofuro[3,4-b]pyridin-3-carbonsäureethylester.
Fp.: 87 bis 89 °C.

Beispiel 161

4-[2-Benzylthio)phenyl]-2-ethyl-5-oxo-1,4,5,7-tetrahydrofuro[3,4-b]pyridin-3-carbonsäuremethylester.
Fp.: 174 bis 176 °C.

Beispiel 162

4-[2-(4-tert.-Butylbenzylthio)phenyl]-2-methyl-5-oxo-1,4,5,7-tetrahydrofuro[3,4-b]-pyridin-3-carbonsäure-ethylester.
Fp.: 127 bis 130 °C.

Beispiel 163

2-Methyl-4-[2-(3-methylbenzylthio)phenyl]-5-oxo-1,4,5,7-tetrahydrofuro[3,4-b]pyridin-3-carbonsäureethyl-ester.
Fp.: 114 bis 116 °C.

Beispiel 164

4-[2-(4-tert.-Butylbenzylthio)phenyl]-2-methyl-5-oxo-1,4,5,7-tetrahydrofuro(3,4-b]-pyridin-3-carbonsäure-methylester.
Fp.: 112 bis 113 °C.

Beispiel 165

2-Methyl-4-[2-(3-methylbenzylthio)phenyl]-5-oxo-1,4,5,7-tetrahydrofuro[3,4-b]pyridin-3-carbonsäuremethylester.
Fp.: 166 bis 168 °C.

Beispiel 166

4-[2-(3-Chlorbenzylthio)phenyl]-2-methyl-5-oxo-1,4,5,7-tetrahydrofuro[3,4-b]pyridin-3-carbonsäuremethy lester.
Fp.: 209 bis 211 °C.

Beispiel 167

2-Methyl-4-[2-(4-methylbenzylthio)phenyl]-5-oxo-1,4,5,7-tetrahydrofuro[3,4-b]pyridin-3-carbonsäureoctylester.
$^1$H-NMR ($CDCl_3$): δ = 0,9 (t,J = 7 Hz, 3H), 1,0-1,5 (m, 12H), 2,3 und 2,35 (2s, je 3H), 3,75-4,1 (m, 2H), 4,1 und 4,15 (2d, J = 14 Hz, je 1H), 4,45 (s, 2H), 5,5 (s, 1H) 7,0-7,4 (m, 8H), 8,1 (s, NH) ppm.

Beispeil 168

2-Methyl-4-[2-(4-methylbenzylthio)phenyl]-5-oxo-1,4,5,7-tetrahydrofuro[3,4-b]pyridin-3-carbonsäurebutylester.
Fp.: 87 bis 89 °C.

Beispiel 169

4-[2-(Benzylthio)phenyl]-2-methyl-5-oxo-1-propyl-1,4,5,7-tetrahydrofuro[3,4-b]pyridin-3-carbonsäureethyl-ester.
Fp.: 132 °C.

Beispiel 170

1-Allyl-4-[2-(benzylthio)phenyl]-2-methyl-5-oxo-1,4,5,7-tetrahydrofuro[3,4-b]pyridin-3-carbonsäureethylester.
Fp.: 115 bis 177 °C.

Beispiel 171

2-Methyl-4-[2-(4-methylbenzylthio)phenyl]-5-oxo-1,4,5,7-tetrahydrofuro[3,4-b]pyridin-3-carbonsäure-(2-methoxy)-ethylester.
Fp.: 137 bis 139 °C.

Beispiel 172

2-Methyl-4-[2-(4-methylbenzyloxy)phenyl]-5-oxo-1,4,5,7-tetrahydrofuro[3,4-b]pyridin-3-carbonsäureethylester.
Fp.: 208 bis 209 °C.

Beispiel 173

4-[2-(benzylthio)phenyl]-1-ethyl-2-methyl-5-oxo-1,4,5,7-tetrahydrofuro[3,4-b]pyridin-3-carbonsäureethylester.
Fp.: 80 bis 82 °C.

Beispiel 174

4-[2-(Benzylthio)phenyl]-1,2-dimethyl-5-oxo-1,4,5,7-tetrahydrofuro[3,4-b]pyridin-3-carbonsäureethylester.
Fp.: 188-190 °C.

Beispiel 175

4-[3-(Benzylthio)phenyl]-2-methyl-5-oxo-1,4,5,7-tetrahydrofuro[3,4-b]pyridin-3-carbonsäureethylester.
Fp.: 183 bis 185 °C.

Beispiel 176

4-[2-(4-Methoxycarbonylbenzylthio)phenyl]-2-methyl-5-oxo-1,4,5,7-tetrahydrofuro[3,4-b]-pyridin-3-carbonsäureethylester.
Fp.: 102 bis 110 °C.

Beispiel 177

2-Methyl-4-[2-(1-phenylethylthio)phenyl]-5-oxo-1,4,5,7-tetrahydrofuro[3,4-b]pyridin-3-carbonsäureethylester.
Fp.: 93 bis 110 °C (2 : 3-Diastereomerengemisch).

Beispiel 178

2-Methyl-4-[2-(2-methylbenzylthio)phenyl]-5-oxo-1,4,5,7-tetrahydrofuro[3,4-b]pyridin--3-carbonsäureethylester.
Fp.: 217 bis 219 °C.

Beispiel 179

2-Methyl-5-oxo-4-[3-phenylpropylsulfinyl)-phenyl]-1,4,5,7-tetrahydrofuro[3,4-b]pyridin-3-carbonsäureethylester.
Fp.: 185 bis 186 °C.

Beispiel 180

2-Methyl-4-[2-(4-nitrobenzylthio)phenyl]-5-oxo-1,4,5,7-tetrahydrofuro[3,4-b]pyridin-3-carbonsäureethylester.
Fp.: 168 bis 170 °C.

Beispiel 181

4-[2-(Methoxycarbonyl)phenyl]-2-methyl-5-oxo-1,4,5,7-tetrahydrofuro[3,4-b]pyridin-3-carbonsäureethylester.
Fp.: 240 bis 249 °C.

Beispiel 182

4-[2-(Butylthio)phenyl]-2-methyl-5-oxo-1,4,5,7-tetrahydrofuro[3,4-b]pyridin-3-carbonsäureethylester.
Fp.: 163 bis 167 °C.

Beispiel 183

4-[2-(2,5-Dichlorbenzylthio)phenyl]-2-methyl-5-oxo-1,4,5,7-tetrahydrofuro[3,4-b]-pyridin-3-carbonsäureethylester.
Fp.: 244 bis 250 °C.

Beispiel 184

4-[2-(4-Methoxybenzylthio)phenyl]-2-methyl-5-oxo-1,4,5,7-tetrahydrofuro[3,4-b]pyridin-3-carbonsäureethylester.
Fp.: 83 bis 85 °C.

Beispiel 185

4-[2-(4-Chlorbenzylthio)phenyl]-2-methyl-5-oxo-1,4,5,7-tetrahydrofuro[3,4-b]pyridin-3-carbonsäureethylester.
Fp.: 97 bis 99 °C.

Beispiel 186

2-Methyl-4-[2-(1-methylethylthio)phenyl]-5-oxo-1,4,5,7-tetrahydrofuro[3,4-b]pyridin-3-carbonsäureethylester.
Fp.: 193 bis 194 °C.

Beispiel 187

2-Methyl-5-oxo-4-[2-(3-phenylpropylthio)-phenyl]-1,4,5,7-tetrahydro[3,4-b]pyridin-3-carbonsäureethylester.
Fp.: 179 bis 180 °C.

Beispiel 188

2-Methyl-5-oxo-4-[2-(tetralin-2-yl-methylthio)-phenyl]-1,4,5,7-tetrahydrofuro-[3,4-b]pyridin-3-carbonsäureethylester.
Fp.: 102 bis 104 °C.

Beispiel 189

4-(2,3-Dimethylphenyl)-2-methyl-5-oxo-1,4,5,7-tetrahydrofuro[3,4-b]pyridin-3-carbonsäureethylester.
Fp.: 225 bis 227 °C.

Beispiel 190

2-Methyl-5-oxo-[2-(2-[phenylcarbonyloxy]-ethylthio)-phenyl]-1,4,5,7-tetrahydrofuro-[3,4-b]pyridin-3-carbonsäureethylester.
Fp.: 190 bis 196 °C.

Beispiel 191

2-Methyl-4-[2-(1-naphtylmethylthio)phenyl]-5-oxo-1,4,5,7-tetrahydrofuro[3,4-b]pyridin-3-carbonsäureethylester.
Fp.: 93 bis 100 °C.

Beispiel 192

4-[2-(Benzylsulfinylthio)phenyl]-2-methyl-5-oxo-1,4,5,7-tetrahydrofuro[3,4-b]pyridin-3-carbonsäureethylester.
Fp.: 250 °C.

Beispiel 193

4-[2-(3,4-Dichlorbenzylthio)phenyl]-2-methyl-5-oxo-1,4,5,7-tetrahydrofuro[3,4-b]-pyridin-3-carbonsäure-ethylester.
Fp.: 90 bis 95 °C.

Beispiel 194

4-(3-Benzyloxyphenyl]-2-methyl-5-oxo-1,4,5,7-tetrahydrofuro[3,4-b]pyridin-3-carbonsäureethylester.
Fp.: 103 bis 104 °C.

Beispiel 195

4-[2-(2-Hydroxyethylthio)phenyl]-2-methyl-5-oxo-1,4,5,7-tetrahydrofuro[3,4-b]pyridin-3-carbonsäureethyl-ester.
Fp.: 130 bis 134 °C.

Beispiel 196

4-(2-Benzylphenyl)-2-methyl-5-oxo-1,4,5,7-tetrahydrofuro[3,4-b]pyridin-3-carbonsäureethylester.
Fp.: 239 bis 240 °C.

Beispiel 197

4-(2-Ethylthio)phenyl-2-metyl-5-oxo-1,4,5,7-tetrahydrofuro[3,4-b]pyridin-3-carbonsäureethylester.
Fp.: 235 bis 236 °C.

Beispiel 198

2-Methyl-4-[2-(methylthio)phenyl]-5-oxo-1,4,5,7-tetrahydrofuro[3,4-b]pyridin-3-carbonsäureethylester.
Fp.: 233 bis 234 °C.

Beispiel 199

2-Methyl-4-[2-(2-phenylethylthio)phenyl]-5-oxo-1,4,5,7-tetrahydrofuro[3,4-b]pyridin-3-carbonsäureethylester.
Fp.: 184 bis 185 °C.

Beispiel 200

2-Methyl-4-[2-(4-methylbenzylthio)phenyl]-5-oxo-1,4,5,7-terahydrofuro[3,4-b]pyridin-carbonsäureethylester
Fp.: 132 bis 134 °C.

Beispiel 201

4-(2-Benzyloxyphenyl)-2-metyl-5-oxo-1,4,5,7-tetrahydrofuro[3,4-b]pyridin-3-carbonsäuremethylester.
Fp.: 245 bis 246 °C.

Beispiel 202

4-(2-Ethyphenyl)-2-methyl-5-oxo-1,4,5,7-tetrahydrofuro[3,4-b]pyridin-3-carbonsäureethylester.
Fp.: 197 bis 200 °C.

Beispiel 203

4-(2-Benzylthiophenyl)-2-methyl-5-oxo-1,4,5,7-tetrahydrofuro[3,4-b]pyridin-3-carbonsäureoctylester. Fp.: 212 °C.

Beispiel 204

4-(2-Benzylthiophenyl)-2-methyl-5-oxo-1,4,5,7-tetrahydrofuro[3,4-b]pyridin-3-carbonsäurebutylester (amorphe Substanz).

Beispiel 205

4-(2-Benzyloxyphenyl)-2-methyl-5-oxo-1,4,5,7-tetrahydrofuro[3,4-b]pyridin-3-carbonsäureethylester. Fp.: 177 °C.

Beispiel 206

4-(2-Benzylthiophenyl)-2-methyl-5-oxo-1,4,5,7-tetrahydrofuro[3,4-b]pyridin-3-carbonsäuremethylester. Fp.: 215 °C.

Beispiel 207

2-Methyl-5-oxo-4-(2-trifluormethylphenyl)-1,4,5,7-tetrahydrofuro[3,4-b]pyridin-3-carbonsäureethylester. Fp.: 228 bis 230 °C.

Beispiel 208

4-(2-Benzylthiophenyl)-2-methyl-5-oxo-1,4,5,7-tetrahydrofuro[3,4-b]pyridin-3-carbonsäureisopropylester. Fp.: 158 bis 161 °C.

Beispiel 209

2-Methyl-5-oxo-4-(2-phenylphenyl)-1,4,5,7-tetrahydrofuro[3,4-b]pyridin-3-carbonsäureethylester. Fp.: 217 bis 220 °C.

Beispiel 210

2-Methyl-4-(2-methylphenyl)-5-oxo-1,4,5,7-tetrahydrofuro[3,4-b]pyridin-3-carbonsäureisopropylester. Fp.: 208 °C.

Beispiel 211

2-5-Methyl-4-(2-methylphenyl)-5-oxo-1,4,5,7-tetrahydrofuro[3,4-b]pyridin-3-carbonsäurebutylester. Fp.: 167 bis 176 °C.

Beispiel 212

2-Methyl-4-(2-methylphenyl)-5-oxo-1,4,5,7-tetrahydrofuro[3,4-b]pyridin-3-carbonsäureallylester. Fp.: 170 bis 175 °C.

Beispiel 213

3-Acetyl-4-(2-benzylthiophenyl)-2-methyl-5-oxo-1,4,5,7-tetrahydrofuro[3,4-b]pyridin. Fp.: 212 °C.

Beispiel 214

2-Methyl-5-oxo-4-(2-trifluormethylphenyl)-1,4,5,7-tetrahydrofuro[3,4-b]pyridin-3-carbonsäuremethylester. Fp.: 229 bis 233 °C.

Beispiel 215

2-Methyl-5-oxo-4-(2-methylphenyl)-1,4,5,7-tetrahydrofuro[3,4-b]pyridin-3-carbonsäuremethylester. Fp.: 196 bis 201 °C.

Beispiel 216

4-(3-Chlorphenyl)-1,2-dimethyl-5-oxo-1,4,5,7-tetrahydrofuro[3,4-b]pyridin-3-carbonsäure-2-methoxyethylester. Fp.: 128 bis 133 °C.

Beispiel 217

3-Acetyl-2-methyl-5-oxo-4-[2-trifluormethylphenyl]-tetrahydrofuro[3,4-b]pyridin. Fp.: 212 bis 213 °C.

Beispiel 218

2-Methoxymethyl-5-oxo-4-(2-trifluormethylphenyl)-1,4,5,7-tetrahydrofuro-[3,4-b]pyridin-3-carbonsäureethylester. Fp.: 218 bis 219 °C.

Beispiel 219

4-(3-Chlorphenyl)-1,2-dimethyl-5-oxo-1,4,5,7-tetrahydrofuro[3,4-b]pyridin-3-carbonsäureethylester. Fp.: 188 bis 190 °C.

Beispiel 220

4-(3-Chlorphenyl)-2-methyl-5-oxo-1,4,5,7-tetrahydrofuro[3,4-b]pyridin-3- carbonsäurebutylester. Fp.: 142 bis 144 °C.

Beispiel 221

4-(3-Chlorphenyl)-2-methyl-5-oxo-1,4,5,7-tetrahydrofuro[3,4-b]pyridin-3-carbonsäureallylester. Fp.: 164-167 °C.

Beispiel 222

2-Methyl-5-oxo-4-(2-trifluormethylphenyl)-1,4,5,7-tetrahydrofuro[3,4-b]pyridin-3-carbonsäure-82-pyridyl) -methylester. Fp.: 209 bis 210 °C.

Beispiel 223

2-Methyl-5-oxo-1-propyl-4-(2-trifluormethylphenyl)-1,4,5,7-tetrahydrofuro-[3,4-b]pyridin-3-carbonsäure-ethylester. Fp.: 106 bis 107 °C.

Beispiel 224

2-Methyl-5-oxo-4-(2-trifluormethylphenyl)-1,4,5,7-tetrahydrofuro[3,4-b]pyridin-3-carbonsäurebenzylester. Fp.: 218 bis 219 °C.

Beispiel 225

4-(3-Chlorphenyl)-2-methyl-5-oxo-1,4,5,7-tetrahydrofuro[3,4-b]pyridin-3-carbonsäurehexylester.
Fp.: 130 bis 133 °C.

Beispiel 226

1-Ethyl-2-methyl-5-oxo-4-(2-trifluormethylphenyl)-1,4,5,7-tetrahydrofuro-[3,4-b]pyridin-3-carbonsäure-ethylester.
Fp.: 157 bis 160 °C.

Beispiel 227

2-Methyl-5-oxo-4-(2-trifluormethylphenyl)-1,4,5,7-tetrahydrofuro[3,4-b]pyridin-3-carbonsäureoctylester.
Fp.: 119 bis 120 °C.

Beispiel 228

4-(2,3-Dichlorphenyl)-2-methyl-5-oxo-1,4,5,7-tetrahydrofuro[3,4-b]pyridin-3-carbonsäureethylester.
Fp.: 249 bis 251 °C.

Beispiel 229

2-Methyl-5-oxo-4-(3-pyridyl)-1,4,5,7-tetrahydrofuro[3,4-b]pyridin-3-carbonsäureethylester.
Fp.: 192 bis 194 °C.

Beispiel 230

2-Methyl-5-oxo-4-(2-trifluormethoxyphenyl)-1,4,5,7-tetrahydrofuro[3,4-b]pyridin-3-carbonsäureethylester.
Fp.: 174 bis 176 °C.

Beispiel 231

2-Methyl-4-(2-naphthyl)-5-oxo-1,4,5,7-tetrahydrofuro[3,4-b]pyridin-3-carbonsäureethylester.
Fp.: >270 °C.

Beispiel 232

5-Oxo-4-(2-trifluormethylphenyl)-1,2,7-trimethyl-1,4,5,7-tetrahydrofuro[3,4-b]-pyridin-3-carbonsäureethylester.
Fp.: 171 bis 172 °C.

Beispiel 233

2-Methyl-5-oxo-4-(3-trifluormethylphenyl)-1,4,5,7-tetrahydrofuro[3,4-b]pyridin-3-carbonsäureethylester.
Fp.: 180 bis 182 °C.

Beispiel 234

2-Ethyl-5-oxo-4-(2-trifluormethylphenyl)-1,4,5,7-tetrahydrofuro[3,4-b] pyridin-3-carbonsäureethylester.
Fp.: 237 bis 239 °C.

Beispiel 235

2-Methyl-5-oxo-4-(2-trifluormethylphenyl)-1,4,5,7-tetrahydrofuro[3,4-b]pyridin-3-carbonsäureallylester.
Fp.: 190 bis 192 °C.

Beispiel 236

1,2-Dimethyl-5-oxo-4-(2-trifluormethylphenyl)-1,4,5,7-tetrahydrofuro[3,4-b]pyridin-3-carbonsäureethylester. Fp.: 196 bis 197 °C.

Beispiel 237

4-[(2-Fluor-3-chlor)phenyl]-2-methyl-5-oxo-1,4,5,7-tetrahydrofuro[3,4-b]pyridin-3-carbonsäuremethylester. Fp.: 220 bis 223 °C.

Beispiel 238

4-[2-(Cyclohexylmethylthio)phenyl]-2-methyl-5-oxo-1,4,5,7-tetrahydrofuro[3,4-b]pyridin-3-carbonsäurebutylester. Fp.: 123 bis 128 °C.

Beispiel 239

4-[2-(Cyclohexylmethylthio)phenyl]-2-methyl-5-oxo-1,4,5,7-tetrahydrofuro[3,4-b]pyridin-3-carbonsäuree thylester. Fp.: 77 bis 82 °C.

Beispiel 240

2-Methyl-5-oxo-4-(2-trifluormethylphenyl)-1,4,5,7-tetrahydrofuro[3,4-b]pyridin-3-carbonsäurehexylester. Fp.: 164 bis 165 °C.

Beispiel 241

2-Methyl-5-oxo-4-(2-phenylthiophenyl)-1,4,5,7-tetrahydrofuro[3,4-b]pyridin-3-carbonsäureethylester. Fp.: 191 bis 192 °C.

Beispeil 242

4-(2-Chlorphenyl)-2-methyl-5-oxo-1,4,5,7-tetrahydrofuro[3,4-b]pyridin-3-carbonsäureallylester. Fp.: 206 °C.

Beispiel 243

Analog zu dem Verfahren gemäss Beispiel Nr. 35 werden die folgenden Verbindungen durch Hydrierung mit Lithium-Aluminium-Hydrid aus den entsprechenden Diestern erhalten.
1,4-Dihydro-2,3,6-trimethyl-4-(2-chlorphenyl)-pyridin-5-carbonsäureethylester
vom Schmp. 112 °C.

Beispiel 244

1,4-Dihydro-2,3,6-trimethyl-4-(4-chlorphenyl)-pyridin-5-carbonsäuremethylester
vom Schmp. 94 °C.

Beispiel 245

1,4-Dihydo-2,3,6-trimethyl-4-(2-methylphenyl)-pyridin-5-carbonsäuremethylester
vom Schmp. 147 bis 153 °C.

Beispiel 246

1,4-Dihydro-2,3,6-trimethyl-4-[2-(4-methylbenzylthio)-phenyl]-pyridin-5-carbonsäuremethylester
vom Schmp.

109 bis 111 °C.

Beispiel 247

1,4-Dihydro-2,3,6-trimethyl-4-(2-trifluormethylphenyl)-pyridin-5-carbonsäuremethylester vom Schmp. 145 bis 149 °C.

Beispiel 248

1,4-Dihydro-2,3,6-trimethyl-4-(4-methylphenyl)-pyridin-5-carbonsäuremethylester vom Schmp. 146 bis 150 °C.

Beispiel 249

1,4-Dihydro-2,3,6-trimethyl-4-(3-thienyl)-pyridin-5-carbonsäuremethylester vom Schmp. 150 bis 158 °C.

Beispiel 250

1,4-Dihydro-2,3,6-trimethyl-4-(2-benzyloxyphenyl)-pyridin-5-carbonsäuremethylester, isoliert als farbloses Harz.
Massenspektrum: Die wichtigsten Peaks findet man bei:
m/e = 363, 348, 304, 272, 180.

Beispiel 251

1,4-Dihydro-2,3,6-trimethyl-4-[2-(4-chlorbenzyloxy)-phenyl]-pyridin-5-carbonsäuremethylester, isoliert als farbloses Harz.
Massenspektrum: m/e = 396 (180).

Beispiel 252

1,4-Dihydro-2,3,6-trimethyl-4-[2-(2,6-dichlorbenzyloxy)-phenyl]-pyridin-5-carbonsäuremethylester, isoliert als Öl.

Beispiel 253

1,4-Dihydro-2,3,6-trimethyl-4-[2-(4-methylbenzyloxy)-phenyl]-pyridin-5-carbonsäuremethylester, isoliert als Öl.

Beispiel 254

1,4-Dihydro-2,3,6-trimethyl-4-(2-phenethyloxyphenyl)-pyridin-5-carbonsäuremethylester, isoliert als gelbes Öl.

Beispiel 255

1,4-Dihydro-2,3,6-trimethyl-4-(2-phenylmercaptomethoxyphenyl)-pyridin-5-carbonsäuremethylester, isoliert als gelbes Öl.
Massenspektrum: m/e = 395 (180).

Beispeil 256

1,4-Dihydro-2,3,6-trimethyl-4-[3-(4-methylbenzyloxy)-phenyl]-pyridin-5-carbonsäuremethylester, isoliert als gelbes Öl.

Beispeil 257

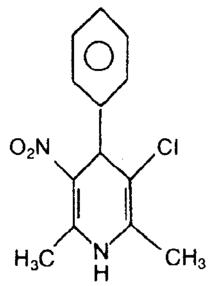

Analog Beispiel 18 erhält man aus 0,1 Mol 2-Amino-1-nitro-1-propen und 0,1 Mol 2-Chlor-1-phenyl-buten-1-on-3 duch Erhitzen in siedendem Ethanol das
3-Chlor-1,4-dihydro-2,6-dimethyl-5-nitro-4-phenylpyridin
vom Schmelzpunkt 192 °C (Isopropanol).
Ausbeute: 28% der Theorie.

Beispeil 258

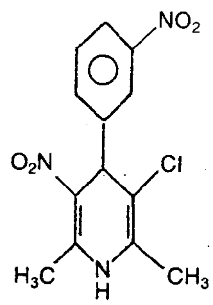

Analog Beispiel 18 werden 0,1 Mol 2-Amino-1-nitro-1-propen und 0,1 Mol 2-Chlor-1-(3-nitrophenyl)-buten-1-on-3 in siedendem Ethanol erhitzt. Man erhält
3-Chlor-1,4-dihydro-2,6-dimethyl-5-nitro-4-(3-nitrophenyl)-pyridin
vom Schmp. 198 °C (Zers.).
Ausbeute:

Beispeil 259

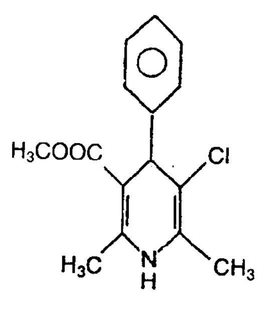

Analog Beispiel 18 werden 0,1 Mol Aminocrotonsäuremethylester und 2-Chlor-1-phenyl-buten-1-on-3 in siedendem Ethanol erhizt. Man erhält 3-Chlor-1,4-dihydro-2,6-dimethyl-4-phenylpyridin-5-carbonsäuremethylester
vom Schmp. 147 °C.
Ausbeute: 23% der Theorie.

Beispiel 260

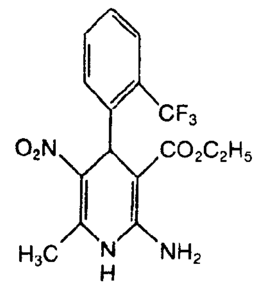

Analog Beispiel 18 wurde duch Umsetzung von 2-Nitro-1-(2-trifluormethylphenyl)-buten-1-on-3 und Amidinoessigsäureethylester in Ethanol 2-Amino-1,4-dihydro-6-methyl-5-nitro-4-(2-trifluormethylphenyl)-pyridin-5-carbon-säureester vom Schmp. 210 °C (Isopropanol) erhalten.
Ausbeute: 32% der Theorie.

Beispiel 261

4-(2-Benzylthiophenyl)-3-cyano-1,4-dihydro-2,6-dimethyl-pyridin-5-carbonsäuremethylester

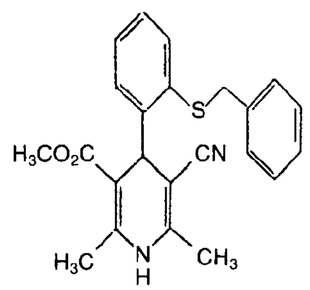

Eine Lösung von 22,7 g (69 mMol) 2-(2-Benzylthiobenzyliden)-acetessigsäuremethylester und 5,7 g (69 mMol) β-Aminocrotonsäurenitril in 80 ml Methanol wurde 12 Stunden unter Stickstoff zum Sieden eritzt. Anschliessend wurde das Lösungsmittel i.V. eingeengt und der ölige Rückstand durch Verreiben mit wenig Ether zur Kristallisation gebracht. Das Rohprodukt wurde abgesaugt und aus Methanol umkristallisiert.
Schmelzpunkt: 183-185 °C.
Ausbeute: 15,7 g (58%).

Beispiel 262

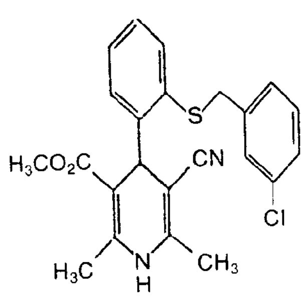

Analog Beispiel 261 wurde durch Umsetzung von
2-(2-(3-Chlorbenzylthio)-benzyliden)-acetessigsäuremethylester
und β-Aminocrotonsäurenitril in Methanol der 4-(2-(3-Chlorbenzylthio)-phenyl)-3-cyano-1,4-dihydro-2,6-dimethylpyridin-5-carbonsäuremethylester
vom Fp.: 155 °C (Methanol) erhalten.
Ausbeute: 51 % der Theorie.

Beispiel 263

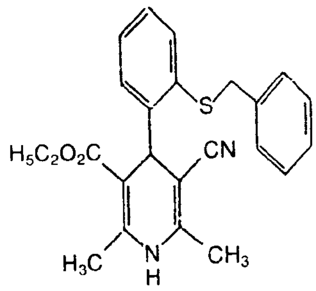

Analog Beispiel 261 wurde durch Umsetzung von 2-(2-Benzylthiobenzyliden)-acetessigsäureethylester und β-Aminocrotonsäurenitril in Ethanol der 4-(2-Benzylthiophenyl)-3-cyano-1,4-dihydro-2,6-dimethyl-pyridin-5-carbonsäureethylester vom Fp.: 127 °C erhalten.
Ausbeute: 49% der Theorie.

Beispeil 264

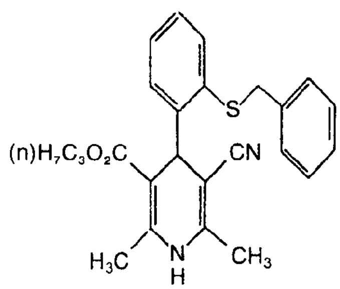

Analog Beispiel 261 wurde durch Umsetzung von 2-(2-Benzylthiobenzyliden)-acetessigsäure-n-propylester und β-Aminocrotonsäurenitril in Ethanol der 4-(2-Benzylthiophenyl)-3-cyano-1,4-dihydro-2,6-dimethyl-pyridin-5-carbonsäure-n-propylester vom Fp.: 101 °C erhalten.
Ausbeute: 45% der Theorie.

Beispeil 265

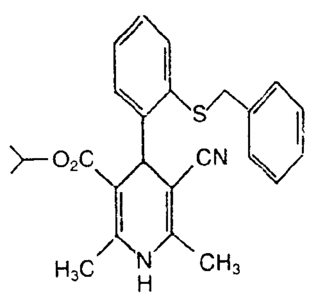

Analog Beispiel 261 wurde durch Umsetzung von 2-(2-Benzylthiobenzyliden)-acetessigsäureisopropylester und β-Aminocrotonsäurenitril in Ethanol der 4-(2-Benzylthiophenyl)-3-cyano-1,4-dihydro-2,6-dimethylpyridin-5-carbonsäureisopropylester vom Fp.: 152 °C erhalten.
Ausbeute: 61 % der Theorie.

## Patentansprüche

1. Verwendung von 1,4-Dihydropyridinen der allgemeinen Formel (I)

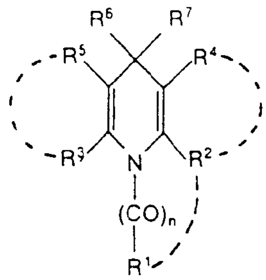

(I)

in welcher
n für 0, 1 oder 2 steht,

$R^1$

a) für Wasserstoff, einen geradkettigen, verzweigten, cyclischen, gesättigten oder ungesättigten aliphatischen Kohlenwasserstoffrest steht, der gegebenenfalls 1 bis 3 gleiche oder verschiedene Hetero-Kettenglieder aus der Gruppe O, CO, $SO_m$ (m = 0, 1 oder 2), = N-, $NR^I$ oder $SiR^{II}R^{III}$ enthält und wobei dieser Kohlenwasserstoffrest gegebenenfalls substituiert ist durch Halogen, $NO_2$, CN, $N_3$, Hydroxy, Aryl oder Heteroaryl oder

b) für einen Aryl- oder Heteroarylrest steht, wobei diese Reste gegebenenlalls 1 bis 5 gleiche oder verschiedene Substituenten aus der Gruppe Aryl, Alkyl, Alkenyl, Alkinyl, Alkenoxy, Alkinoxy, Aralkyl, Acyl, Alkylen, Dioxyalkylen, Halogen, $CF_3$, $OCF_3$, $SCF_3$, $NO_2$, NO, CN, $N_3$, $COR^{IV}$, $COOR^V$, $OR^{VI}$, $NR^I$ oder $NR^{VII}R^{VIII}$ tragen, wobei die Alkyl-, Alkoxy- und Arylreste der oben genannten Substituenten ihrerseits wieder durch Halogen, $COOR^V$ oder $NR^{VII}R^{VIII}$ substituiert sein können oder

c) für den Rest $NR^{VII}R^I$ steht, und wobei die unter a), b) und c) genannten Reste $R^I$, $R^{II}$, $R^{III}$, $R^{IV}$,, $R^V$, $R^{VI}$, $R^{VII}$ und $R^{VIII}$ die unten angegebene bedeutung haben,

$R^2$

a) für einen der unter $R^1$ angegebenen Substituenten steht aber nicht mit diesem identisch sein muss oder

b) für die Reste $NHR^I$ oder

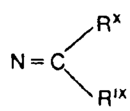

steht,

wobei $R^I$, $R^{IX}$ und $R^X$ die unten angegebene Bedeutung besitzen,

oder die Substituenten $R^1$ und $R^2$ gemeinsam einen 5- bis 8-gliedrigen, gesättigen oder ungesättigten Ring bilden, der gegebenenfalls 1, 2 oder 3 gleiche oder verschiedene Ringglieder aus der Gruppe O, S, $NR^I$ oder CO enthält und der gegebenenfalls 1 bis 3 gleiche oder verschiedene Substituenten aus der Gruppe Halogen, Hydroxy, Alkyl, Alkoxy, Aryl oder Aralkyl enthält,

$R^3$ für einen der unter $R^2$ angegebenen Substituenten steht, und mit diesem gleich oder von diesem verschieden ist, wobei nur einer der beiden Substituenten $R^2$ oder $R^3$ jeweils für Alkoxy, Alkylthio oder $NHR^I$ steht,

$R^4$

a) für Wasserstoff, $NO_2$, NO, CN, $SO_mR^{XI}$(m = 0, 1 oder 2), Halogen,

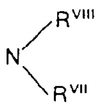, 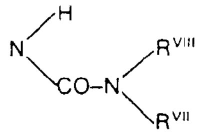, 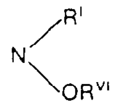

oder 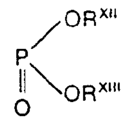

steht, wobei $R^IR^{VII}$, $R^{VIII}$, $R^{XI}$ un $R^{XIII}$ die unten angegebene Bedeutung haben, oder

b) für einen verzweigen oder unverzweigten, cyclischen, gesättigten oder ungesättigten aliphatischen Kohlenwasserstoffrest steht, der gegebenenfalls substituiert ist durch Halogen, OH, CN, Alkoxy, Alkylthio, Aryloxy, $COOR^V$ oder

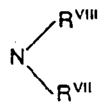,

wobei $R^V$, $R^{VII}$ und $R^{VIII}$ die unten angegebene Bedeutung haben, oder

c) für einen aromatischen Kohlenwasserstoffrest oder für einen 5- bis 7-gliedrigen gesättigten oder ungesättigten Heteroring mit 1 bis 4 gleichen oder verschiedenen Heterogliedern aus der Gruppe O, S, -N =, $NR^I$, wobei dieser Heteroring entweder über ein Kohlenstoffatom oder ein Stickstoffatom mit dem Dihydropyridinring verknüpft ist, und wobei der aromatische Kohlenwasserstoffrest und die Heteroringe gegebenenlalls 1 bis 3 gleiche oder verschiedene Substituenten aus der Gruppe Halogen, OH, CN, $CF_3$, $OCF_3$, $SCF_3$, $NO_2$, Alkyl, Alkoxy, Aryl und

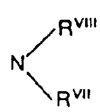,

tragen,

oder

d) für den Rest

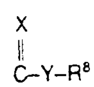

steht, wobei X Sauerstoff, Schwefel oder $NR^I$ bedeutet und Y für eine einlache bindung, O, S oder $NR^I$ steht, und $R^8$ die für $R^1$ angegebene bedeutung hat und mit dem Substituenten $R^1$ gleich oder von diesem verschieden ist, oder

e) für den Rest

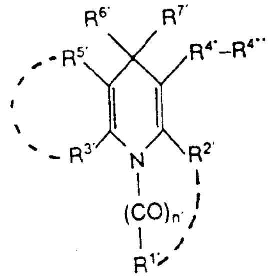

steht,

wobei n′, $R^{1'}$, $R^{2'}$, $R^{3'}$, $R^{5'}$, $R^{6'}$ und $R^{7'}$ die für n, $R^1$, $R^2$, $R^3$, $R^5$, $R^6$ und $R^7$ angegebene Bedeutung haben und mit diesen gleich oder von diesen verschieden sind, und

$R^{4*}$ und $R^{4**}$ gleich oder verschieden sind und jeweils für einen um ein Wasserstoff verminderten Rest der für $R^4$ unter a) bis d) angegebenen Substituenten stehen, oder

$R^2$ und $R^4$ gemeinsam einen verzweigten, unverzweigten, gesättigten oder ungesättigten 5- bis 8-gliedrigen Ring bilden, der gegebenenfalls 1, 2 oder 3 gleiche oder verschiedene Ringglieder aus der Gruppe O, CO, CS, C = $NR^I$, = N-, $NR^I$, $SO_m$ (m = 0, 1 oder 2) oder $SiR^{II}R^{III}$ enthält, und der gegebenenfalls substituiert ist durch Halogen, Hydroxy, Alkoxy, Aryl,

Aralkyl

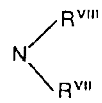 ,

oder durch eine geradkettige oder verzweigte Alkylenkette mit 3 bis 8 Kohlenstollatomen disubstituiert ist, wobei dieser gemeinsame Ring von $R^2$ und $R^4$ auch direckt mit dem gemeinsamen Ring von $R^1$ und $R^2$ anneliert sein kann,

wobei die Reste $R^I$, $R^{II}$, $R^{III}$, $R^{VII}$, $R^{VIII}$ die unten angegebene Bedeutung haben,

$R^5$ die für $R^4$ angegeben Bedeutung besitzt und mit $R^4$ gleich oder von diesem verschieden ist, oder $R^5$ und $R^3$ gemeinsam einem Ring bilden, der mit dem durch $R^2$ und $R^4$ gebildeten Ring gleich oder von diesem verschieden ist,

$R^6$ für Wasserstoff, Alkyl oder Halogenalkyl steht, und

$R^7$

a) für einen gesättigten, ungesättigten, cyclischen, geradkettigen oder verzweigten aliphatischen Kohlenwasserstoffrest steht, der gegebenenfalls substituiert ist durch Halogen, Aryl oder Heteroaryl, oder

b) für einen Aryl- oder Heteroarylrest sleht, der gegebenenfalls 1 bis 5 gleiche oder verschiedene Substituenten aus der Gruppe $NO_2$, CN, $N_3$, NO, $CF_3$, Halogen, $COR^{IV}$, $COR^V$. $OR^{VI}$,

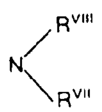 , $SO_mR^{XI}$ (m = 0, 1 oder 2), 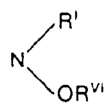 ,

Alkyl, Aryl, Alkenyl, Alkinyl, Alkenoxy, Alkinoxy. Aralkyl, Acyl, Alkylen oder Dioxyalkylen enthält, wobei die vorgenannten Alkyl- und Aryl-Substituenten ihrerseits wieder durch Halogen, $COOR^V$ oder

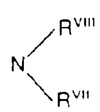 ,

substituiert sei n können, und

wobei in den vorgenannten Definitionen der Substituenten $R^1$ bis $R^8$

$R^I$ für Wasserstoff. Alkyl, Aryl, Aralkyl, Heteroaryl oder Acyl steht,

$R^{II}$ und $R^{III}$ gleich oder verschieden sind und jeweils für Alkyl, Aryl, Aralkyl oder Heteroaryl stehen,

$R^{IV}$, $R^V$ und $R^{VI}$ jeweils gleich oder verschieden sind und für Wasserstoff, Alkyl, Aryl, Aralkyl oder Heteroaryl stehen, wobei die Alkyl- und Arylreste gegebenenfalls durch Halogen, Nitro, Trifluormethyl, Alkoxy, Alkylthio oder Alkyl substituiert sind,

$R^{VII}$ und $R^{VIII}$ jeweils gleich oder verschieden sind und für Wasserstoff, Aryl oder Aralkyl stehen oder für Alkyl, welches gegebenenlalls durch O, S oder $NR^I$ unterbrochen ist, stehen oder gemeinsam mit dem Stickstollatom einen 5- bis 7-gliedrigen Ring bilden, der 1 oder 2 gleiche oder verschiedene Heteroringglieder aus der Gruppe O, S, oder $NR^I$ enthalten kann oder

wobei einer der Reste $R^{VII}$ oder $R^{VIII}$ für eine aliphatische Acylgruppe mit bis zu 6 Kohlenstoffatomen steht, $R^{IX}$, $R^X$, $R^{XI}$ $R^{XII}$ und $R^{XIII}$ jeweils gleich oder verschieden sind und für Alkyl, Aryl oder Aralkyl stehen,

wobei die unter $R^1$ bis $R^8$ und unter $R^I$ bis $R^{XIII}$ genannten Alkyl-, Aryl-, Aralkyl-, Heteroaryl- und Acylreste sowie der mit $R^{VII}$ und $R^{VIII}$ gebildete Heteroring ihrerseits gegebenenfalls substituiert sind durch OH, $CF_3$, $OCF_3$, CN, $NO_2$, Halogen, niederes Thioalkyl, niederes Alkyl, niederes Alkoxy, Aryl oder Aralkyl,

und wobei als Heteroaryl folgende Substituenten genannt seien:
Thienyl, Fuyl, Pyryl, Pyridyl, Chinolyl, Isochinoly, Pyrimidyl, Pyradazinyl, Chinazolyl, Chinoxalyl, Benzothienyl, Pyrazolyl, Imidazolyl, Oxazolyl, Isoxazolyl, Thiazolyl, Triazolyl, Oxydiazolyl, Pyrazinyl, Oxazinyl, Thiazinyl, Indolizinyl, Indolyl, Benzofuranyl, Indazolyl, Benzothienyl, Benzimidazolyl, Benzoxazolyl, Benzisoxazolyl, Benzthiazolyl, Benztriazolyl, benzoxadiazolyl, Cinnolinyl, Phthalazinyl, Naphthyridinyl oder Benzotriazinyl,
in Form von Isomeren, Isomerengemischen, Racematen und optischen Antipoden sowie ihrer pharmazeutisch unbedenklichen Salze, die ab einer Konzentration von $10^{-5}$ g/ml am linken isolierten Vorhof des Meerschweinchenherzens eine Kontraktionsverstärkung um mindestens 25% bewirken, zur Herstellung von Arzneimitteln, die den $Ca^{++}$ Einstrom in die Zelle erhöhen.

2. Verwendung gemäss Anspruch 1 von Verbindungen der allgemeinen Formel (I) zur Herstellung von Arzneimitteln mit positiv-inotroper Wirkung.

3. Verwendung gemäss Anspruch 1 von Verbindungen der allgemeinen Formel (I) zur Herstellung von Arzneimitteln mit Cardiotonika-Wirkung.

4. Verwendung gemäss Anspruch 1 von Verbindungen der allgemeinen Formel (I) zur Herstellung von Arzneimitteln mit Antihypotonikumswirkung.

5. Verwendung gemäss Anspruch 1 von Verbindungen der allgemeinen Formel (I) zur Herstellung von Arzneimitteln zur Senkung von Blutzukker.

6. Verwendung gemäss Anspruch 1 von Verbindungen der allgemeinen Formel (I) zur Herstellung von Arzneimitteln zur Abschwellung von Schleimhäuten.

7. Verwendung gemäss Anspruch 1 von Verbindungen der allgemeinen Formel (I) zur Herstellung von Arzneimitteln zur Beeiflussung des Salzund Flüssigkeitshaushaltes.

8. Verwendung nach Ansprüchen 1 bis 7 von Verbindungen der allgemeinen Formel (I) gemäss Anspruch 1, in welcher
n für 0, 1 oder 2 steht,
$R^1$

a) für Wasserstoff, einen geradkettigen, verzweigten cyclischen gesättigten oder ungesättigten aliphatischen Kohlenwasserstoffrest mit bis zu 10 C-Atomen steht, der gegebenenfalls 1 oder 2 gleiche oder verschiedene Heterokettenglieder aus der Gruppe O, CO, S, $SO_2$, =N- oder $NR^I$ enthält und wobei dieser Kohlenwasserstoffrest gegebenenfalls substituiert ist durch Halogen, $NO_2$, CN, $N_3$, Hydroxy, mit 1 bis 4 C-Atomen, Phenyl, Naphthyl oder Heteroaryl oder
b) für einen Phenyl-, Naphthyl- oder Heteroarylrest steht, wobei diese Reste gegebenenfalls 1 bis 3 gleiche oder verschiedene Substituenten aus der Gruppe Phenyl, Alkyl, Alkenyl, Alkinyl, Alkenoxy mit jeweils bis zu 4 C-Atomen, Aralkyl mit 7 bis 14 C-Atomen, Acyl mit bis zu 6 C-Atomen, Alkylen, Dioxyalkylen mit bis zu 4 Kohlenstolfatomen in der Alkylenkette, Halogen, $CF_3$, $OCF_3$ $SCF_3$, $NO_2$, CN, $N_3$, $COR^{IV}$, $COOR^V$, $OR^{VI}$, $NR^I$ oder $NR^{VII}R^{VIII}$ tragen, wobei die Alkyl-, Alkoxy- und Arylreste der oben genannten Substituenten ihrerseits wieder durch Halogen, $COOR^V$ oder $NR^{VII}R^{VIII}$ substituiert sein können oder
c) für den Rest $NR^{VII}R^I$ steht,
und wobei die unter a), b) und c) genannten Reste $R^I$, $R^{II}$, $R^{III}$, $R^{IV}$, $R^V$, $R^{VI}$, $R^{VII}$ und $R^{VIII}$ die unten angegebene bedeutung haben,

$R^2$ ein

a) für einen der unter $R^1$ angegebenen Substituenten steht aber nicht mit diesen identisch sein muss oder
b) für die Reste $NHR^I$ oder

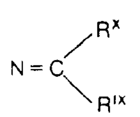 steht,

wobei $R^I$, $R^{IX}$ und $R^X$ die unten angegebene bedeutung besitzen,
oder die Substituenten

$R^1$ und $R^2$ gemeinsam einen 5- bis 7-gliedrigen gesättigten oder ungesättigten Ring bilden, der gegebenenfalls ein oder zwei gleiche oder verschiedene Ringglieder aus der Gruppe O, S, $NR^I$ oder CO enthält und der gegebenenfalls ein bis drei gleiche oder verschiedene Substituenten aus der Gruppe Halogen, Hydroxy, Alkyl, Alkoxy mit jeweils 1 bis 4 C-Atomen, Phenyl, Naphthyl oder Aralkyl mit 7 bis 14 C-Atomen enthält,

$R^3$ für einen der unter $R^2$ angegebenen Substituenten steht und mit diesem gleich oder von diesem verschieden ist, wobei nur einer der beiden Substituenten $R^2$ oder $R^3$ jeweils für Alkoly, Alkylthio, oder $NHR^I$ steht,

$R^4$

a) für Wasserstoff, $NO_2$, NO, CN, $SO_m$-$R^{IX}$(m = 0 oder 2), Halogen,

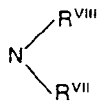 , 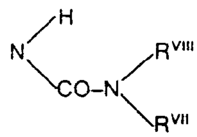 , 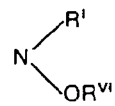

oder

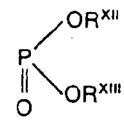

steht,

wobei $R^{I}$, $R^{VII}$, $R^{VIII}$, $R^{XII}$ und $R^{XIII}$ die unten angegebene Bedeutung haben, oder

b) für einen verzweigten oder unverzweigte, cyclischen, gesättigten oder ungesättigten aliphatischen Kohlenwasserstoffrest mit bis zu 10 C-Atomen steht, der gegebenenfalls substituiert ist durch Halogen, OH, CN, Alkoxy, Alkylthio mit jeweils 1 bis 4 kohlenstoffatomen, Phenyloxy, Naphthoxy, $COOR^{V}$ oder

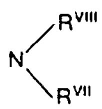 ,

wobei $R^{V}$, $R^{VII}$ und $R^{VIII}$ die unten angegebene Bedeutung haben, oder

c) für einen aromatischen Kohlenwasserstoffrest mit 6 bis 10 C-Atomen oder für einen 5- bis 7-gliedrigen gesättigten oder ungesättigten Heteroring mit 1 bis 3 gliechen oder verschiedenen Heterogliedern aus der Gruppe O, S, -N=, $NR^{I}$, steht, wobei dieser Heteroring entweder über ein Kohlenstoffatom oder ein Stickstoffatom mil dem Dihydropyridinring verknüpft ist, und wobei der aromatische Kohlenwasserstoffrest und die Heteroringe gegebenenfalls 1 bis 3 gleiche oder verschieden Substituenten aus der Gruppe Halogen, OH, CN, $CF_3$, $OCF_3$, $SCF_3$, $NO_2$, Alkyl, Alkoxy mit jeweils 1 bis 4 C-Atomen, Phenyl, Naphthyl und

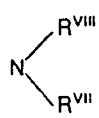

tragen,

oder

d) für den Rest

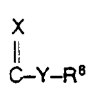

steht, wobei X Sauerstoff, Schwefel oder $NR^{I}$ bedeutet, und Y für eine einlache bindung, O, S oder $NR^{I}$ steht, und $R^{8}$ die für $R^{1}$ angegebene Bedeutung hat und mit dem Subtituenten $R^{1}$ gleich oder von diesem verschieden ist, oder

e) für den Rest

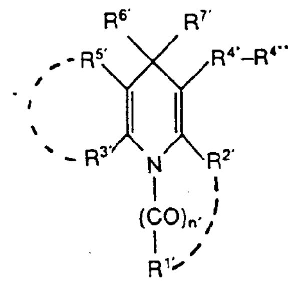

steht,

wobei n′, $R^{1'}$, $R^{2'}$, $R^{3'}$, $R^{5'}$, $R^{6'}$ und $R^{7'}$ die für n, $R^1$, $R^2$, $R^3$, $R^5$, $R^6$ und $R^7$ angegebene bedeutung haben und mit diesen gleich oder von diesen verschieden sind, und

$R^{4*}$ und $R^{4**}$ gleich oder verschieden sind und jeweils für einen um ein Wasserstoff verminderten Rest der für $R^4$ unter a) bis d) angegebenen Substituenten stehen, oder

$R^2$ und $R^4$ gemeinsam einen verzweigten, unverzweigten, gesättigten oder ungesättigten 5- bis 7-gliedrigen Ring bilden, der gegebenenfalls 1, 2 oder 3 gleiche oder verschiedene Ringglieder aus der Gruppe O, CO, CS, C=$NR^I$, =N-, $NR^I$ oder $SO_m$ (m = 0 oder 2) enthält und der gegebenenfalls substituiert ist durch Halogen, Hydroxy, Alkoxy mit 1 bis 4 C-Atomen, Phenyl, Naphthyl, Aralkyl mit 7 bis 14 C-Atomen,

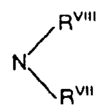

oder durch eine geradkettige oder verzweigte Alkylenkette mit 3 bis 8 Kohlenstoffatomen disubstituiert sit, wobei dieser gemeinsame Ring von $R^2$ und $R^4$ auch direkt mit dem gemeinsamen Ring von $R^1$ und $R^2$ anneliert sein kann, wobei die Reste $R^I$, $R^{II}$, $R^{III}$, $R^{VII}$, $R^{VII}$ die unten angegebene Bedeutung haben,

$R^5$ die für $R^4$ angegebene Bedeutung besitzt und mit $R^4$ gleich oder von diesem verschieden ist,

$R^6$ für Wasserstoff, Alkyl oder Halogenalkyl mit jeweils 1 bis 4 C-Atomen steht und

$R^7$

a) für einen gesättigten, ungesättigten cyclischen, geradkettigen oder verzweigten aliphatischen Kohlenwasserstoffrest mit bis zu 10 C-Atomen steht, der gegebenenfalls substituiert ist durch Halogen, Phenyl, Naphthyl oder Heteroaryl oder

b) einen Phenyl-, Naphthyl- oder Heteroarylrest steht, der gegebenenfalls 1 bis 3 gleiche oder verschiedene Substituenten aus der Gruppe $NO_2$, Halogen, CN, $N_3$, NO, $CF_3$, $COR^{IV}$, $COOR^V$, $OR^{VI}$,

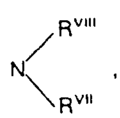 , $SO_mR^{XI}$ (m = 0, 1 oder 2), 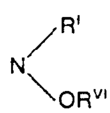 ,

Alkyl mit 1 bis 4 C-Atomen, Phenyl, Naphthyl, Alkenyl, Alkinyl, Alkenoxy, Alkinoxy mit jeweils bis zu 4 C-Atomen, Aralkyl mit 7 bis 14 C-Atomen, Acyl mit 1 bis 4 C-Atomen, Alkylen oder Dioxyalkylen mit jeweils bis zu 4 C-Atomen enthält, wobei die vorgenannten Alkyl- und Arylsubstituenten ihrerseits wieder durch Halogen, $COOR^V$ oder

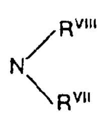 ,

substituiert sein können, und wobei in den vorgenannten Definitionen der Substituenten $R^1$ bis $R^7$ $R^I$ für Wasserstoff, Alkyl mit 1 bis 6 C-Atomen, Phenyl, Naphthyl, Aralkyl mit 7 bis 12 C-Atomen, Heteroaryl oder Acyl mit bis zu 7 C-Atomen steht, $R^{II}$ und $R^{III}$ gleich oder verschieden sind und jeweils für Alkyl mit 1 bis 6 C-Atomen, Phenyl, Naphthyl, Aralkyl mit 7 bis 12 C-Atomen oder Heteroaryl stehen,

$R^{VII}$, $R^V$ und $R^{VI}$ jeweils gleich oder verschieden sind und für Wasserstoff, Alkyl mit 1 bis 6 C-Atomen, Phenyl, Naphthyl, Aralkyl mit 7 bis 12 C-Atomen oder Heteroaryl stehen, wobei die Alkyl- und Arylreste gegebenenfalls durch Halogen, Nitro, Trifluormethyl, Alkoxy, Alkylthio und Alkyl mit jeweils 1 bis 4 C-Atomen substituiert sind, $R^{VII}$ und $R^{VIII}$ jeweils gleich oder verschieden sind und für Wasserstoff, Phenyl, Naphthyl oder Aralkyl mit 7 bis 12 C-Atomen stehen oder für Alkyl mit 1 bis 6 C-Atomen, welches gegebenenfalls durch O, S oder $NR^{I}$ unterbrochen ist, stehen oder gemeinsam mit dem Stickstoffatom einen 5- bis 7-gliedrigen Ring bilden, der 1 oder 2 gleiche oder verschiedene Heteroringglieder aus der Gruppe O, S oder $NR^{I}$ enthalten kann oder

wobei einer der Reste $R^{VII}$ und $R^{VIII}$ für eine aliphatische Acylgruppe mit bis zu 6 Kohlenstoffalomen steht, $R^{IX}$, $R^{X}$, $R^{XII}$ und $R^{XIII}$ jeweils gleich oder verschieden sind und für Alkyl mit 1 bis 6 C-Atomen, Phenyl, Naphthyl oder Aralkyl mit 7 bis 12 C-Atomen stehen,

wobei die unter $R^1$ bis $R^8$ und unter $R^{I}$ bis $R^{VIII}$ genannten Alkyl-, Aryl-, Aralkyl-, Heteroaryl- und Acylreste sowie der mit und $R^{VII}$ und $R^{VIII}$gebildete Heteroring ihrerseits gegebenenfalls substituiert sind durch OH, $CF_3$, $OCF_3$, CN, $NO_2$, Halogen, Alkyl mit 1 bis 4 C-Atomen, Alkoxy, Alkylthio mit jeweils 1 bis 4 C-Atomen, Phenyl oder benzyl,

und wobei als Heteroaryl folgende Substituenten genannt seien: Thienyl, Furyl, Pyryl, Pyridyl, Chinolyl, Isochinolyl, Pyrimidyl, Pyridazinyl, Chinazolyl, Chinoxalyl, benzothienyl, Pyrazolyl, Imidazolyl, Oxazolyl, Isoxazolyl, Thiazolyl, Triazolyl, Oxydiazolyl, Pyrazinyl, Oxazinyl, Thiazinyl, Indolizinyl, Indolyl, benzofuranyl, Indazolyl, benzothienyl, benzimidazolyl, benzoxazolyl, benzisoxazolyl, benzthiazolyl, benztriazolyl, benzoxadiazolyl, Cinnolinyl, Phthalazinyl, Naphthyridinyl oder benzotriazinyl, in Form von Isomeren, Isomerengemischen, Racematen und optischen Antipoden sowie ihrer pharmazeutisch unbedenklichen Salze.

9. Verwendung nach Ansprüchen 1 bis 7 von Verbindungen der allgemeinen Formel (I) gemäss Anspruch 1, in welcher

n für 0 oder 1 steht,

$R^1$

a) für Wasserstoff, einen geradkettigen, verzweigten cyclischen gesättigten oder ungesättigten aliphatischen Kohlenwasserstoffrest mit bis zu 10 C-Atomen steht, der gegebenenfalls durch ein oder zwei gleiche oder verschiedene Heterokettenglieder aus der Gruppe O, CO, S, = N- oder $NR^{I}$ enthält und wobei dieser Kohlenwasserstolfrest gegebenenfalls substituiert ist durch F, Cl, Br, $NO_2$, CN, OH, Phenyl oder Pyridyl oder

b) für einen Phenyl-, Naphthyl- oder Pyridylrest steht, wobei diese Reste gegebenenlalls 1 oder 2 gleiche oder verschiedene Substituenten aus der Gruppe Phenyl, Alkyl, Alkenyl, Alkoxy, Alkenoxy mit jeweils bis zu 4 C-Atomen, benzyl, Acetyl, Alkylen, Dioxyalkylen mit 2 bis 4 C-Atomen, Fluor, Chlor, Brom, $CF_3$, $OCF_3$, $SCF_3$, $NO_2$, CN, $COOR^{V}$, $OR^{VI}$ $NR^{I}$ oder $NR^{VII}R^{VIII}$ tragen, wobei die Alkyl-, Alkoxy- und Arylreste der o.g. Substituenten ihrerseits wieder Halogen substituiert sein können oder

c) für den Rest $NR^{VII}R^{VIII}$ steht,

und wobei die unter a), b) und c) genannten Reste $R^{I}$ bis $R^{VIII}$ die unten angegebene Beteutung haben,

$R^2$

a) für einen der unter $R^1$ angegebenen Substituenten steht aber nicht mit diesen identisch sein muss oder

b) für die Reste $NHR^{I}$ oder

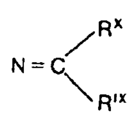

steht, wobei $R^{I}$, $R^{IX}$ und $R^{X}$ die unten angegebene bedeutung besitzen

oder die Substituenten

$R^1$ und $R^2$ gemeinsam einen 5- bis 7-gliedrigen gesättigten oder ungesättigten Ring bilden, der gegebenenfalls 1 oder 2 gleiche oder verschiedene Ringglieder aus der Gruppe O, S, $NR^{I}$ oder CO enthält und der gegebenenfalls 1 oder 2 gleiche oder verschiedene Substituenten aus der Gruppe Halogen, Hydroxy, Alkyl, Alkoxy mit je 1 bis 4 C-Atomen, Phenyl oder benzyl enthält,

$R^3$ für einen der unter $R^2$ angegebenen Substituenten steht und mit diesem gleich oder von diesem verschieden ist, wobei nur einer der beiden Substituenten $R^2$ oder $R^3$ jeweils für Alkoxy, Alkylthio oder $NHR^{I}$ steht,

$R^4$

a) für Wasserstoff, $NO_2$, CN, $SR^{XI}$, $SO_2R^{XI}$,

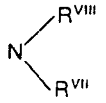, 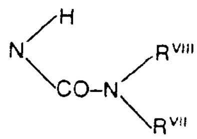 oder 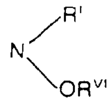

steht, wobei $R^I$, $R^{VI}$, $R^{VII}$, $R^{VIII}$, und $R^{IX}$ die unten angegebene bedeutung haben,

b) für einen verzweigten oder unverzweigten Alkyl- oder Cycloalkylrest mit bis zu 8 C-Atomen steht, der gegebenenfalls substituiert ist durch Halogen, Hydroxy, Cyano, Alkoxy mit 1 bis 4 C-Atomen, Phenyloxy, $COOR^V$ oder

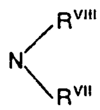

wobei $R^V$, $R^{VII}$, und $R^{VIII}$ die unten angegebene bedeutung haben oder

c) für einen aromatischen Kohlenwasserstoffrest mit 6 bis 10 C-Atomen oder für einen 5- bis 7-gliedrigen gesättigten oder ungesättigten Heteroring mit 1 bis 3 gleichen oder verschiedenen Heterogliedern aus der Gruppe O, S, =N-, $NR^I$, steht, wobei dieser Heteroring entweder über ein Kohlenstoffatom oder ein Stickstoffatom mit dem Dihydropyridinring verknüpft ist und wobei der aromatische Kohlenwasserstoffrest und die Heteroringe gegebenenfalls 1 oder 2 gleiche oder verschiedene Substituenten aus der Gruppe Halogen, Hydroxy, Cyano, $CF_3$, $NO_2$, Phenyl, Alkyl und Alkoxy mit je 1 bis 4 C-Atomen tragen

oder

d) für den Rest

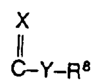

steht, wobei X Sauerstoff oder $NR^I$ und Y für eine einfache bindung, Sauerstoff oder $NR^I$ steht und $R^8$ für für $R^1$ angegebene Bedeulung hat und mit dem Substituenten gleich oder von diesem verschieden ist,

oder

e) für den Rest

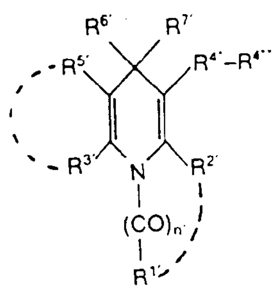

steht, wobei n′, $R^{1'}$, $R^{2'}$, $R^{3'}$, $R^{5'}$, $R^{6'}$ und $R^{7'}$ die für n, $R^1$, $R^2$, $R^3$, $R^5$, $R^6$, und $R^7$ angegebene Bedeutung haben und mit diesen gleich oder von diesen verschieden sind, und

$R^{4*}$ und $R^{4**}$ gleich oder verschieden sind und jeweils für einen um ein Wasserstoff verminderten Rest der für $R^4$ unter a) bis d) angegebenen Substituenten stehen, oder

$R^2$ und $R^4$ gemeinsam einen verzweigten unverzweigten, gesättigten oder ungesättigten 5- bis 7-gliedrigen Ring bilden, der gegebenenfalls 1 oder 2 gleiche oder verschiedene Ringglieder aus der Gruppe O, CO, CS, $C{=}NR^I$, =N- oder $NR^I$ enthält und der gegebenenfalls substituiert ist durch Halogen oder Hydroxy,

wobei $R^1$ die unten angegebene bedeutung hat,

$R^5$ die für $R^4$ angegebene bedeutung besitzt und mit $R^4$ gleich oder von diesen verschieden ist oder

$R^5$ und $R^3$ gemeinsam einen Ring bilden, wie er für $R^2$ und $R^4$ definiert ist und mit diesem gleich oder von diesem verschieden ist,

$R^6$ für Wasserstoff oder Alkyl mit 1 bis 4 C-Atomen steht, welches gegebenenfalls durch Fluor, Chlor oder brom substituiert ist und

$R^7$

a) für einen gesättigten, ungesättigten cyclischen, geradkettigen oder verzweigten aliphatischen Kohlenwasserstoffrest mit bis zu 8 C-Atomen steht, der gegebenenfalls substituiert ist durch Halogen, oder

b) für einen Phenyl- oder Heteroarylrest steht, der gegebenenfalls 1 bis 3 gleiche oder verschiedene Substituenten aus der Gruppe $NO_2$, CN, $N_3$, $CF_3$, Halogen, $COR^{IV}$, $COR^{V}$, $OR^{VI}$, $SR^{XI}$,

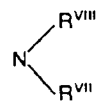

Phenyl, Alkyl mit 1 bis 4 C-Atomen, Benzyl oder Acyl mit 1 bis 4 C-Atomen enthält, wobei in den vorgenannten Definitionen der Substituenten $R^1$ bis $R^8$

$R^I$ für Wasserstoff, Alkyl mit 1 bis 6 C-Atomen, phenyl, Naphthyl, benzyl, Phenethyl, Heteroaryl oder Acyl mit bis zu 4 C-Atomen steht,

$R^{II}$ und $R^{III}$ gleich oder verschieden sind und jeweils für Alkyl mit 1 bis 6 C-Atomen, phenyl, Naphthyl, benzyl oder Heteroaryl stehen,

$R^{IV}$, $R^V$ und $R^{VI}$ jeweils gleich oder verschieden sind und für Wasserstoff, Alkyl mit 1 bis 6 C-Atomen, Phenyl, Naphthyl, Benzyl oder Heteroaryl stehen, wobei die Alkyl-, Phenyl- und benzylreste gegebenenfalls substituiert sind durch Fluor, Chlor, Nitro, $CF_3$, Methyl, Methoxy und Methylthio, $R^{VII}$ und $R^{VIII}$ jeweils gleich oder verschieden sind und für Wasserstoff, Phenyl oder benzyl stehen oder für Alkyl mit 1 bis 6 Kohlenstoffatomen stehen, welches gegebenenfalls durch O oder $NR^I$ unterbrochen ist, oder

gemeinsam mit dem Stickstoffatom einen 5- bis 7-gliedrigen Ring bilden, der 1 oder 2 gleiche verschiedene Heteroringglieder aus der Gruppe O, S oder $NR^I$ enthalten kann,

oder

wobei einer der Reste $R^{VII}$ oder $R^{VIII}$ für eine aliphatische Acylgruppe mit bis zu 6 Kohlenstoffatomen steht und $R^{IX}$, $R^X$, $R^{XI}$, $R^{XII}$ und $R^{XIII}$ jeweils gleich oder verschieden sind und für Alkyl mit 1 bis 6 C-Atomen, Phenyl oder benzyl stehen, wobei als Heteroaryl folgende Substituenten genannt seien:

Thienyl, Furyl, Pyryl, Pyridyl, Chinolyl, Isochinolyl, Pyrimidyl, Pyridazinyl, Chinazolyl, Chinoxalyl, benzothienyl, Pyrazolyl, Imidazolyl, Oxazolyl, Isoxazolyl, Thiazolyl, Triazolyl, Oxydiazolyl, Pyrazinyl, Oxazinyl, Thiazinyl, Indolyl, benzofuranyl, Indazolyl, benzothienyl, benzimidazolyl, benzoxazolyl, benzisoxazolyl, benzthiazolyl, benztriazolyl oder benzoxadiazolyl.

10. Verwendung nach Ansprüchen 1 bis 7 von Verbindungen der allgemeinen Formel (I) gemäss Anspruch 1, in welcher

n für 0 steht,

$R^1$

a) für Wasserstoff, einen aliphatischen Kohlenwasserstoffrest mit bis zu 6 C-Atomen, der gegebenenfalls ein Heterokettenglied aus der Gruppe O, CO, = N- oder $NR^I$ enthält und der gegebenenfalls substituiert ist durch Halogen, Nitro, Hydroxy oder Pheny oder

b) für einen Phenyl- oder Pyridylrest steht, die gegebenenfalls substituiert sind durch Halogen, $NO_2$, $CF_3$, $OCF_3$, CN, $COOR^V$ oder $NR^{VII}R^{VIII}$,

$R^2$ für einen der unter $R^1$ angegebenen Substituenten steht, aber nicht mit diesem identisch sein muss oder für einen der Reste $NHR^I$ oder $N = CR^XR^{XI}$ steht,

$R^3$ für einen der unter $R^2$ angegebenen Substituenten steht und mit diesem gleich oder von diesem verschieden ist,

$R^4$

a) für Wasserstoff, $NO_2$, $NR^{VII}R^{VIII}$,

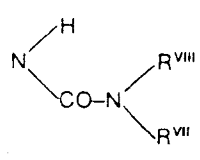

oder Halogen steht, oder

b) für einen Alkylrest mit 1 bis 4 C-Atomen steht, der gegebenenfalls substituiert ist durch Halogen, OH,

CN, Alkoxy mit 1 bis 4 C-Atomen, $COOR^{V}$ oder $NR^{VII}R^{VIII}$ oder

c) für Phenyl, Pyridyl oder Thienyl steht, die gegebenenfalls substituiert sind durch Halogen, OH, CN, Alkyl oder Alkoxy mit jeweils 1 bis 4 C-Atomen oder durch $NR^{VII}R^{VIII}$oder

d) für den Rest

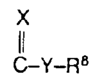

steht,

wobei X Sauerstoff bedeutet und Y für eine einfache Bindung, Sauerstoff oder $NR^{I}$ steht und $R^8$ die für $R^1$ angegebene bedeutung hat und mit dem Substituenten $R^1$ gleich oder von diesem verschieden ist oder

e) für den Rest

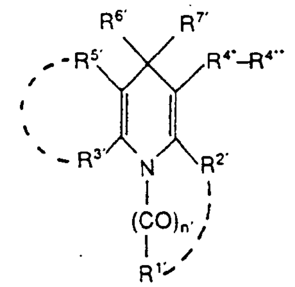

steht,

wobei n', $R^{1'}$, $R^{2'}$, $R^{3'}$, $R^{5'}$, $R^{6'}$ und $R^{7'}$ die für n, $R^1$, $R^2$, $R^3$, $R^5$, $R^6$ und $R^7$ angegebene Bedeutung haben und mit diesen gleich oder von diesen verschieden sind, und

$R^{4*}$ und $R^{4**}$ gleich oder verschieden sind und jeweils für einen um ein Wasserstoff verminderten Rest der für $R^4$ unter a) bis d) angegebenen Substituenten stehen, oder

$R^2$ und $R^4$ gemeinsam einen 5- bis 7-gliedrigen Ring bilden, der gegebenenfalls 1 oder 2 verschiedene Ringglieder aus der Gruppe O, CO, CS oder $C{=}NR^{I}$ enthält und der gegebenenfalls substituiert ist durch Halogen,

$R^5$ die für $R^4$ angegebene Bedeutung besitzt und mit $R^4$ gleich oder von diesem verschieden ist,

$R^6$ für Wasserstoff oder Alkyl mit 1 bis 4 C-Atomen steht und $R^7$ die oben angegebene Bedeutung besitzt, wobei in den vorgennanten Definitionen die mit römischen Ziffern gekennzeichneten Substituenten $R^{I}$ bis $R^{VIII}$ die in Anspruch 4 angegebene Bedeutung besitzen.

11. Verwendung nach Ansprüchen 1 bis 7 von Verbindungen der allgemeinen Formel (I) gemäss Anspruch 1, in welcher wenigstens einer der Substituenten $R^4$ un $R^5$ für $NO_2$ stehen und/oder in welcher $R^2$ und $R^4$ gemeinsam oder $R^3$ und $R^5$ gemeinsam einen Lactonring bilden.

12. Verbindungen der allgememen Formel (I)

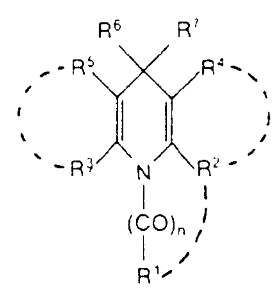 (I)

in welcher mindestens einer der Substituenten $R^4$ und $R^5$ für die Gruppe

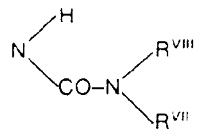

steht, wobei $R^{VIII}$ und $R^{VII}$ und die Substituenten $R^1$, $R^2$, $R^3$, $R^6$, $R^7$ und n sowie der verbleibende Rest von $R^4$ und $R^5$ die in Ansprüchen 1 und 8 bis 11 angegebene Bedeutung haben.

13. Verbindungen der allgemeinen Formel (I)

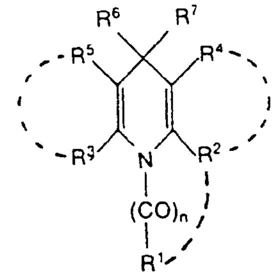

(I)

in welcher mindestens einer der Substituten $R^4$ und $R^5$ für Methyl steht, wobei $R^1$, $R^2$, $R^3$, $R^6$, $R^7$ und n sowie der verbleibende Rest von $R^4$ und $R^5$ die in Anprüchen 1 und 8 bis 11 angegebene Bedeutung haben.

14. Verbindugen der allgemeinen Formel (I)

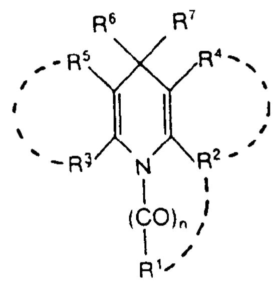

(I)

in welcher einer der Reste $R^4$ oder $R^5$ Wasserstoff bedeutet, $R^1$ für Wasserstoff und n für 0 steht und $R^2$, $R^3$, $R^6$ und $R^7$ sowie der andere Rest von $R^4$ und $R^5$ die in Ansprüchen 1 und 8 bis 11 angegebene Bedeutung haben.

15. Verbindungen der allgemeinen Formel (I)

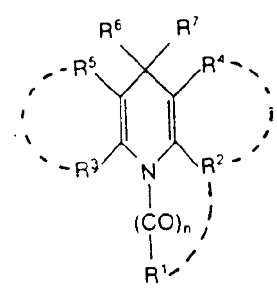

(I)

in welcher einer der Reste $R^4$ oder $R^5$ Halogen bedeutet und $R^1$, $R^2$, $R^3$, $R^6$ und $R^7$ und n sowie der andere Rest von $R^4$ und $R^5$ die in Anspruch 1 und 8 bis 11 angegebene Bedeutung haben.

16. Verbindugen gemäss Anspruch 15, in denen einer der Reste $R^4$ oder $R^5$ für Fluor oder Chlor steht.

17. Verfahren zur Herstellung von Verbindungen gemäss Ansprüchen 12 bis 16, dadurch gekennzeichnet, dass man

a) Carbonylverbindungen der allgemeinen Formel (II)

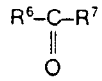 (II)

mit Ketonen der Formeln (III) und (IV)

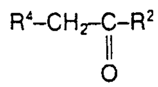 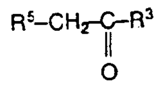

(III) (IV)

und primären Aminen der Formel (V)

$$H_2N\text{-}(CO)_n\text{-}R^1$$

in welcher n = 0 ist,
gegebenenfalls in Gegenwart von inerten Lösungsmitteln umsetzt,
oder
b) Carbonylverbindungen der Formel (II)

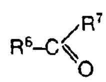 (II)

mit Enaminen der Formel (VI)

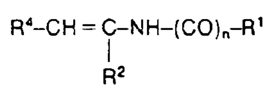 (VI)

und Ketonen der Formel (IV)

$$R^3\text{-}CO\text{-}CH_2\text{-}R^5$$

gegebenenfalls in Gegenwart von inerten Lösungsmitteln umsetzt oder
c) Enamine der Formel (VI)

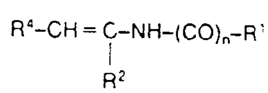 (VI)

mit Ylidenverbindungen der Formel (VIII)

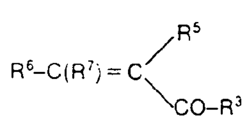

gegebenenfalls in Gegenwart von inerten Lösungsmitteln, umsetzt, oder
d) eine oder mehrere funktionelle Gruppen von Dihydropyridinen nach üblichen Methoden der Hydrolyse, Veresterung, Umesterung, Lactonisierung, Kondensation, Acylierung, Reduktion oder Cyclisierung umwandelt.

18. Arzneimittel enthaltend mindestens eine Verbindung gemäss Ansprüchen 12 bis 16.

19. Verfahren zur Herstellung von Arzneimitteln, dadurch gekennzeichnet, dass man mindestens eine Verbindung gemäss Ansprüchen 12 bis 16, gegebenenfalls unter Verwendung inerter Hilfs- undifferent Trägerstoffein eine geeignete Applikationsform übeführt.

## Claims

1. Use of 1,4-dihydropyridines of the general formula (I)

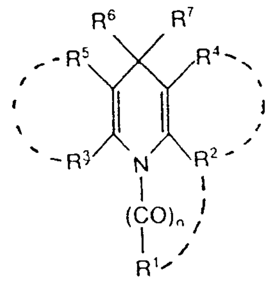

(I)

in which
n represents 0, 1 or 2,

$R^1$

a) represents hydrogen, a straight-chain, branched, cyclic, saturated or unsaturated aliphatic hydrocarbon radical which optionally contains 1 to 3 identical or different hetero chain members from the group comprising O, CO, $SO_m$ (m = 0,1 or 2), = N-, $NR^I$ and $SiR^{II}R^{III}$, this hydrocarbon radical optionally being substituted by halogen, $NO_2$, CN, $N_3$, hydroxyl, aryl or heteroaryl or

b) represents an aryl or heteroaryl radical, these radicals optionally carrying 1 to 5 identical or different substituents from the group comprising aryl, alkyl, alkenyl, alkinyl, alkenoxy, alkinoxy, aralkyl, acyl, alkylene, dioxyalkylene, halogen, $CF_3$, $OCF_3$, $SCF_3$, $NO_2$, NO, CN, $N_3$, $COR^{IV}$, $COOR^V$, $OR^{VI}$, $NR^I$, or $NR^{VII}R^{VIII}$, it being possible for the alkyl, alkoxy and aryl radicals of the abovementioned substituents in their turn also to be substituted by halogen, $COOR^V$ or $NR^{VII}$ $R^{VIII}$ or

c) represents the radical $NR^{VII}R^I$, the radicals $R^I$, $R^{II}$, $R^{III}$, $R^{IV}$, $R^V$, $R^{VI}$, $R^{VII}$ and $R^{VIII}$ mentioned under a), b), and c) having the meaning given below,

$R^2$

a) represents one of the substituents mentioned under $R^1$ but does not have to be identical thereto, or

b) represents the radical $NHR^I$ or

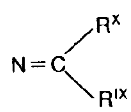

$R^I$, $R^{IX}$ and $R^X$ having the meaning given below, or the substituents $R^1$ and $R^2$ together form a 5- to 8-membered, saturated or unsaturated ring which optionally contains 1, 2 or 3 identical or different ring members from the group comprising O, S, $NR^I$ and CO and which optionally contains 1 to 3 identical or different substituents from the group comprising halogen, hydroxyl, alkyl, alkoxy, aryl or aralkyl,

$R^3$ represents one of the substituents mentioned under $R^2$ and is identical thereto or differs therefrom, only one of the two substituents
$R^2$ or $R^3$ in each case representing alkoxy, alkylthio or $NHR^I$,

$R^4$

a) represents hydrogen, $NO_2$, NO, CN, $SO_mR^{XI}$ (m = 0, 1 or 2), halogen

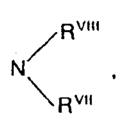
,
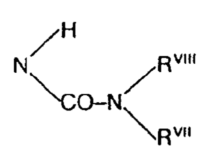
,
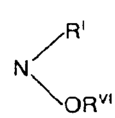

or
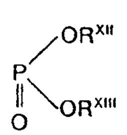

$R^I R^{VII}$, $R^{VIII}$, $R^{XI}$ and $R^{XIII}$ having the meaning given below, or

b) represents a branched or unbranched, cyclic saturated or unsaturated aliphatic hydrocarbon radical which is optionally substituted by halogen, OH, CN, alkoxy, alkylthio, aryloxy, $COOR^V$ or

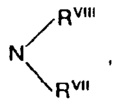

$R^V$, $R^{VII}$ and $R^{VIII}$ having the meaning given below, or

c) represents an aromatic hydrocarbon radical or a 5- to 7-membered saturated or unsaturated hetero ring with 1 to 4 identical or different hetero members from the group comprising O, S, -N = and $NR^I$, this hetero ring being linked to the dihydropyridine ring either via a carbon atom or a nitrogen atom, the aromatic hydrocarbon radical and the hetero rings optionally carrying 1 to 3 identical or different substituents from the group comprising halogen, OH, CN, $CF_3$, $OCF_3$, $SCF_3$, $NO_2$, alkyl, alkoxy, aryl and

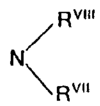

or

d) represents the radical

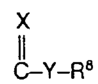

wherein X denotes oxygen, sulphur or $NR^I$, and Y represents a single bon, O, S or $NR^I$, and $R^8$ has the meaning given for $R^1$ and is identical to the substituent $R^1$ or differs therefrom, or

e) represents the radical

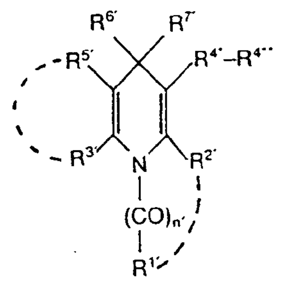

wherein

n', $R^{1'}$, $R^{2'}$, $R^{3'}$, $R^{5'}$, $R^{6'}$, and $R^{7'}$ have the meaning given for n, $R^1$, $R^2$, $R^3$, $R^5$, $R^6$ and $R^7$ and are identical thereto or differ therefrom, and

$R^{4*}$ and $R^{4**}$ are identical or different and each represent a radical, minus one hydrogen, of the substituents mentioned for $R^4$ under a) to d), or $R^2$ and $R^4$ together form a branched, unbranched, saturated or unsaturated 5- to 8-membered ring which optionally contains 1, 2 or 3 identical or different ring members from the group comprising O, CO, CS, $C{=}NR^I$, =N-, $NR^I$, $SO_m$ (m = 0, 1 or 2) and $SiR^{II}R^{III}$, and which is optionally substituted by halogen, hydroxyl, alkoxy, aryl, aralkyl

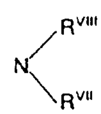

or disubstituted by a straigh-chain or branched alkylene chanin with 3 to 8 carbon atoms, it being possible for this common ring of $R^2$ and $R^4$ also to be directly fused to the common ring of $R^1$ and $R^2$, the radicals $R^I$, $R^{II}$, $R^{III}$, $R^{VII}$ and $R^{VIII}$ having the meaning given below,

$R^5$ has the meaning given for $R^4$ and is identical to $R^4$ or different therefrom, or

$R^5$ and $R^3$ together form a ring which is identical to the ring formed by $R^2$ and $R^4$ or differs therefrom, or

$R^6$ represents hydrogen, alkyl or halogenoalkyl, and

$R^7$

a) represents a saturated, unsaturated, cyclic, straight-chain or branched aliphatic hydrocarbon radical which is optionally substituted by halogen, aryl or heteroaryl, or

b) represents an aryl or heteroaryl radical which optionally contains 1 to 5 identical or different substituents from the group comprising $NO_2$, CN, $N_3$, NO, $CF_3$, halogen, $COR^{IV}$, $COR^V$, $OR^{VI}$,

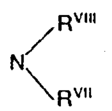 , $SO_mR^{XI}$ (m = 0, 1 or 2), 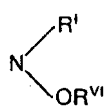 ,

alkyl, aryl, alkenyl, alkinyl, alkenoxy, alkinoxy, aralkyl, acyl, alkylene and dioxyalkylene, it being possible for the abovementioned alkyl and aryl substituents in their turn also to be substituted by halogen, $COOR^V$ or

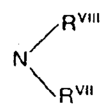

and in the abovementioned definitions of the substituents $R^1$ to $R^8$

$R^I$ represents hydrogen, alkyl, aryl, aralkyl, heteroaryl or acyl,

$R^{II}$ and $R^{III}$ are identical or different and each represent alkyl, aryl, aralkyl or heteroaryl, $R^{IV}$, $R^V$ and $R^{VI}$ are in each case identical or different and represent hydrogen, alkyl, aryl, aralkyl or heteroaryl, the alkyl and aryl radicals optionally being substituted by halogen, nitro, trifluoromethyl, alkoxy, alkylthio or alkyl,

$R^{VII}$ and $R^{VIII}$ are in each case identical or different and represent hydrogen, aryl or aralkyl,different or represent alkyl which is optionally interrupted by O, S or $NR^I$, or, together with the nitrogen atom, form a 5- to 7-membered ring which can contain 1 or 2 identical or different hetero ring members from the group comprising O, S and $NR^I$, or

one of the radicals $R^{VII}$ or $R^{VIII}$ represents an aliphatic acyl group with up to 6 carbon atoms, $R^{IX}$, $R^X$, $R^{XI}$, $R^{XII}$ and $R^{XIII}$ are in each case identical or different and represent alkyl, aryl or aralkyl,

the alkyl, aryl, aralkyl, heteroaryl and acyl radicals mentioned under $R^1$ to $R^8$ and under $R^I$ to $R^{XIII}$, and the hetero ring formed with $R^{VII}$ and $R^{VIII}$, in their turn optionally being substituted by OH, $CF_3$, $OCF_3$, CN, $NO_2$, halogen, lower thioalkyl, lower alkyl, lower alkoxy, aryl or aralkyl,

and the following substituents being mentioned as heteroaryl:

thienyl, fuyl, pyryl, pyridyl, quinolyl, isoquinolyl, pyrimidyl, pyradazinyl, quinazolyl, quinoxalyl, benzothienyl, pyrazolyl, imidazolyl, oxazolyl, isoxazolyl, thiazolyl, triazolyl, oxydiazolyl, pyrazinyl, oxazinyl, thiazinyl, indolizinyl, indolyl, benzofuranyl, indazolyl, benzothienyl, benzimidazolyl, benzoxazolyl, benzisoxazolyl, benzthiazolyl, benztriazolyl, benzoxadiazolyl, cinnolinyl, phthalazinyl, naphthyridinyl, or benzotriazinyl,

in the form of isomers, isomer mixtures, racemates and optical antipodes, and of their pharmaceutically acceptable salts, which, from a concentration of $10^{-5}$ g/ml upwards, cause an at least 25% augmentation of the contraction of the isolated left atrium of the guinea pig heart, for the preparation of medicaments which increase the influx of $Ca^{++}$ into the cell.

2. Use according to Claim 1 of compounds of the general formula (I) for the preparation of medicaments with a positive inotropic action.

3. Use according to Claim 1 of compounds of the general formula (I) for the preparation of medicaments with a cardiotonic action.

4. Use according to Claim 1 of compounds of the general formula (I) for the preparation of medicaments with an antihypotonic action.

5. Use according to Claim 1 of compounds of the general formula (I) for the preparation of medicaments for reducing blood-sugar.

6. Use according to Claim 1 of compounds of the general formula (I) for the preparation of medicaments

for decreasing the swelling of mucous membranes.

7. Use according to Claim 1 of compounds of the general formula (I) for the preparation of medicaments for influencing the salt and liquid balance.

8. Use according to Claims 1 to 7 of compounds of the general formual (I) according to Claim 1, in which n represents 0,1 or 2,

$R^1$

a) represents hydrogen, a straight-chain, branched cyclic saturated or unsaturated aliphatic hydrocarbon radical with up to 10 C atoms which optionally contains 1 or 2 identical or different hetero chain members from the group comprising O, CO, S, $SO_2$, =N- and $NR^I$, this hydrocarbon radical optionally being substituted by halogen, $NO_2$, CN, $N_3$, hydroxyl, with 1 to 4 C atoms, phenyl, naphthyl or heteroaryl or

b) represents a phenyl, naphthyl or heteroaryl radical, these radicals optionally carrying 1 to 3 identical or different substituents from the group comprising phenyl, alkyl, alkenyl, alkinyl, alkenoxy, alkinoxy with in each case up to 4 C atoms, aralkyl with 7 to 14 C atoms, acyl with up to 6 C atoms, alkylene, dioxyalkylene with up to 4 carbon atoms in the alkylene chain, halogen, $CF_3$, $OCF_3$, $SCF_3$, $NO_2$, CN, $N_3$, $COR^{IV}$, $COOR^V$, $OR^{VI}$, $NR^I$ or $NR^{VII}R^{VIII}$, it being possible for the alkyl, alkoxy and aryl radicals of the abovementioned substituents in their turn also to be substituted by halogen, $COOR^V$ or $NR^{VII}R^{VIII}$ or

c) represents the radical $NR^{VII}R^I$, the radicals $R^I$, $R^{II}$, $R^{III}$, $R^{IV}$, $R^V$, $R^{VI}$, $R^{VII}$ and $R^{VIII}$ mentioned under a), b) and c) having the meaning given below,

$R^2$ an

a) represents one of the substituents mentioned under $R^1$ but does not have to be identical thereto or

b) represents the radicals $NHR^I$ or

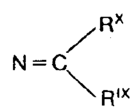

$R^I$, $R^{IX}$ and $R^X$ having the meaning given below,

or the substitutents

$R^1$ and $R^2$ together form a 5- to 7-membered saturated or unsaturated ring which optionally contains one or two identical or different ring members from the group comprising O, S, $NR^I$ and CO and which optionally contains one to three identical or different substituents from the group comprising halogen, hydroxyl, alkyl, alkoxy with in each case 1 to 4 C atoms, phenyl, naphthyl or aralkyl with 7 to 14 C atoms,

$R^3$ represents one of the substituents mentioned under $R^2$ and is identical thereto or differs therefrom, only one of the two substituents $R^2$ or $R^3$ in each case representing alkoxy, alkylthio or $NHR^I$,

$R^4$

a) represents hydrogen, $NO_2$, NO, CN, $SO_m$-$R^{IX}$ (m = 0 or 2), halogen,

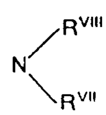 , 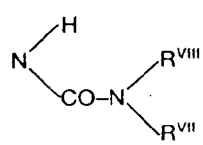 , 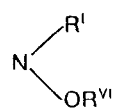

or 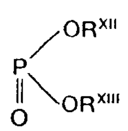

$R^I$, $R^{VII}$, $R^{VIII}$, $R^{XII}$ and $R^{XIII}$ having the meaning given below, or

b) represents a branched or unbranched, cyclic, saturated or unsaturated aliphatic hydrocarbon radical with up of 10 C atoms, which is optionally substituted by halogen, OH, CN, alkoxy, alkylthio with in each case 1 to 4 carbon atoms, phenyloxy, napththoxy, $COOR^V$ or

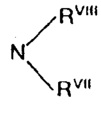

$R^V$, $R^{VII}$ and $R^{VIII}$ having the meaning given below, or

c) represents an aromatic hydrocarbon radical with 6 to 10 C atoms or a 5- to 7-membered saturated or unsaturated hetero ring with 1 to 3 identical or different hetero members from the group comprising O, S, -N = and $NR^I$, this hetero ring being linked to the dihydropyridine ring either via a carbon atom or a nitrogen atom and the aromatic hydrocarbon radical and the hetero rings optionally carrying 1 to 3 identical or different substituents from the group comprising halogen, OH, CN, $CF_3$, $OCF_3$, $SCF_3$, $NO_2$, alkyl, alkoxy with in each case 1 to 4 C atoms, phenyl, naphthyl and

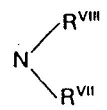

or

d) represents the radical

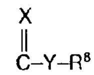

wherein X denotes oxygen, sulphur or $NR^I$, and Y represents a single bond, O, S or $NR^I$, and $R^8$ has the meaning given for $R^1$ and is identical to the substituent $R^1$ or differs therefrom, or

e) represents the radical

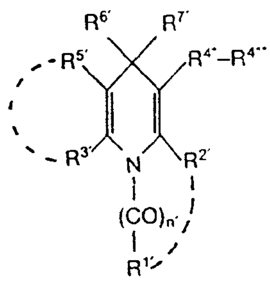

wherein

n′, $R^{1'}$, $R^{2'}$, $R^{3'}$, $R^{5'}$, $R^{6'}$ and $R^{7'}$ have the meaning given for n, $R^1$, $R^2$, $R^3$, $R^5$, $R^6$ and $R^7$ and are identical thereto or differ therefrom, and

$R^{4*}$ and $R^{4**}$ are identical or different and each represent a radical, minus one hydrogen, of the substituents mentioned for $R^4$ under a) to d), or

$R^2$ and $R^4$ together form a branched, unbranched, saturated or unsaturated 5- to 7-membered ring which optionally contains 1, 2 or 3 identical or different ring members from the group comprising O, CO, CS, C = $NR^I$, = N-, $NR^I$ and $SO_m$ (m = 0 or 2) and which is optionally substituted by halogen, hydroxyl, alkoxy with 1 to 4 C atoms, phenyl, naphthyl, aralkyl with 7 to 14 C atoms, or

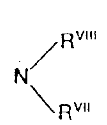

or disubstituted by a straight-chain or branched alkylene chain with 3 to 6 carbon atoms, it being possible for this common ring of $R^2$ and $R^4$ also to be directly fused to the common ring of $R^1$ and $R^2$, the radicals $R^I$, $R^{II}$, $R^{III}$, $R^{VII}$ and $R^{VIII}$ having the meaning given below,

$R^5$ has the meaning given for $R^4$ and is identical to $R^4$ or differs therefrom,

$R^6$ represents hydrogen, alkyl or halogenoalkyl with in each case 1 to 4 C atoms and

$R^7$

a) represents a saturated, unsaturated cyclic, straight-chain or branched aliphatic hydrocarbon radical with up to 10 C atoms, which is optionally substituted by halogen, phenyl, naphthyl or heteroaryl or

b) represents a phenyl, naphthyl or heteroaryl radical which optionally contains 1 to 3 identical or different substituents from the group comprising $NO_2$, halogen, CN, $N_3$, NO, $CF_3$, $COR^{IV}$, $COOR^{V}$ $OR^{VI}$,

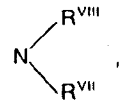 , $SO_mR^{XI}$ (m = 0, 1 or 2), 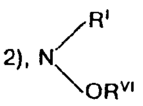 ,

alkyl with 1 to 4 C atoms, phenyl, naphthyl, alkenyl, alkinyl, alkenoxy, alkinoxy with in each case up to 4 C atoms, aralkyl with 7 to 14 C atoms, acyl with 1 to 4 C atoms, alkylene and dioxyalkylene with in each case up to 4 C atoms, it being possible for the abovementioned alkyl and aryl substituents in their turn also to be substituted by halogen, $COOR^{V}$ or

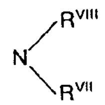

and in the abovementioned definitions of the substituents $R^1$ to $R^7$
$R^I$ represents hydrogen, alkyl with 1 to 6 C atoms, phenyl, naphthyl, aralkyl with 7 to 12 C atoms, heteroaryl or acyl with up to 7 C atoms, $R^{II}$ and $R^{III}$ are identical or different and each represent alkyl with 1 to 6 C atoms, phenyl, naphthyl, aralkyl with 7 to 12 C atoms or heteroaryl,
$R^{VII}$, $R^{V}$ and $R^{VI}$ are in each case identical or different and represent hydrogen, alkyl with 1 to 6 C atoms, phenyl, naphthyl, aralkyl with 7 to 12 C atoms or heteroaryl, the alkyl and aryl radicals optionally being substitute by halogen, nitro, trifluoromethyl, alkoxy, alkylthio and alkyl with each case 1 to 4 C atoms,
$R^{VII}$ and $R^{VIII}$ are in each case identical or different and represent hydrogen, phenyl, naphthyl or aralkyl with 7 to 12 C atoms or represent alkyl with 1 to 6 C atoms which is optionally interrupted by O, S or $NR^I$, or, together with the nitrogen atom, from a 5- to 7-membered ring which can contain 1 or 2 identical or different hetero ring members from the group comprising O, S and $NR^I$, or
one of the radicals $R^{VII}$ and $R^{VIII}$ represents an aliphatic acyl group with up to 6 carbon atoms, $R^{IX}$, $R^{X}$, $R^{XII}$ and $R^{XIII}$ are in each case identical or different and represent alkyl with 1 to 6 C atoms, phenyl, naphthyl or aralkyl with 7 to 12 C atoms, the alkyl, aryl, aralkyl, heteroaryl and acyl radicals mentioned under $R^1$ to $R^8$ and under $R^I$ to $R^{VIII}$ and the hetero ring formed with $R^6$ and $R^7$ in their turn optionally being substituted by OH, $CF_3$, $OCF_3$, CN, $NO_2$, halogen, alkyl with 1 to 4 C atoms, alkoxy, alkylthio with in each case 1 to 4 C atoms, phenyl or benzyl, and the following substituents being mentioned as heteroaryl:
thienyl, furyl, pyryl, pyridyl, quinolyl, isoquinolyl, pyrimidyl, pyridazinyl, quinazolyl, quinoxalyl, benzothienyl, pyrazolyl, imidazolyl, oxazolyl, isoxazolyl, thiazolyl, triazolyl, oxydiazolyl, pyrazinyl, oxazinyl, thiazinyl, indolizinyl, indolyl, benzofuranyl, indazolyl, benzothienyl, benzimidazolyl, benzoxazolyl, benzisoxazolyl, benzthiazolyl, benztriazolyl, benzoxadiazolyl, cinnolinyl, phthalazinyl, naphthyridinyl or benzotriazinyl,
in the form of isomers, isomer mixtures, racemates and optical antipodes and of their pharmaceutical ly acceptable salts.

9. Use according to Claims 1 to 7 of compounds of the general formula (I) according to Claim 1, in which n represents 0 or 1,

$R^1$

a) represents hydrogen, a straight-chain, branched cyclic saturated or unsaturated aliphatic hydrocarbon radical with up to 10 C atoms which optionally contains by one or two identical or different hetero chain members from the group comprising O, CO, S, = N- and $NR^I$, this hydrocarbon radical optionally being substituted by F, Cl, Br, $NO_2$, CN, OH, phenyl or pyridyl or

b) represents a phenyl, naphthyl or pyridyl radical, these radicals optionally carrying 1 or 2 identical or different substituents from the group comprising phenyl, alkyl, alkenyl, alkoxy, alkenoxy with in each case up to 4 C atoms, benzyl,acetyl, alkylene, dioxyalkylene with 2 to 4 C atoms, fluorine, chlorine, bromine, $CF_3$, $OCF_3$, $SCF_3$, $NO_2$, CN, $COOR^{V}$, $OR^{VI}$, $NR^I$ and $NR^{VII}R^{VIII}$, it being possible for the alkyl, alkoxy and aryl radicals of the abovementioned substituents in their turn also to be halogen substituted or

c) represents the radical $NR^{VII}R^{VIII}$, the radicals $R^I$ to $R^{VIII}$ mentioned under a), b) and

c) having the meaning given below,

$R^2$

a) represents one of the substituents mentioned under $R^1$ but does not have to be identical thereto or

b) represents the radicals $NHR^I$ or

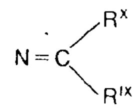

$R^I$, $R^{IX}$ and $R^X$ having the meaning given below, or the substituents,

$R^1$ and $R^2$ together form a 5- to 7-membered saturated or unsaturated ring which optionally contains 1 or 2 identical or different ring members from the group comprising O, S, $NR^I$ and CO and which optionally contains 1 or 2 identical or different substituents from the group comprising halogen, hydroxyl, alkyl, alkoxy each with 1 to 4 C atoms, phenyl and benzyl,

$R^3$ represents one of the substituents mentioned under $R^2$ and is identical thereto or differs therefrom, only one of the two substituents $R^2$ or $R^3$ in each case representing alkoxy, alkylthio or $NHR^I$,

$R^4$

a) represents hydrogen, $NO_2$, CN, $SR^{XI}$, $SO_2R^{XI}$,

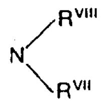
,
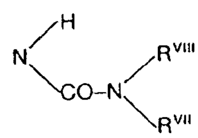

or

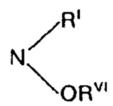

$R^I$, $R^{VI}$, $R^{VII}$, $R^{VIII}$ and $R^{IX}$ having the meaning given below,

b) represents a branched or unbranched alkyl or cycloalkyl radical with up to 8 C atoms, which is optionally substituted by halogen, hydroxyl, cyano, alkoxy with 1 to 4 C atoms, phenyloxy, $COOR^V$ or

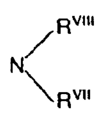

$R^V$, $R^{VII}$ and $R^{VIII}$ having the meaning given below or

c) represents an aromatic hyrocarbon radical with 6 to 10 C atoms or a 5- to 7-membered saturated or unsaturated hetero ring with 1 to 3 identical or different hetero members from the group comprising O, S, =N- and $NR^I$, this hetero ring being linked to the dihydropyridine ring either via a carbon atom or a nitrogen atom, and the aromatic hydrocarbon radical and the hetero rings optionally carrying 1 or 2 identical or different substituents from the group comprising halogen, hydroxyl, cyano, $CF_3$, $NO_2$, phenyl, alkyl and alkoxy each with 1 to 4 C atoms, or

d) represents the radical

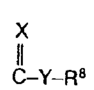

wherein X represents oxygen or $NR^I$ and Y represents a single bond, oxygen or $NR^I$ and $R^8$ has for meaning given for $R^1$ and is identical to the substituent or differs therefrom, or

e) represents the radical

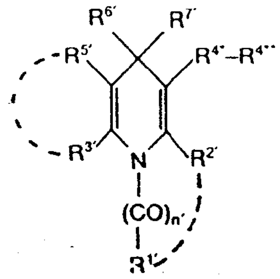

wherein

n′, $R^{1'}$, $R^{2'}$, $R^{3'}$, $R^{5'}$, $R^{6'}$ and $R^{7'}$ have the meaning given for n, $R^1$, $R^2$, $R^3$, $R^5$, $R^6$ and $R^7$ and are identical thereto or diHer therefrom, and

$R^{4*}$ and $R^{4**}$ are identical or different and each represent a radical, minus one hydrogen, of the substituents mentioned for $R^4$ under a) to d), or

$R^2$ and $R^4$ together form a branched unbranched saturated or unsaturated 5- to 7- membered ring which optionally contains 1 or 2 identical or different ring members from the group comprising O, CO, CS, C=NR$^I$, =N- and NR$^I$ and which is optionally substituted by halogen or hydroxyl

$R^1$ having the meaning given below,

$R^5$ has the meaning given for $R^4$ and is identical to $R^4$ or differs therefrom or

$R^5$ and $R^3$ together form a ring of the kind defined for $R^2$ and $R^4$ and is identical thereto or differs therefrom,

$R^6$ represents hydrogen or alkyl with 1 to 4 C atoms which is optionally substituted by fluorine, chlorine or bromine and

$R^7$

a) represents a saturated, unsaturated cyclic, straight-chain or branched aliphatic hydrocarbon radical with up to 8 C atoms, which is optionally substituted by halogen, or

c) represents a phenyl or heteroaryl radical which optionally contains 1 to 3 identical or different substituents from the group comprising $NO_2$, CN, $N_3$, $CF_3$, halogen, COR$^{IV}$, COR$^V$, OR$^{VI}$, SR$^{XI}$,

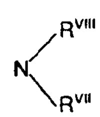

phenyl, alkyl with 1 to 4 C atoms, benzyl and acyl with 1 to 4 C atoms,

and in the abovementioned definitions of the substituents $R^1$ to $R^8$

$R^I$ represents hydrogen, alkyl with 1 to 6 C atoms, phenyl, naphthyl, benzyl, phenethyl, heteroaryl or acyl with up to 4 C atoms,

$R^{II}$ and $R^{III}$ are identical or different and in each case represent alkyl with 1 to 6 C atoms, phenyl, naphthyl, benzyl or heteroaryl,

$R^{IV}$, $R^V$ and $R^{VI}$are in each case identical or different and represent hydrogen, alkyl with 1 to 6 C atoms, phenyl, naphthyl, benzyl or heteroaryl, the alkyl, phenyl and benzyl radicals optionally being substituted by fluorine, chlorine, nitro, $CF_3$, methyl, methoxy and methylthio,

$R^{VII}$ and $R^{VIII}$ are in each case identical or different and represent hydrogen, phenyl or benzyl or represent alkyl vvith 1 to 6 carbon atoms, which is optionally interrupted by O or NR$^I$, or together with the nitrogen atom, form a 5- to 7- membered ring which can contain 1 or 2 identical different hetero ring members from the group comprising O, S and NR$^I$,

or

one of the radicals $R^{VII}$ or $R^{VIII}$ represents an aliphatic acyl group with up to 6 carbon atoms and, $R^{IX}$, $R^X$, $R^{XI}$, $R^{XII}$, and $R^{XIII}$ are in each case identical or different and represent alkyl with 1 to 6 C atoms, phenyl or benzyl, the following substituents being mentioned as heteroaryl:

thienyl, furyl, pyryl, pyridyl, quinolyl, isoquinolyl, pyrimidyl, pyridazinyl, quinazolyl, quinoxalyl, benzothienyl, pyrazolyl, imidazolyl, oxazolyl, isoxazolyl, thiazolyl, triazolyl, oxydiazolyl, pyrazinyl, oxazinyl, thiazinyl, indolyl, benzofuranyl, indazolyl, benzothienyl, benzimidazolyl, benzoxazolyl, benzisoxazolyl, benzthiazolyl, benztriazolyl or benzoxadiazolyl.

10. Use according to Claims 1 to 7 of compounds of the general formula (I) according to Claim 1 in which

n represents O,

$R^1$

a) represents hydrogen, an aliphatic hydrocarbon radical with up to 6 C atoms, which optionally contains a hetero chain member from the group comprising O, CO, =N- and $NR^I$ and which is optionally substituted by halogen, nitro, hydroxyl or phenyl or

b) represents a phenyl or pyridyl radical which are optionally substituted by halogen, $NO_2$, $CF_3$, $OCF_3$, CN, $COOR^V$ or $NR^{VII}R^{VIII}$,

$R^2$ represents one of the substituents mentioned under

$R^1$ but does not have to be identical thereto or represents one of the radicals $NHR^I$ or $N{=}CR^XR^{XI}$,

$R^3$ represents one of the substituents mentioned under

$R^2$ and is identical thereto or differs therefrom,

$R^4$

a) represents hydrogen, $NO_2$, $NR^{VII}R^{VIII}$,

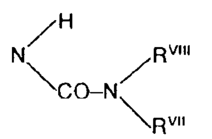

or halogen, or

b) represents an alkyl radical with 1 to 4 C atoms, which is optionally substituted by halogen, OH, CN, alkoxy with 1 to 4 C atoms, $COOR^V$ or $NR^{VII}R^{VIII}$ or

c) represeents phenyl, pyridyl or thienyl, which are optionally substituted by halogen, OH, CN, alkyl or alkoxy with in each case 1 to 4 C atoms or by $NR^{VII}R^{VIII}$ or

d) represents the radical

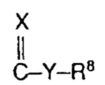

wherein X denotes oxygen and Y represents a single bond, oxygen or $NR^I$ and $R^8$ has the meaning given for $R^1$ and is identical to the substituent $R^1$ or differs therefrom or

e) represents the radical

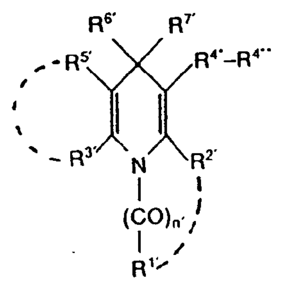

wherein n′, $R^{1'}$, $R^{2'}$, $R^{3'}$, $R^{5'}$, $R^{6'}$ and $R^{7'}$ have the meaning given for n, $R^1$, $R^2$, $R^3$, $R^5$, $R^6$ and $R^7$ and are identical thereto or differs therefrom, and

$R^{4*}$ and $R^{4**}$ are identical or different and each represent a radical, minus one hydrogen, of the substituents mentioned for $R^4$ under a) to d), or

$R^2$ and $R^4$ together form a 5- to 7-membered ring which optionally contains 1 to 2 different ring members from the group comprising O, CO, CS and $C{=}NR^I$ and which is optinally substituted by halogen,

$R^5$ has the meaning given for $R^4$ and is identical to $R^4$ or differs therefrom,

$R^6$ represents hydrogen or alkyl with 1 to 4 C atos and $R^7$ has the abovementioned meaning, the subtituent $R^I$ to $R^{VIII}$ marked with Roman numerals in the abovementioned definitions having the meaning given in Claim 4.

11. Use according to Claims 1 to 7 of compounds of the general formula (I) according to Claim 1, in which at least one of the substituents $R^4$ and $R^5$ representents $NO_2$ and/or in which $R^2$ and $R^4$ together or $R^3$ and $R^5$ together form a lactone ring.

12. Compounds of the general formula (I)

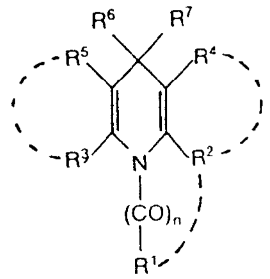

(I)

in which at least one of the substituent $R^4$ and $R^5$ represents the group

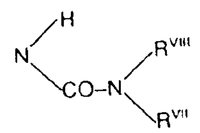

wherein $R^{VIII}$ and $R^{VII}$ and the substituents $R^1$, $R^2$, $R^3$, $R^6$, $R^7$ and n and the remaining radical of $R^4$ and $R^5$ have the meaning given in Claims 1 and 8 to 11.

13. Compounds of the general formula (I)

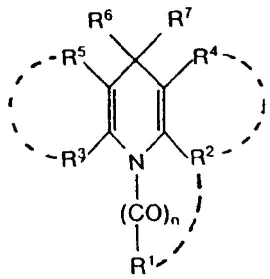

in which at least one of the substituents $R^4$ and $R^5$ represents methyl, wherein $R^1$, $R^2$, $R^3$, $R^6$, $R^7$ and n and the remaining radical of $R^4$ and $R^5$ have the meaning given in Claims 1 and 8 to 11.

14. Compounds of the general formula (I)

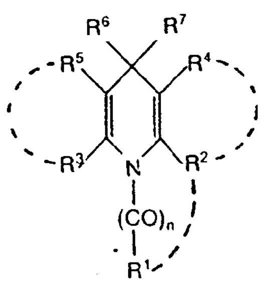

in which one of the radical $R^4$ or $R^5$ denotes hydrogen, $R^1$ represents hydrogen and n represents O an $R^2$, $R^3$, $R^6$ and $R^7$ and the other radical of $R^4$ and $R^5$ have the meaning given in Claims 1 and 8 to 11.

15. Compounds of the general formula (I)

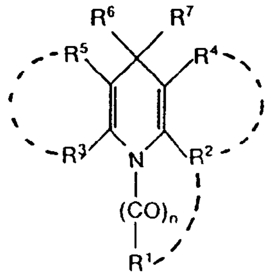

in which one of the radicals $R^4$ and $R^5$ denotes halogen and $R^1$, $R^2$, $R^3$, $R^6$ and $R^7$ and n and the other radical of $R^4$ or $R^5$ have the meaning given in Claim 1 and 8 to 11.

16. Compounds according to Claim 15, in which one of the radicals $R^4$ or $R^5$ represents fluorine or chlorine.

17. Process for the preparation of compounds according to Claims 12 to 16, characterised in that

a) carbonyl compounds of the general formula (II)

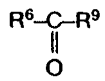 (II)

are reacted, with ketones of the formulae (III) and (IV)

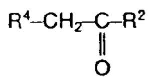 (III) 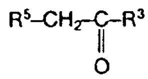 (IV)

and primary amines of the formula (V)

$H_2N-(CO)_n-R^1$

in which n is 0,

if appropriate in the presence of inert solvents, or

b) carbonyl compounds of the formula (II)

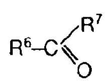 (II)

are reacted with enamines of the formula (VI)

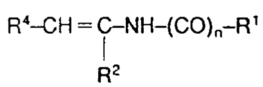 (VI)

and ketones of the formula (IV)

$R^3-CO-CH_2-R^5$

if appropriate in the presence of inert solvents or

c) enamines of the formula (VI)

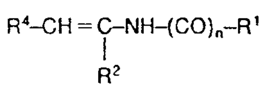 (VI)

are reacted with ylidene compounds of the formula (VIII)

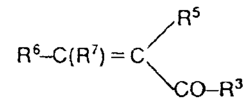

(VII)

if appropriate in the presence of inert solvents, or

d) one or more functional groups of dihydropyridines are modified by customary methods of hydrolysis, esterification, transesterification, lactonisation, condensation, acylation, reduction or cyclisation.

18. Medicaments containing at least one compound according to Claims 12 to 16.

19. Process for the preparation of medicaments, characterised in that at least one compound according to Claims 12 to 16 is converted into a suitable form of administration, if appropriate using inert auxiliaries and excipients.

## Revendications

1. Utilisation des 1,4-dihydropyridines de formule générale (I)

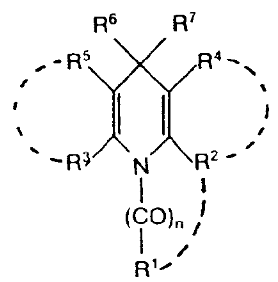

(I)

dans laquelle
n signifie 0, 1 ou 2

$R^1$

a) représente de l'hydrogène, un radical hydrocarbure cyclique à chaîne droite ou ramifiée, aliphatique saturé ou insaturé, qui comporte éventuellement 1 à 3 hétéroéléments de chaîne identiques ou différents appartenant au groupe O, CO, $SO_m$(m = 0, 1 ou 2), =N-, $NR^I$ ou $SiR^{II}R^{III}$, ce radical d'hydrocarbure étant éventuellement substitué par un halogène, $NO_2$, CN, $N_3$, un radical hydroxy, aryle ou hétéroaryle, ou bien

b) représente un radical aryle ou hétéroaryle où ces radicaux comportent éventuellement 1 à 5 produits de substitution identiques ou différents, provenant des groupes aryle, alkyle, alkényle, alkinyle, alkénoxy, alkinoxy, aralkyle, acyle, alkylène, dioxyalkylène, halogène, $CF_3$, $OCF_3$, $SCF_3$, $NO_2$, NO, CN, $N_3$, $COR^{IV}$, $COOR^V$, $OR^{VI}$, $NR^I$ ou $NR^{VII}R^{VIII}$, les radicaux alkyle, alkoxy et aryle des produits de substitution pouvant être, à leur tour, substitués par un halogène $COOR^V$ ou $NR^{VII}R^{VIII}$ ou

c) représente le radical $NR^{VII}R^I$, tandis que les radicaux mentionnés sous a), b) et c), $R^I$, $R^{II}$, $R^{III}$, $R^{IV}$, $R^V$, $R^{VI}$, $R^{VII}$, et $R^{VIII}$ ont la signification mentionnée ci-dessous.

$R^2$

a) représente l'un des produits de substitution mentionnés sous $R^I$ mais ne doit pas être identique à celui-ci, ou

b) représente les radicaux $NHR^I$ ou

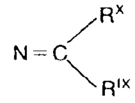

où $R^I$, $R^{IX}$ et $R^X$ on les significations mentionnées ci-dessous,

les produits de substitution $R^1$ et $R^2$ forment ensemble un anneau saturé ou insaturé à 5 à 8 éléments, qui contient, éventuellement, 1, 2 ou 3 éléments d'anneau identiques ou différents parmi O, S, $NR^I$ ou CO et qui contient, éventuellement, 1 à 3 produits de substitution identiques ou différents des groupes halogène, hydroxy, alkyle, alkoxy, aryle ou aralkyle,

$R^3$ représente l'un des groupes substituants mentionnés sous $R^2$ et est identique ou différent de celui-ci, tandis qu'un seulement des deux groupes substituants $R^2$ ou $R^3$ représente, chaque fois, un alkoxy, un alkylthio ou un $NHR^I$,

$R^4$

a) représente de l'hydrogène, $NO_2$, NO, CN, $SO_m$-$R^{XI}$ (m = 0,1 ou 2), ou un halogène,

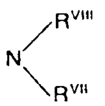
,
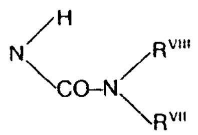
,
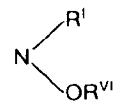

ou
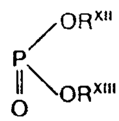

où $R^I$, $R^{VII}$, $R^{VIII}$ $R^{XI}$ et $R^{XIII}$ ont la signification mentionnée ci-dessous, ou

b) représente un radical d'hydrocarbure cyclique ramifié ou non ramifié, aliphatique saturé ou non saturé qui est éventuellement substitué par un halogène, OH, CN, un alkoxy, un alkylthio, un aryloxy, $COOR^V$, ou

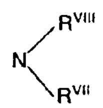

tandis que $R^V$, $R^{VII}$ et $R^{VIII}$ ont la signification mentionnée ci-dessous, ou

c) représente un radical hydrocarbure aromatique ou un anneau hétérocyclique saturé ou insaturé à 5 à 7 atomes avec 1 à 4 hétéroéléments identiques ou différents des groupes O, S, -N =, $NR^I$, où cet anneau hétérocyclique est relié par un atome de carbone ou par un atome d'azote à l'anneau de la dihydropyridine et où le radical d'hydrocarbure aromatique et les anneaux hétérocycliques portent éventuellement 1 à 3 groupes substituants identiques ou différents des groupes halogène, OH, CN, $CF_3$, $OCF_3$, $SCF_3$, $NO_2$, alkyle, alkoxy, aryle et

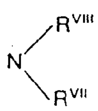

d) représente le radical

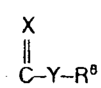

où X est de l'oxygène, du soufre ou $NR^I$ et où Y représente une liaison simple, O, S ou $NR^I$ et où $R^8$ a la signification indiquée pour $R^1$ et est identique au groupe substituant $R^1$ ou est différent de celui-ci, ou

e) représente le radical

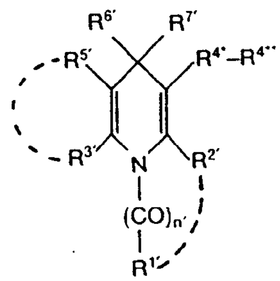

où

n′, $R^{1'}$, $R^{2'}$, $R^{3'}$, $R^{5'}$, $R^{6'}$ et $R^{7'}$ ont les significations mentionnées pour n, $R^1$, $R^2$, $R^3$, $R^5$, $R^6$ et $R^7$ et sont identiques à ceux-ci ou différents de ceux-ci, et où

$R^{4*}$ et $R^{4**}$ sont identiques ou différents et représentent chacun un radical diminué d'un atome d'hydogène des groupes substituants mentionnés sous a) à d) pour $R^4$, ou

$R^2$ et $R^4$ forment ensemble un anneau ramifié, non ramifié, saturé, non saturé, à 5 à 8 éléments, qui contient éventuellement 1, 2 ou 3 éléments d'anneaux différents ou identiques, parmi O, CO, CS, C=$NR^I$, =N-, $NR^I$, $SO_m$(m = 0,1 ou 2) ou $SiR^{II}R^{III}$ et qui est éventuellement substitué par un halogène, un hydroxy, un alkoxy, un aryle, un aralkyle,

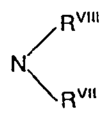

ou qui est disubstitué par une chaîne alkylène droite ou ramifiée à 3 ou 8 atomes de carbone, où cet anneau commun de $R^2$ et $R^4$ peut être condensé directement avec l'anneau commun de $R^1$ et $R^2$

où les radicaux $R^I$, $R^{II}$, $R^{III}$, $R^{VII}$, $R^{VIII}$ ont la signification mentionnée ci-dessous.

$R^5$ possède la signification mentionnée pour $R^4$ et est identique à $R^4$ ou est différent de celui-ci, ou

$R^5$ et $R^3$ constituent ensemble un anneau qui est identique à l'anneau formé par $R^2$ et $R^4$ ou est différent de celui-ci,

$R^6$ représente de l'hydrogène, un alkyle ou un alkyle halogéné, et

$R^7$

a) représente un radical d'hydrocarbure cyclique saturé ou insaturé ou aliphatique à chaîne droite ou ramifiée, qui est substitué éventuellement par un halogène, un aryle ou un hétéroaryle, ou

b) représente un radical aryle ou hétéroaryle qui contient éventuellement 1 à 5 groupes substituants identiques ou différents parmi $NO_2$, CN, $N_3$, NO, $CF_3$, des halogènes, $COR^{IV}$, $COOR^V$, $OR^{VI}$.

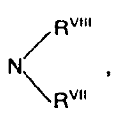 , $SO_mR^{XI}$ (m = 0, 1 ou 2), 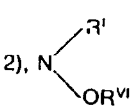 ,

alkyle, aryle, alkenyle, alkinyle, alkenoxy, alkinoxy, aralkyle, acyle, alkylène ou dioxyalkylène, où les groupes substituants alkyle et aryle précités peuvent être substitués, à leur tour, par un halogène, $COOR^V$ ou par

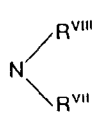

et où, dans les définitions précitées des groupes substituants $R^1$ à $R^8$,
$R^I$ représente de l'hydrogène, un alkyle, un aryle, un aralkyle, un hétéroaryle ou un acyle,
$R^{II}$ et $R^{III}$ sont identiques ou différents et représentent chacun un alkyle, un aryl, un aralkyle ou un hétéroaryle, où $R^{IV}$, $R^V$ et $R^{VI}$ sont chacun identiques ou différents et représentent de l'hydrogène, un alkyle, un aryle, un aralkyle ou un hétéroaryle, où les radicaux alkyle et aryle sont substitués éventuellement par un halogène, un nitro, un trifluorométhyle, un alcoxy, un alkylthio ou un alkyle,
$R^{VII}$ et $R^{VIII}$ sont chacun égaux ou différents et représentent de l'hydrogène, un aryle ou un aralkyle ou représentent un alkyle qui est éventuellement interrompu par O, S ou $NR^I$, ou forment avec l'atome d'azote, un anneau à 5-7 éléments qui peut contenir 1 ou 2 èléments d'anneaux hétérocycliques identiques ou différents des groupes O, S, $NR^I$, ou où l'un des radicaux
$R^{VII}$ ou $R^{VIII}$ représente un groupe acyle aliphatique à 6 atomes de carbone au maximum, où $R^{IX}$, $R^X$, $R^{XI}$, $R^{XII}$, $R^{XIII}$ sont chacun identiques ou différents et représentent un alkyle, un aryle ou un aralkyle, où les radicaux alkyle, aryle, aralkyle, hétéroalkyle et acyle
mentionnées sous $R^1$ à $R^8$ sous $R^I$ à $R^{XIII}$ ainsi que l'anneau hétérocyclique formé avec $R^{VII}$ et $R^{VIII}$ sont éventuellement substitués, pour leur part, par OH, $CF_3$, $OCF_3$, CN, $NO_2$, un halogène, un thioalkyle inférieur, un alkyle inférieur, un alkoxy inférieur, un aryle ou un aralkyle,
et où les groupes substituants suivants peuvent être cités comme hétéroaryles:
thiényle, furyle, pyryle, pryridyle, quinoléyle, isoquinoléyle, pyrimidyle, pyridazinyle, quinazolyle, quinoxalyle, benzothiényle, pyrarolyle, imidazolyle, oxazolyle, isoxazolyle, thiazolyle, triazolyle, oxydiazolyle, pyrazinyle, oxazinyle, thiazinyle, indolizinyle, indolyte, benzofuranyle, indazolyle, benzothiényle, benzimidazolyle, benzoxazolyle, benzisoxazolyte, benzthiazolyte, benstriazolyte, benzoxadiazolyte, cinnolinyle, phtalazinyle, naphtyridinyle ou benzotriazinyle.
sous forme d'isomères, de mélanges d'isomères, de racémates et d'antipodes optique, ainsi que de leurs sels qui ne présentent pas d'inconvénients en pharmacie et qui, dès une concentration de $10^{-5}$ g/ml provoquent dans l'oreillette gauche isolé du cobaye un renforcement des contractions d'au moins 25%, en vue de la production de médicaments augmentant la pénétration de l'ion $Ca^{++}$
dans les cellules.

2. Utilisation, selo la revendication 1, des composés de la formule générale (I) pour la production de médicaments ayant un effet inotrope positif.

3. Utilisation, selon la revendication 1, de composés de la formule générale (I) pour la production de médicaments ayant un effet de tonique cardiaque.

4. Utilisation, selon la revendication 1, de composés de la formule générale (I) pour la production de médicaments ayant un effet anti-hypotonique.

5. Utilisation, selon la revendication 1, des composés de la formule générale (I) pour la production de médicaments, afin de diminuer la teneur en sucre dans le sang.

6. Utilisation, selon la revendication 1, des composés de la formule générale (I) pour la production de médicaments, en vue de la décongestion des muqueuses.

7. Utilisation, selon la revendication 1, de composés de la formule générale (I) pour la production de médicaments, afin d'influencer le métabolisme des sels et des liquides.

8. Utilisation, selon les revendications 1 à 7, des composés de la formule générale (I) et, selon la revendication 1, dans laquelle:
n représente 0, 1 ou 2
$R^1$

a) représente de l'hydrogène, un radical d'hydrocarbure aliphatique à chaîne droite ou ramifiée, cyclique saturé ou non saturé, avec au maximum 10 atomes de carbone qui contient éventuellement 1 ou 2 hétéroéléments de chaîne identiques ou différents des groupes O, CO, S, $SO_2$, = N-, ou $NR^I$ et où ce radical d'hydrocarbure est substitué éventuellement par un halogène $NO_2$, CN, $N_3$, hydroxy, avec 1 à 4 atomes de carbone, du phényle, du naphtyle ou de l'hétéroaryle ou

b) représente un radical phényle, naphtyle ou hétéroaryle où ces radicaux portent éventuellement 1 à 3 groupes substituants identiques ou différents des groupes phényle, alkyle, alkényle, alkinyle, alkénoxy, alkinoxy avec, chaque fois, un maximum de 4 atomes de carbone, aralkyle avec 7 à 14 atomes de carbone, acyle avec un maximum de 6 atomes de carbone, alkylène, dioxyalkylène, avec un maximum de 4 atomes de carbone dans la chaîne alkylène, un halogène, $CF_3$, $OCF_3$, $SCF_3$, $NO_2$, CN, $N_3$, $COR^{IV}$, $COOR^V$, $OR^{VI}$, $NR^I$ ou $NR^{VII}R^{VIII}$, où les radicaux alkyle, alkoxy et aryle des groupes substituants précités peuvent être substitués à leur tour à nouveau par un halogène, $COOR^V$ ou $Nr^{VII}R^{VIII}$, ou

c) représente le radical $NR^{VII}R^I$ et où les radicaux $R^I$, $R^{II}$, $R^{III}$, $R^{IV}$, $R^V$, $R^{VI}$, $R^{VII}$ et $R^{VIII}$ mentionnés sous a), b) et c) ont la signification indiquée ci-dessous,

$R^2$

a) représente un groupe substituant indiqué sous $R^1$, mais ne doit pas être identique à celui-ci, ou
b) représente le radical $NHR^I$ ou

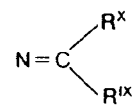

où $R^I$, $R^{IX}$ et $R^X$ possèdent la signification mentionnée ci-dessous,
ou les groupes substituants

$R^1$ et $R^2$ forment ensemble un anneau saturé ou insaturé à 5 ou 7 éléments qui contient éventuellement 1 ou 2 éléments d'anneaux identiques ou différents des groupes O, S, $NR^I$ ou CO et qui contient éventuellement 1 à 3 groupes substituants identiques ou différents des groupes halogène, hydroxy, alkyle, alkoxy, avec chacun 1 à 4 atomes de carbone, phényle, naphtyle ou aralkyle avec 7 à 14 atomes de carbone,

$R^3$ représente un groupe substituant mentionné sous $R^2$ et est identique à celui-ci ou est différent de celui-ci, où un seul des deux groupes substituants $R^2$ ou $R^3$ représente chaque fois un alkoxy, un alkylthio ou $NHR^I$,

$R^4$

a) représente de l'hydrogène, $NO_2$, NO, CN, $SO_m$-$R^{IX}$(M = 0, ou 2), un halogène,

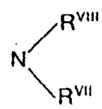
,
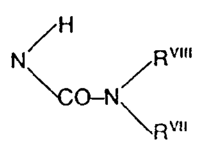
,
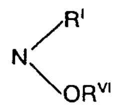

ou
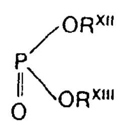

où $R^I$, $R^{VII}$, $R^{VIII}$, $R^{XI}$ et $R^{XIII}$ ont la signification mentionnée ci-dessous, ou
b) représente un radical d'hydrocarbure cyclique ramifié ou non ramifié, aliphatique saturé ou non saturé, avec au maximum 10 atomes de carbone, qui est substitué éventuellement par un halogène, OH, CN, un alkoxy, un alkoxy, un alkylthio, avec fois 1 à 4 atomes de carbone, un phényloxy, un naphtoxy, $COOR^V$ ou

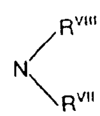

où $R^V$, $R^{VII}$ et $R^{VIII}$ ont la signification mentionnée ci-dessous, ou
c) représente un radical d'hydrocarbure aromatique avec de 6 à 10 atomes de carbone ou un anneau hétérocyclique saturé ou insaturé à 5 à 7 éléments, avec 1 à 3 hétéroéléments identiques ou différents du groupe O, S, -N=, $NR^I$, où cet anneau hétérocyclique est relié soit par un atome de carbone, soit par un atome d'azote, à l'anneau de la dihydropyridine et où le radical d'hydrocarbure aromatique et les anneaux hétérocycliques portent éventuellement 1 à 3 groupes substituants identiques ou différents du groupe des halogènes, OH, CN, $CF_3$, $OCF_3$, $SCF_3$, $NO_2$, alkyle, alkoxy avec chaque fois 1 à 4 atomes de carbone, phényle, naphtyle et

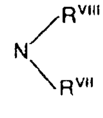

ou
d) représente un radical

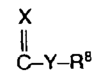

où X est de l'oxygène, du soufre ou $NR^I$ et où Y représente une laison simple, O, S ou $NR^I$ et où $R^8$ a la signification mentionnée pour $R^1$ et est identique au substituant $R^1$ ou est différent de celui-ci ou

e) représente le radical

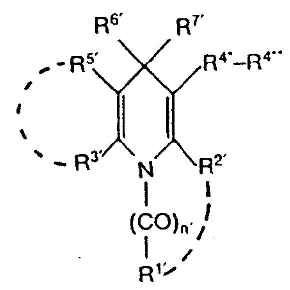

où

n', $R^{1'}$, $R^{2'}$, $R^{3'}$, $R^{5'}$, $R^{6'}$ et $R^{7'}$ on la significationindiquée pour n, $R^1$, $R^2$, $R^3$, $R^5$, $R^6$ et $R^7$ et sont identiques à ceux-ci ou différents de ceux-ci et

$R^{4*}$ et $R^{4**}$ sont identiques ou différents et représentent, chaque fois, un radical réduit d'un atome d'hydrogène des groupes substituants mentionnés pour $R^4$ sous a) à b), ou

$R^2$ et $R^4$ constituent ensemble un anneau ramifié, non ramifié, saturé ou insaturé à 5 à 7 éléments, qui contient éventuellement 1, 2 ou 3 éléments d'anneau identiques ou différents des groupes O, CO, CS, $C{=}NR^I$, =N-, $NR^I$ ou $SO_m$ (m = 0 ou 2) et qui est éventuellement substitué par un halogène, un hydroxy, un alkoxy, avec 1 à 4 atomes de carbone, un radical phényle, napthyle, aralkyle avec 7 à 14 atomes de carbone,

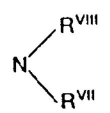

ou qui est disubstitué par une chaîne alkylène droite ou ramifiée, avec 3 à 8 atomes de carbone et où cet anneau commun de $R^2$ et $R^4$ peut former aussi directement un anneau avec l'anneau commun de $R^1$ et $R^2$ et où les radicaux $R^I$, $R^{II}$, $R^{III}$, $R^{VII}$, $R^{VIII}$ on la signification mentionnée ci-dessous,

$R^5$ possède la signification mentionnée pour $R^4$ et est identique à $R^4$ ou est différent de celui-ci,

$R^6$ représente de l'hydrogène, un alkyle ou un alkyle halogéné, avec chaque fois 1 à 4 atomes de carbone et

$R^7$

a) représente un radical d'hydrocarbure cyclique saturé ou insaturé aliphatique à chaîne droite ou ramifiée, avec 10 atomes de carbone au maximum, qui est substitué éventuellement par un halogène, un groupe phényle, naphtyle ou hétéroaryle, ou

b) représente un radical phényle, naphtyle ou hétéroaryle qui contient éventuellement 1 à 3 groupes substituants identiques ou différents parmi les groupes $NO_2$, Halogène, CN, $N_3$, NO, $CF_3$, $COR^{IV}$, $COOR^V$, $OR^{VI}$,

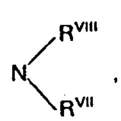 , $SO_mR^{XI}$ (m = 0, 1 ou 2), 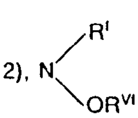 ,

alkyle avec 1 à 4 atomes de carbone, phényle, naphtyle, alkenyle, alkinyle, alkenoxy, alkinoxy avec chaque fois 4 atomes de carbone au maximum, aralkyle avec 7 à 14 atomes de carbone, acyle avec 1 à 4 atomes de carbone, alkylène ou dioxyalkylène avec chaque fois 4 atomes de carbone au maximum, où les groupes substituants alkyle et aryle précités peuvent être substitués à nouveau à leur tour par un halogène, $COOR^V$ ou

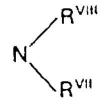

et où, dans les définitions précitées des groupes substituants $R^1$ à $R^7$,
$R^I$ représente de l'hydrogène, un alkyle à 1 à 6 atomes de carbone, un phényle, un naphtyle, un aralkyle à 7 à 12 atomes de carbone, un hétéroaryle ou un acyle à 7 atomes de carbone au maximum, où $R^{II}$ et $R^{III}$ sont identiques ou différents et représentent chacun un alkyle à 1 à 6 atomes de carbone, un phényle, un naphtyle, un aralkyle avec 7 à 12 atomes de carbone ou un hétéroaryle,
$R^{VII}$, $R^V$ et $R^{VI}$ sont chaque fois identiques ou différents et représentent de l'hydrogène, un alkyle avec 1 à 6 atomes de carbone, un phényle, un naphtyle, un aralkyle avec 7 à 12 atomes de carbone ou un hétéroaryle, les radicaux alkyle et aryle étant éventuellement substitués par halogène, nitro, trifuoro-méthyle, alcoxy, alkylthio et alkyle avec chaque fois 1 à 4 atomes de carbone, $R^{VII}$ et $R^{VIII}$ sont chaque fois identiques ou différents et représentent de l'hydrogène, un phényle, un naphtyle ou un aralkyle à 7 à 12 atomes de carbone, ou un alkyle à 1 à 6 atomes de carbone qui est interrompue éventuellement par O, S ou $NR^I$ ou constituent, avec l'atome d'azote, un anneau à 5 à 7 éléments qui peut contenir 1 ou 2 éléments d'anneaux hétérocycliques identiques ou différents des groupes O, S ou $NR^I$ ou
où l'un des radicaux $R^{VII}$ et $R^{VIII}$ représente un groupe acyle aliphatique avec 6 atomes de carbone au maximum, $R^{IX}$, $R^X$, $R^{XII}$ et $R^{XIII}$ sont chaque fois identiques ou différents et représentent un alkyle avec 1 à 6 atomes de carbone, un phényle, un naphyle ou un aralkyle à 7 à 12 atomes, où les radicaux alkyle, aryle, aralkyle, hétéroaryle et acyle
mentionnés sous $R^1$ à $R^8$, ainsi que l'anneau hétérocyclique formé avec $R^6$ et $R^7$ sont substituées éventuellement, pour leur part, par OH, $CF_3$, $OCF_3$, CN, $NO_2$, un halogène, un alkyle à 1 à 4 atomes de carbone, un alkoxy, un alkylthio à 1 à 4 atomes de carbone, un radical phényle ou benzyle, et où les groupes substituants suivants peuvent être mentionnés comme hétéroaryles:
thiényle, furyle, pyryle, pyridyle, quinoléyle, isoquinoléyle, pyrimidyle, pyridazinyle, quinazolyle, quinoxalyde, benzothiényle, pyrazolyle, imidazolyle, oxazolyle, isoxazolyle, thiazolyle, triazolyle, oxydiazolyle, pyrazinyle, oxazinyle, thiazinyle, indolizinyle, indolyle, benzofuranyle, indazolyle, benzothiényle, benzimidazolyle, benzoxazolyle, benzisoxazolyle, benzthiazolyle, benztriazolyle, benzoxadiazolyle, cinnolinyle, phtalazinyle, naphtyridinyle ou benzotriazinyle, sous forme d'isomères, de mélanges d'isomères,
de racémates et d'antipodes optiques, ainsi que de leurs sels ne présentant pas de contre-indications pharmaceutiques.

9. Utilisation, selon les revendications 1 à 7, des composés de la formule générale (I) selon la revendication 1, dans laquelle
n représente 0 ou 1,

$R^1$

a) représente un radical d'hydrocarbure cyclique à chaîne droite ou ramifiée, aliphatique saturé ou non saturé, avec 10 atomes de carbone au maximum, qui contient éventuellement 1 ou 2 éléments de chaîne hétérocyclique identiques ou différents parmi O, CO, S, =N- ou $NR^I$ et où ce radical d'hydrocarbure est substitué éventuellement par F, Cl, Br, $NO_2$, CN, OH, un phényle ou un pyridyle ou

b) représente un radical phényle, naphtyle ou pyridyle où ces radicaux portent éventuellement 1 ou 2 groupes substituants identiques ou différents faisant partie des groupes phényle, alkyle, alkényle, alkoxy, alkénoxy avec chaque fois 4 atomes de carbone au maximum, benzyle, acétyle, alkylène, dioxyalkylène avec 2 à 4 atomes de carbone, fluor, chlore, brome, $CF_3$, $OCF_3$, $SCF_3$, $NO_2$, CN, $COOR^V$, $OR^{VI}$, $NR^I$ ou $NR^{VII}R^{VIII}$, où les radicaux alkyle, alkoxy et aryle des groupes substituants précités peuvent être substitués à leur tour à nouveau par un halogène ou

c) représente le radical $NR^{VII}R^{VIII}$, et où les radicaux $R^I$ à $R^{VIII}$ mentionnés sous a), b) et c) ont la signification mentionnée ci-dessous.

$R^2$

a) représente l'un des groupes substituants mentionnés sous $R^1$ mais ne doit pas être identique à celui-ci ou

b) représente le radical $NHR^I$ ou

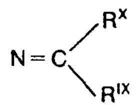

où $R^I$, $R^{IX}$ et $R^X$ possèdent la signification mentionnée ci-dessous ou les groupes substituants $R^1$ et $R^2$ forment ensemble un anneau saturé ou insaturé à 5 à 7 éléments, qui contient éventuellement un ou 2 éléments d'anneau identiques ou différents faisant partie des groupes O, S, $NR^I$ ou CO et qui contient éventuellement 1 ou 2 groupes substituants identiques ou différents faisant partie des groupes halogène, hydroxy, alkyle, alkoxy, avec de 1 à 4 atomes de carbone, phényle ou benzyle,

$R^3$ représente l'un des groupes substituants mentionnés sous $R^2$ et est identiques à celui-ci ou différent de celui-ci, tandis qu'un seul des deux groupes substituants $R^2$ ou $R^3$ représente chaque fois un alkoxy, un alkylthio ou $NHR^I$,

$R^4$

a) représente de l'hydrogène, $NO_2$, CN, $SR^{XI}$, $SO_2R^{XI}$,

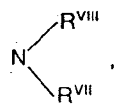 , 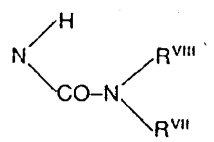

ou 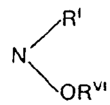

où $R^I$, $R^{VI}$ $R^{VII}$, $R^{VIII}$ et $R^{IX}$ ont la signification mentionnée ci-dessous,

b) représente un radical alkyle oou cycloalkyle ramifié ou non ramifié, avec 8 atomes de carbone au maximum qui est substitué éventuellement par un halogène, un hydroxy, un cyano, un alkoxy à 1 à 4 atomes de carbone, un phényloxy, $COOR^V$ ou

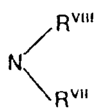

où $R^V$, $R^{VII}$ et $R^{VIII}$ ont la signification mentionnée ci-dessous, ou

c) représente un radical d'hydrocarbure aromatique à 6 à 10 atomes de carbone, ou un anneau hétérocyclique saturé ou insaturé à 5 à 7 éléments, avec 1 à 3 éléments hétérocycliques identiques ou différents, choisis dans les groupes O, S, = N-, $NR^I$, où cet anneau hétérocyclique ést relié soit par un atome de carbone, soit par un atome d'azote, à l'anneau de la dihydropyridine et où le radical d'hydrocarbure aromatique et les anneaux hétérocycliques portent éventuellement un ou deux groupes substituants identiques ou différents, choisis parmi les groupes des halogènes, hydroxy, cyano, $CF_3$, $NO_2$, phényle, alkyle et alkoxy avec, chacun, 1 à 4 atomes de carbone, ou

d) représente le radical

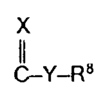

où X représente e l'oxygène ou $NNR^I$ et où Y représente une liaison simple, de l'oxygène ou $NR^I$ et où $R^8$ a la signification mentionnée pour $R^1$ et est identique au groupe substituant ou est différent de celui-ci ou

e) représente le radical

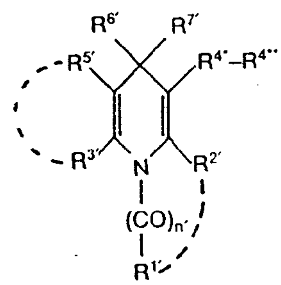

où

n′, $R^{1'}$, $R^{2'}$, $R^{3'}$, $R^{5'}$, $R^{6'}$ et $R^{7'}$ on la signification indiquée pour n, $R^1$, $R^2$, $R^3$, $R^5$, $R^6$ et $R^7$ et sont identiques à ceux-ci ou différents de ceux-ci et

$R^{4*}$ et $R^{4**}$ sont identiques ou différents et représent chacun un radical diminué d'un atome d'hydrogène des groupes substituants mentionnés pour $R^4$ sous a) à d), ou

$R^2$ et $R^4$ forment ensemble un anneau ramifié ou non ramifié, saturé ou non saturé, à 5 ou 7 éléments, qui contient éventuellement un ou deux éléments d'anneau identiques ou différents, choisis dans les groupes O, CO, CSD, C=$NR^I$, =N- ou $NR^I$ et qui est éventuellement substitué par un halogène ou un hydroxy, où $R^1$ a la signification mentionnée ci-dessous,

$R^5$ possède la signification mentionnée pour $R^4$ et est identique à $R^4$ ou différent de celui-ci, ou $R^5$ et $R^3$ forment ensemble un anneau qui est défini comme pour $R^2$ et $R^4$ et qui est identique à celui-ci ou différent de celui-ci

$R^6$ représente de l'hydrogène ou un radical alkyle à 1 à 4 atomes de carbone, qui est substitué éventuellement par le fluor, le chlore, le brome et

$R^7$

a) représente un radical d'hydrocarbure cyclique saturé ou insaturé, aliphatique à chaîne droite ou ramifiée, à 8 atomes de carbone au maximum, qui est substitué éventuellement par un halogène, ou

b) représente un radical phényle ou hétéroaryle qui contient éventuellement 1 à 3 groupes substituants identiques $NO_2$, CN, $N_3$, un halogène, $COR^{IV}$, $COR^V$, $OR^{VI}$, $SR^{XI}$,

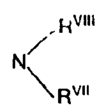

pényle, alkyle, avec 1 à 4 atomes de carbone, benzyle ou acyle à 1 à 4 atomes de carbone, où, dans les définitions précitées des groupes substituants

$R^1$ à $R^8$

$R^I$ représente de l'hydrogène, un alkyle à 1 à 6 atomes de carbone, un phényle, un nathtyle, un benzyle, un phénéthyle, un hètéroaryle ou un acyle à 4 atomes de carbone au maximum,

$R^{II}$ et $R^{III}$ sont identiques ou différents et représentent chacun un alkyle à 1 à 6 atomes de carbone, un phènyle, un naphtyle, un benzyle ou un hétéroaryle,

$R^{IV}$, $R^V$ et $R^{VI}$ sont chaque fois identiques ou différents et représentent de l'hydrogène, un alkyle à 1 à 6 atomes de carbone, un phényle, un naphtyle, un benzyle ou un hétéroaryle, où les radicaux alkyle, phényle et benzyle sont éventuellement substitués par fluor, chlore, nitro, $CF_3$, méthyle, méthoxy et méthylthio,

$R^{VII}$ et $R^{VIII}$ sont chaque fois indentiques ou différents et représentent de l'hydrogène, un phényle ou un benzyle ou un alkyle à 1 à 6 atomes de carbone, qui est interrompu éventuellement par O ou par $NR^I$, ou forment avec l'atome d'azote, un anneau à 5 à 7 éléments qui peut contenir 1 ou 2 éléments d'anneau hétérocycliques identiques ou diffèrents, choisis dans les groupes O, S ou $NR^I$, ou où l'un des radicaux $R^{VII}$ ou $R^{VIII}$ représente un groupe acyle aliphatique avec 6 atomes de carbone au maximum et

$R^{IX}$, $R^X$, $R^{XI}$, $R^{XII}$ et $R^{XIII}$ sont chaque fois identiques ou différents et représentent un groupe alkyle à 1 à 6 atomes de carbone, un phényle ou un benzyle, où les groupes substituants suivants peuvent être mentionnés comme hétéroaryles:

thiényle, furyle, pyryle, pyridyle, quinoléyle, isoquinoléyle, pyrimidyle, pyridazinyle, quinazolyle, quinoxalyle, benzothiényle, pyrazolyte, imidazolyte, oxazolyle, isoxazolyle, thiazolyle, triazolyle, oxydiazolyle, pyrazinyle, oxazinyle, thiazinyle, indolyle, benzofuranyle, indazolyle, benzothiényle, benzimisazolyle, benzoxazolyle, benzisoxazolyle, benzthiazolyle, benztriazolyle et benzoxadiazolyle.

10. Utilisation, selon les revendications 1 à 7, des composés de la formule générale (I) selon la revendication 1, dans laquelle
n représente 0
$R^1$
a) représente de l'hydrogène, un radical d'hydrocarbure aliphatique avec 6 atomes de carbone au maximum, qui contient éventuellement un élément de chaîne hétérocyclique choisi dans les groupes O, CO, =N- ou $NR^I$ et qui est substitué éventuellement par un halogène, un groupe nitro, hydroxy ou phényle, ou
b) représente un radical phényle ou pyridyle qui est substitué éventuellement par un halogène, $NO_2$, $CF_3$, $OCF_3$ CN $COOR^V$ ou $NR^{VII}R^{VIII}$,
$R^2$ représente l'un des groupes substituants mentionnés sous $R^1$ mais ne doit pas être identique à celui-ci ou représente l'un des radicaux $NHR^I$ ou $N{=}CR^XR^{XI}$,
$R^3$ représente l'un des groupes substituants mentionnés sous $R^2$ et est identiques à celui-ci ou différent de celui-ci
$R^4$
a) représente de l'hydrogène, $NO_2$, $NR^{VII}R^{VIII}$, NH-CO-$NR^{VII}R^{VIII}$ ou un halogène, ou
b) représente un radical alkyle à 1 à 4 atomes de carbone, qui est substitué éventuellement par un halogène, OH, CN, un groupe alkoxy à 1 à 4 atomes de carbone, $COOR^V$ ou $NR^{VII}R^{VIII}$, ou
c) représente un groupe phényle, pyridyle ou thiényle qui est substitué éventuellement par un halogène, OH, CN, un alkyle ou un alkoxy, avec chaque fois 1 à 4 atomes de carbone ou par $NR^{VII}R^{VIII}$ ou
d) représente le radical

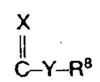

où X représente de l'oxygène et Y représente une liaison simple, l'oxygène ou $NR^I$ et où $R^8$ a la signification mentionnée pour $R^1$ et est identique au groupe substituant $R^1$ ou différent de celui-ci ou
e) représente le radical

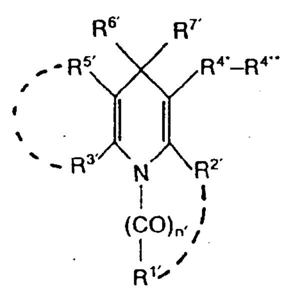

où n', $R^{1'}$, $R^{2'}$, $R^{3'}$, $R^{5'}$, $R^{6'}$ et $R^{7'}$ ont la signification mentionnée pour n, $R^1$, $R^2$, $R^3$, $R^5$, $R^6$ et $R^7$ et sont identiques à ceux-ci ou différents de ceux-ci, et
$R^{4*}$ et $R^{4**}$ sont identiques ou différents et représentent chacun un radical diminué d'un atome d'hydrogène des groupes substituants mentionnés pour $R^4$ sous a) à d), ou
$R^2$ et $R^4$ forment ensemble un anneau, à 5-7 éléments, qui contient éventuellement un ou deux éléments d'anneau identiques ou différents, choisis dans les groupes O, CO, CS, C=$NR^I$, et qui est éventuellement substitué par un halogène,
$R^5$ possède la signification mentionnée pour $R^4$ et est identique à $R^4$ ou différent de celui-ci,
$R^6$ représente de l'hydrogène ou un radical alkyle à 1 à 4 atomes de carbone, et $R^7$ possède la signification mentionnée ci-dessus, où, dans les définitions précitées, les groupes substituants désignés par des chiffres romains $R^I$ à $R^{VIII}$ possèdent la signification mentionnée à la revendication 4.

11. Utilisation, selon les revendications 1 à 7, des composés de la formule générale 1 selon la revendication 1, dans laquelle au moins l'un des groupes substituants $R^4$ et $R^5$ représente $NO_2$ et/ ou dans laquelle $R^2$ et $R^4$ forment ensemble ou bien dans laquelle $R^3$ et $R^5$ forment ensemble un anneau de lactone.

12. Composés de la formule générale (I)

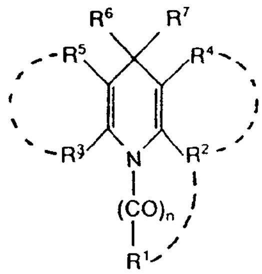

dans laquelle au moins l'un des groupes substituants $R^4$ et $R^5$ représente les groupes

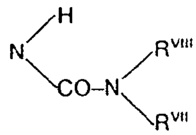

où $R^{VIII}$ et $R^{VII}$ et les groupes substituants $R^1$, $R^2$, $R^3$, $R^6$, $R^7$ et n, ainsi que le radical restant de $R^4$ et $R^5$ ont les significations mentionnées aux revendications 1 et 8 à 11.

13. Composés de la formule générale (I)

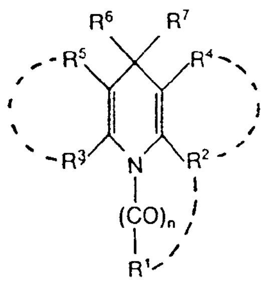

dans laquelle au moins l'un des groupes substituants $R^4$ et $R^5$ représente un radical méthyle, tandis que $R^1$, $R^2$, $R^3$, $R^6$, $R^7$ n, ainsi que le radical restant de $R^4$ et $R^5$ ont la signification mentionnée aux revendications 1 et 8 à 11.

14. Composés de la formule générale (I)

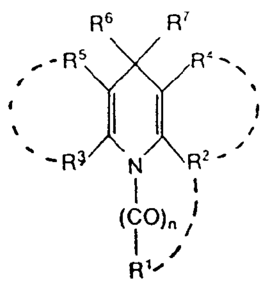

dans laquelle l'un des radicaux $R^4$ ou $R^5$ représente de l'hydrogène, $R^1$ représente de l'hydrogène et n représente 0 et $R^2$, $R^3$, $R^6$ et $R^7$, ainsi que l'autre radical de $R^4$ et $R^5$ ont la signification mentionnée dans les revendications 1 et 8 à 11.

15. Composés de la formule générale (I)

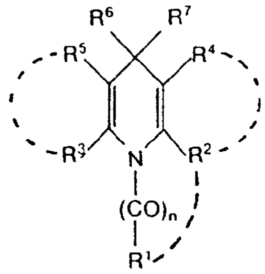

dans laquelle l'un des radicaux $R^4$ ou $R^5$ représente un halogène et où $R^1$, $R^2$, $R^3$, $R^6$ et $R^7$ et n, ainsi que l'autre radical de $R^4$ ou $R^5$ ont la signification mentionnée aux revendications 1 et 8 à 11.

16. Composés selon la revendication 15, dans lesquels les radicaux $R^4$ ou $R^5$ représentent du fluor ou du chlore.

17. Procédé pour la production de composés selon les revendications 12 à 16, caractérisé en ce que l'on fait réagir

a) des composés carbonyles de la formule générale (II)

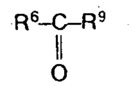 (II)

avec des cétones des formules (III) et (IV)

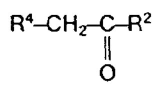 (III) 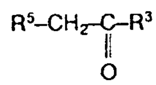 (IV)

et des amines primaires de la formule (V)

$$H_2N\text{-}(CO)_n\text{-}R^1$$

dans lesquels n = 0, éventuellement en présence de solvants inertes, ou

b) des composés carbonyles de la formule (II)

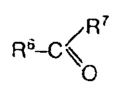 (II)

avec des énamines de la formule (VI)

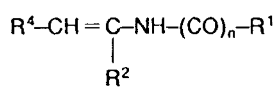 (VI)

et des cétones de la formule (IV)

$$R^3\text{-}CO\text{-}CH_2\text{-}R^5$$

éventuellement en présence de solvants inertes ou

c) des énamines de la formule (VI)

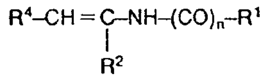 (VI)

avec des composés ylidènes de la formule (VIII) éventuellement en présence de solvants inertes, ou

d) ou que l'on transforme un ou plusieurs groupes fonctionnels des dihydropyridines suivant les procédés classiques de l'hydrolyse, de l'estérification, de la transestérification, de la lactonisation, de la condensation, de l'acylation, de la réduction ou de la cyclisation.

18. Médicaments contenant au moins un composé selon les revendications 12 à 16.

19. Procédé de préparation de médicaments, caractérisé en ce que l'on introduit au moins un composé selon les revendications 11 à 16, éventuellement avec utilisation de substances de support ou d'adjuvants dans une formulation d'application appropriée.